# EUROPEAN PATENT APPLICATION

(11) **EP 1 961 753 A1**
(43) Date of publication of application: **27.08.2008**
(21) Application number: 06817839.1
(22) Date of filing: 16.11.2006
(51) Int. Cl.: C07D 487/04, A61K 31/519, C07D 239/00, C07D 231/00

(54) **PYRAZOLOPYRIMIDINONE DERIVATIVES, THEIR PREPARATION AND THEIR USE**

(30) Priority: 17.11.2005 CN 200510110485
(71) Applicant: Topharman Shanghai Co., Ltd., Shanghai 201209 (CN); Shanghai Institute of Materia Medica, Chinese Academy of Sciences, Pudong New, Shanghai 201203 (CN); Henan Topfond Pharmaceutical Co., Ltd., Henan 463000 (CN)
(72) Inventor: TIAN, Guanghui, Shanghai 201209 (CN); LAI, Shunan, Shanghai 201209 (CN); WANG, Zhen, Shanghai 201209 (CN); ZHU, Yi, Shanghai 201209 (CN); CHEN, Xinjian, Shanghai 201209 (CN); JI, Yurong, Shanghai 201209 (CN); ZHANG, Jinfeng, Shanghai 201209 (CN); JIN, Weixi, Shanghai 201209 (CN); LV, Heping, Shanghai 201209 (CN); LIU, Jinping, Shanghai 201209 (CN); WANG, Wei, Shanghai 201209 (CN); JI, Ruyun, Shanghai 201209 (CN); SHEN, Jingshan, Shanghai 201209 (CN)
(74) Representative: van Heuvel, Margaretha
(86) International application number: PCT/CN2006/003094
(87) International publication number: WO 2007/056955

(57) **Abstract**

The invention discloses a series of pyrazolopyrimidinone derivatives, which have the following chemical structure, their preparation methods and use, 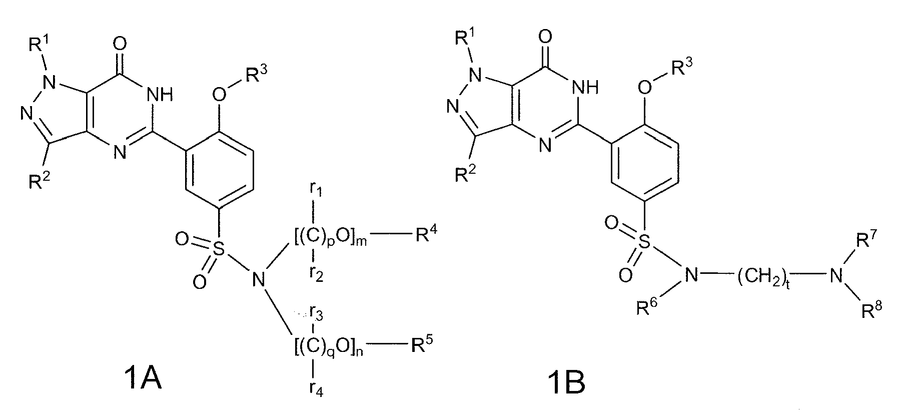 It has been proved by pharmacological experiment that the said pyrazolopyrimidinone derivatives have high inhibitory activity against PDE5, and parts of them have a much stronger potency against PDE5 than against PDE6. Most of the compounds show low toxicity. The pyrazolopyrimidinone derivatives can be used in clinics for the palliative or curative treatment of symptoms or diseases relating to cardiovascular system, urinary system, especially for the palliative or curative treatment of erectile dysfunction.

## Description

### Technical Field

This invention relates to a series of new pyrazolopyrimidinone derivatives (1A and 1B), processes for their preparation, and pharmaceutical compositions containing them. The compounds have potent inhibitory activities against type V phosphodiesterase (PDE5), therefore they are useful for treating erectile dysfunction and other cardiovascular dysfunction.

### Background

International application WO94/28902 disclosed the use of pyrazolo[4,3-d]pyrimidine-7-one as selective cGMP PDE inhibitor for erectile dysfunction, and then WO02/27848 disclosed another series of pyrazolo[4,3-d]pyrimidine-7-one derivates, which also have potent inhibitory activity against PDE5.

The level of cGMP in the smooth muscle cell will increase once the PDE5 in smooth muscle cells are inhibited, cGMP activates protein kinase G (PKG), which subsequently phosphorylates the target protein including smooth muscle myosin, and resulting in the relaxation of smooth muscle and vasodilation. Therefore PDE5 inhibitors are useful in the treatment of a variety of cardiovascular diseases.

Sildenafil, the first launched PDE5 inhibitor, is used for male erectile dysfunction in clicnic, which also demonstrates clinical effect in female sexual dysfunction and hyperpietic. The PDE5 inhibitor under development is also used for the treatment of alimentary canal in the diabetic, insulin resistance and hyperlipemia.

Despite its effectiveness, Sildenafil has shown clinically significant adverse reactions such as headache, flushing, dyspepsia, snuffle, dimness of vision, photosensitive, and other visual disturbances, which may be linked to insufficient selectivity versus the other PDE isoforms and the dosage. Therefore, both potencies toward PDE5 and selectivities against other PDEs, especially PDE6 are the goal for the successful development of new PDE5 inhibitors.

### Summary

It is an object of the present invention to provide a series of new pyrazolopyrimidinone derivatives (1A and 1B).

It is another object of the present invention to provide the processes for the preparation of compounds of formula 1A and 1B.

It is still another object of the present invention to provide the pharmaceutical compositions containing the compounds of formula 1A and/or 1B.

The inventors designed and synthesized a series of novel pyrazolopyrimidinone derivatives (1A and 1B), most of which have higher inhibitory activity towards PDE5 and better selectivity against PDE6 distributing in retina than Sildenafil. Therefore, the compound provided by this invention will demonstrate better safety and efficacy, and has a good prospect in clinical application. 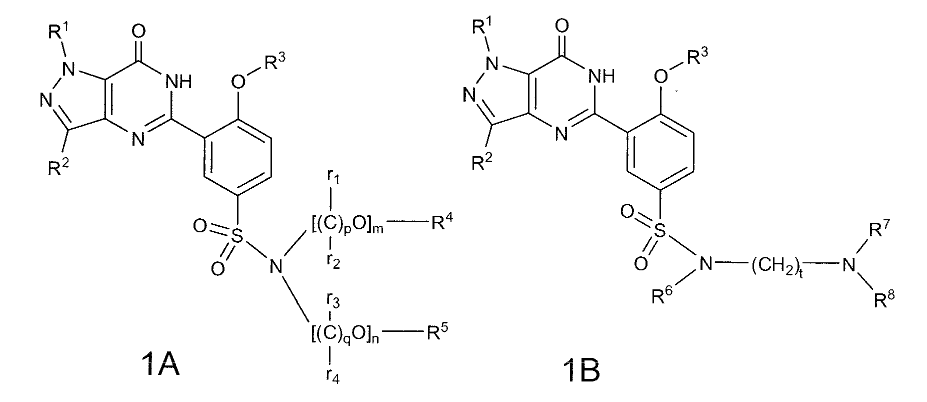 wherein:
R¹ represents H, C1-C6 alkyl, C3-C6 cycloalkyl, C1-C3 alkyl substituted by halogen or C3-C6 cycloalkyl;
R² represents C2-C6 alkyl, C3-C6 cycloalkyl, C1-C3 alkyl substituted by halogen or C3-C6 cycloalkyl;
R³ represents C1-C6 alkyl, C3-C6 cycloalkyl, C1-C3 alkyl substituted by halogen, C1-C3 alkoxyl C3-C6 cycloalkyl;
wherein in formula 1A,
m=1-6 ; n=0-6 ;
p=1-5 ; q=1-5 ;
r₁, r₂, r₃ and r₄ independently represent H or C1-C3 alkyl, r₁ and r₂, r₃ and r₄ together with the carbon to which they are attached form Het;
R⁴ and R⁵ independently represent H, C1-C6 alkyl, (CH₂)ᵤAr , (CH₂)ᵥHet , COR⁹ , C1-C3 alkyl substituted by NR¹⁰R¹¹ or C1-C3 alkyl; in case of n=0, R⁵ does not represent H; in case of R¹ represents methyl, R² represents propyl, R³ represents ethyl, m=1, n=1, p=q=2 , r1,r2,r3 and r4 all represent H, R⁴ and R⁵ do not represent H at same time.
R⁹ represents H, C₁-C₆ alkyl, C₁-C₆ alkyl substituted by C₁-C₃ alkoxyl or NR¹²R¹³, (CH₂)ᵤAr or (CH₂)ᵥHet ;
R¹⁰ and R¹¹ independently represent H, C₁-C₆ alkyl, C₁-C₆ alkyl substituted by C₁-C₃ alkoxyl or NR¹²R¹³, or R¹⁰ and R¹¹ together with the nitrogen to which they are attached form Het;
R¹² and R¹³ independently represent H, C₁-C₆ alkyl;
wherein in formula (1B)
t=1-5 ;
R⁶ represents C1-C6 alkyl, C3-C6 cycloalkyl, C1-C6 haloalkyl, C1-C3 alkoxyl, phenyl, pyridyl, furanyl, pyridazinyl, pyrazinyl, imidazolyl, C₁-C₃ substituted with hydroxyl, C₁-C₃ alkoxyl, acetoxyl, phenyl, pyridyl, furanyl, pyridazinyl, pyrimidinyl, pyrazinyl, imidazolyl; the above phenyl, pyridyl, furanyl, pyridazinyl, pyrimidinyl, pyrazinyl, imidazolyl optionally substituted with one or more substituents selected from halogen, C1-C3 alkyl, C1-C3 alkoxyl;
R⁷ and R⁸ independently represent H, C₁-C₆ alkyl, C3-C6 cycloalkyl, C₁-C₃ haloalkyl, C₁-C₆ alkoxyl, C1-C3 alkyl substituted with hydroxyl, acetoxyl, C₁-C₃ alkoxyl, or R⁷ and R⁸ together with the nitrogen to which they are attached form a four-membered to eight-membered heterocyclic ring, including morpholine, piperidine, pyrrole, piperazine; the above heterocyclic ring optionally substituted with one or more substituents selected from halogen, C1-C3 alkyl, C3-C6 cycloalkyl, C₁-C₃ haloalkyl, C1-C3 alkoxyl;
wherein u, v=0, 1 or 2;
Ar represents phenyl or phenyl substituted by one to two substituents selected from halogen, NH2, C1-C3 alkyl, C1-C3 alkoxy, CONH₂, CN, SO₂NH₂;
Het represents a four-membered and six-membered heterocyclic ring substituted with one or two substituents selected from halogen, C1-C3 alkyl, C1-C3 alkoxy, the heterocyclic contains one to four heteroatoms selected from nitrogen, sulfur, oxygen.

As previously defined, no particular explanation, alkyl with three or more carbon wherein said alkyl may be straight or branched chain. Halogen represents fluorine, chlorine, bromine or iodine.

The compound of formula 1A and 1B may have one or more chiral center, therefore, the compound may exist as a stereomer, that is to say, enantiomer, diastereomer or their mixture. The invention includes within its scope all the possible isomers, stereomers and their mixture of formulae 1A and 1B.

The compounds of formula 1A and 1B may have stereomers and this invention includes all the possible isomers, stereomers and their mixtures thereof.

The invention includes within its all the possible prodrugs of formula 1A and 1B.

The invention includes the pharmaceutically acceptable salts of formula 1A and 1B, preferred salts are hydrochloride and methanesulfonate.

The invention still includes the pharmaceutically acceptable solvates of formula 1A and 1B (e.g. hydrates).

The invention also includes the pharmaceutically oxide of formula 1A and 1B.

Preferred compounds of 1A and 1B include those wherein:
R¹ represents C₁-C₄ alkyl or C₃-C₆ cycloalkyl;
R² represents C₂-C₄ alkyl or C3-C6 cycloalkyl;
R³ represents C₁-C₃ alkyl, C₁-C₃ alkyl substituted with C₁-C₃ alkoxyl;
m=1-2 ; n=0-2 ;
p=2-4 ; q=2-4 ;
r₁, r₂, r₃ and r₄ independently represent H or methyl;
R⁴ and R⁵ independently represent H, C₁-C₃ alkyl, phenyl, pyridimethyl 4-piperidyl, COR⁹, C₁-C₃ alkyl substituted by NR¹⁰R¹¹; in case of n=0, R⁵ does not represent H; in case of R¹ represents methyl, R² represents propyl, R³ represents ethyl, m=1, n=1, p=q=2 , r₁, r₂, r₃ and r₄ all represent H, R⁴ and R⁵ do not represent H at same time;
R⁹ represents C₁-C₄ alkyl, phenyl or pyridyl ;
R¹⁰ and R¹¹ independently represent H, C₁-C₃ alkyl; or R¹⁰ and R¹¹ together with the nitrogen to which they are attached form a heterocyclic ring, including morpholine, piperazine, piperidine, pyrrole;
wherein in 1B:
t=2-3 ;
R⁶ represents C₁-C₃ alkyl, phenyl, pyridyl, benzyl or C₁-C₃ substituted with hydroxyl, C₁-C₃ alkoxyl, acetoxyl, phenyl, pyridyl;
R⁷ and R⁸ together with the nitrogen to which they are attached form a morpholine, piperidine or pyrrole heterocyclic ring;

Particularly preferred compounds of 1A and 1B include those wherein:
R¹ represents methyl or ethyl;
R² represents ethyl and n-propyl;
R³ represents ethyl, n-propyl or methyloxyethyl;
Wherein 1A,
m=1; n=0-1;
p=2-3; q=2-3;
r₁, r₂, r₃ and r₄ represent H;
R⁴ and R⁵ independently represent H, methyl, ethyl or COR⁹; C₁-C₃ alkyl substituted by NR¹⁰R¹¹; in case of n=0, R⁵ does not represent H; in case of R¹ represents methyl, R² represents propyl, R³ represents ethyl, m=1, n=1, p=q=2 , r1,r2,r3 and r4 all represent H, R⁴ and R⁵ do not represent H at same time;
R⁹ represents methyl or pyridyl ;
wherein in 1B,
t=2-3
R⁶ represents methyl, ethyl, benzyl, pyridylmethyl, or C₁-C₃ substituted with hydroxyl, C₁-C₃ alkoxyl, acetoxyl;

The preferable compounds of the present invention are:
1-methyl-5-{2-propyloxy-5-[bis(2-acetoxyethyl)amidosulfonyl]phenyl}-3-n-propyl-1,6-dihydro-7H-pyrazolo[4,3-d]pyrimidin-7-one (compound of example 1)
1-methyl-5-{2-propyloxy-5-[bis(2-hydroxyethyl)amidosulfonyl]phenyl}-3-n-propyl-1,6-dihydro-7H-pyrazolo[4,3-d]pyrimidin-7-one (compound of example 2)
1-methyl-5-{2-propoxy-5-[bis(2-nicotinoyloxyethyl)amidosulfonyl]phenyl}-3-n-propyl-1,6-dihydro-7H-pyrazolo[4,3-d]pyrimidin-7-one (compound of example 3)
1-methyl-5-{2-ethoxy-5-[bis(2-acetoxyethyl)amidosulfonyl]phenyl}-3-n-propyl-1,6-dihydro-7H-pyrazolo[4,3-d]pyrimidin-7-one (compound of example 4)
1-methyl-5-{2-ethoxy-5-[bis(2-nicotinoyloxyethyl)amidosulfonyl]phenyl}-3-n-propyl-1,6-dihydro-7H-pyrazolo[4,3-d]pyrimidin-7-one (compound of example 5)
1-methyl-5-{2-ethoxy-5-[bis(2-acetoxyethyl)amidosulfonyl]phenyl}-3-ethyl-1,6-dihydro-7H-pyrazolo[4,3-d]pyrimidin-7-one (compound of example 6)
1-methyl-5-{2-ethoxy-5-[bis(2-nicotinoyloxyethyl)amidosulfonyl]phenyl}-3-ethyl-1,6-dihydro-7H-pyrazolo[4,3-d]pyrimidin-7-one (compound of example 7)
1-methyl-5-{2-propoxy-5-[bis(2-hydroxyethyl)amidosulfonyl]phenyl}-3-ethyl-1,6-dihydro-7H-pyrazolo[4,3-d]pyrimidin-7-one (compound of example 8)
1-methyl-5-{2-methoxyethyl-5-[bis(2-acetoxyethyl)amidosulfonyl]phenyl}-3-n-propyl-1,6-dihydro-7H-pyrazolo[4,3-d]pyrimidin-7-one (compound of example 9)
1-methyl-5-{2-methoxyethyl-5-[bis(2-hydroxyethyl)amidosulfonyl]phenyl}-3-n-propyl-1,6-dihydro-7H-pyrazolo[4,3-d]pyrimidin-7-one (compound of example 10)
1-methyl-5-{2-ethoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one (compound of example 13)
1-methyl-5-{2-propoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one (compound of example 14)
1-methyl-5-{2-ethoxy-5-[1-(2-diethylaminoethyl)-1-(2-hydroxyethoxyethyl)amidosulfonyl]phenyl}-3-n-propyl-1,6-dihydro-7H-pyrazolo[4,3-d]pyrimidin-7-one (compound of example 16)
1-ethyl-5-{2-ethoxy-5-{[1-ethyl-1-[2-(1-ethyl-1-(2-acetoxyethyl))amino]ethyl]amidosulfonyl}phenyl}-3-n-propyl-1,6-dihydro-7H-pyrazolo[4,3-d]pyrimidin-7-one (compound of example 19)
1-methyl-5-{2-ethoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-one (compound of example 21)
1-methyl-5-{2-propoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-one (compound of example 23)
1-methyl-5-{2-propoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 24)
1-methyl-5-{2-ethoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 25)
1-methyl-5-{2-methoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 26)
1-methyl-5-{2-ethoxy-5-[[1-benzyl-1-(2-morpholin-1-yl)ethyl]amidosulfonyl]phenyl}-3-ethyl-1,6-dihydro-7H-pyrazolo[4,3-d]pyrimidin-7-one (compound of example 27)
1-methyl-5-{2-ethoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 28)
1-methyl-5-{2-ethoxy-5-[[1-(2-hydroxyethyl)-1-(2-piperidin-1-yl)ethyl]amidosulfonyl]phenyl}-3-n-propyl-1,6-dihydro-7H-pyrazolo[4,3-d]pyrimidin-7-one (compound of example 29)
1-methyl-5-{2-propoxy-5-[[1-methyl-1-(2-hydroxyethyl)]amidosulfonyl]phenyl}-3-n-propyl-1,6-dihydro-7H-pyrazolo[4,3-d]pyrimidin-7-one (compound of example 31)
1-methyl-5-{2-propoxy-5-[[1-methyl-1-(2-acetoxyethyl)]amidosulfonyl]phenyl}-3-n-propyl-1,6-dihydro-7H-pyrazolo[4,3-d]pyrimidin-7-one (compound of example 33)
1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 35)
1-methyl-5-{2-ethoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 36)
1-methyl-5-{2-propoxy-5-[bis(3-hydroxypropyl)amidosulfonyl]phenyl}-3-ethyl-1,6-dihydro-7H-pyrazolo[4,3-d]pyrimidin-7-one (compound of example 37)
1-methyl-5-{2-propoxy-5-[[1-(2-hydroxyethyl-1-(3-hydroxypropyl)]amidosulfonyl]phenyl}-3-n-propyl-1,6-dihydro-7H-pyrazolo[4,3-d]pyrimidin-7-one (compound of example 38)
1-methyl-5-{2-propoxy-5-[[1-(2-hydroxyethoxyethyl-1-(3-hydroxypropyl)]amidosulfonyl]phenyl}-3-n-propyl-1,6-dihydro-7H-pyrazolo[4,3-d]pyrimidin-7-one (compound of example 39)
1-methyl-5-{2-propoxy-5-[[1-ethyl-1-(2-morpholin-1-yl)ethyl]amidosulfonyl]phenyl}-3-propyl-1,6-dihydro-7H-pyrazolo[4,3-d]pyrimidin-7-one (compound of example 41)
1-methyl-5-{2-propoxy-5-[bis(2-hydroxypropyl)amidosulfonyl]phenyl}-3-propyl-1,6-dihydro-7H-pyrazolo[4,3-d]pyrimidin-7-one (compound of example 42)
1-methyl-5-{2-ethoxy-5-[[1-ethyl-1 -(2-morpholin-1-yl)ethyl]amidosulfonyl]phenyl}-3-n-propyl-1,6-dihydro-7H-pyrazolo[4,3-d]pyrimidin-7-one (compound of example 43)
1-methyl-5-{2-ethoxy-5-[bis(3-hydroxypropyl)amidosulfonyl]phenyl}-3-n-propyl-1,6-dihydro-7H-pyrazolo[4,3-d]pyrimidin-7-one (compound of example 44)
1-methyl-5-{2-ethoxy-5-[bis(2-hydroxypropyl)amidosulfonyl]phenyl}-3-n-propyl-1,6-dihydro-7H-pyrazolo[4,3-d]pyrimidin-7-one (compound of example 45)
1-methyl-5-{2-ethoxy-5-[[1-(2-hydroxyethyl)-1-(3-hydroxypropyl)]amidosulfonyl]phenyl}-3-n-propyl-1,6-dihydro-7H-pyrazolo[4,3-d]pyrimidin-7-one (compound of example 46)
1-methyl-5-{2-ethoxy-5-[[1-(2-hydroxyethoxyethyl)-1-(3-hydroxypropyl)]amidosulfonyl]phenyl}-3-n-propyl-1,6-dihydro-7H-pyrazolo[4,3-d]pyrimidin-7-one (compound of example 47)
1-methyl-5-{2-ethoxy-5-[[1-ethyl-1-(2-hydroxyethyl)]amidosulfonyl]phenyl}-3-n-propyl-1,6-dihydro-7H-pyrazolo[4,3-d]pyrimidin-7-one (compound of example 51)
1-methyl-5-{2-propoxy-5-[[1-ethyl-1-(2-hydroxyethyl)]amidosulfonyl]phenyl}-3-n-propyl-1,6-dihydro-7H-pyrazolo[4,3-d]pyrimidin-7-one (compound of example 52)
1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 53)
1-methyl-5-{2-propoxy-5-[[1-ethyl-1-(2-morpholin-1-yl)ethyl]amidosulfonyl]phenyl}-3-ethyl-1,6-dihydro-7H-pyrazolo[4,3-d]pyrimidin-7-one (compound of example 54)
1-methyl-5-{2-propoxy-5-[[1-(4-fluorobenzyl)-1-(2-morpholin-1-yl)ethyl]amidosulfonyl]phenyl}-3-n-propyl-1,6-dihydro-7H-pyrazolo[4,3-d]pyrimidin-7-one (compound of example 56)
1-methyl-5-{2-propoxy-5-[[1-(2-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 60)
1-methyl-5-{2-ethoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 61)
1-methyl-5-{2-propoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 62)
1-methyl-5-{2-propoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 63)
1-methyl-5-{2-ethoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 64)
1-methyl-5-{2-propoxy-5-[[1-benzyl-1-(3-hydroxypropyl)]amidosulfonyl]phenyl}-3-n-propyl-1,6-dihydro-7H-pyrazolo[4,3-d]pyrimidin-7-one (compound of example 65)
1-methyl-5-{2-ethoxy-5-[[1-benzyl-1-(3-hydroxypropyl)]amidosulfonyl]phenyl}-3-n-propyl-1,6-dihydro-7H-pyrazolo[4,3-d]pyrimidin-7-one (compound of example 66)
1-methyl-5-{2-propoxy-5-[[1-benzyl-1-(2-hydroxyethyl)]amidosulfonyl]phenyl}-3-n-propyl-1,6-dihydro-7H-pyrazolo[4,3-d]pyrimidin-7-one (compound of example 68)
1-methyl-5-{2-propoxy-5-[[1-ethyl-1 -(3-hydroxypropyl)]amidosulfonyl]phenyl}-3-n-propyl-1,6-dihydro-7H-pyrazolo[4,3-d]pyrimidin-7-one (compound of example 70)
1-methyl-5-{2-propoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7-one (compound of example 72)
1-methyl-5-{2-propoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one (compound of example 78)
1-methyl-5-{2-ethoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one (compound of example 79)
1-methyl-5-{2-propoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 80)
1-methyl-5-{2-ethoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 81)
1-methyl-5-{2-ethoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one (compound of example 82)
1-methyl-5-{2-propoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one (compound of example 83)
1-methyl-5-{2-propoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 84)
1-methyl-5-{2-ethoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 85)
1-methyl-5-{2-propoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one (compound of example 87)
1-methyl-5-{2-ethoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one (compound of example 88)
1-methyl-5-{2-propoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one (compound of example 89)
1-methyl-5-{2-ethoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one (compound of example 90)
1-methyl-5-{2-propoxy-5-[[1-(2-acetoxyethyl)-1-(2-hydroxyethyl)]amidosulfonyl]phenyl}-3-n-propyl-1,6-dihydro-7H-pyrazolo[4,3-d]pyrimidin-7-one (compound of example 91)
1-methyl-5-{2-propoxy-5-[[1-(3-acetoxypropyl)-1-(3-hydroxypropyl)]amidosulfonyl]phenyl}-3-n-propyl-1,6-dihydro-7H-pyrazolo[4,3-d]pyrimidin-7-one (compound of example 92)
1-methyl-5-{2-propoxy-5-[bis(3-acetoxypropyl)amidosulfonyl]phenyl}-3-n-propyl-1,6-dihydro-7H-pyrazolo[4,3-d]pyrimidin-7-one (compound of example 93)
1-methyl-5-{2-propoxy-5-[[-ethyl-1-(2-acetoxyethyl)]amidosulfonyl]phenyl}-3-n-propyl-1,6-dihydro-7H-pyrazolo[4,3-d]pyrimidin-7-one (compound of example 94)
1-methyl-5-{2-propoxy-5-[[1-(2-hydroxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 95)
1-methyl-5-{2-propoxy-5-[[1-(2-acetoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 96)
1-methyl-5-{2-propoxy-5-[[1-ethyl-1-(3-acetoxypropyl)]amidosulfonyl]phenyl}-3-n-propyl-1,6-dihydro-7H-pyrazolo[4,3-d]pyrimidin-7-one (compound of example 97)
1-methyl-5-{2-propoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 98)
1-methyl-5-{2-propoxy-5-[[1-(methoxypropyl)-1-(3-hydroxypropyl)]amidosulfonyl]phenyl}-3-n-propyl-1,6-dihydro-7H-pyrazolo[4,3-d]pyrimidin-7-one (compound of example 99)
1-methyl-5-{2-ethoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 100)
1-methyl-5-{2-ethoxy-5-[[1-(2-methoxyethyl)-1-(2-hydroxyethyl)]amidosulfonyl]phenyl}-3-n-propyl-1,6-dihydro-7H-pyrazolo[4,3-d]pyrimidin-7-one (compound of example 101)
1-methyl-5-{2-ethoxy-5-[[1-(2-methoxyethyl)-1-(3-hydroxypropyl)]amidosulfonyl]phenyl}-3-n-propyl-1,6-dihydro-7H-pyrazolo[4,3-d]pyrimidin-7-one (compound of example 102)
1-methyl-5-{2-propoxy-5-[bis(3-methoxypropyl)amidosulfonyl]phenyl}-3-n-propyl-1,6-dihydro-7H-pyrazolo[4,3-d]pyrimidin-7-one (compound of example 103)
1-methyl-5-{2-propoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 104)
1-methyl-5-{2-propoxy-5-[[1-(2-methoxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 105)
1-methyl-5-{2-propoxy-5-[[1-(2-methoxyethyl)-1-(2-hydroxyethyl)]amidosulfonyl]phenyl}-3-n-propyl-1,6-dihydro-7H-pyrazolo[4,3-d]pyrimidin-7-one (compound of example 106)
1-methyl-5-{2-ethoxy-5-[[1-methyl-1-(2-acetoxyethyl)]amidosulfonyl]phenyl}-3-n-propyl-1,6-dihydro-7H-pyrazolo[4,3-d]pyrimidin-7-one (compound of example 107)
1-methyl-5-{2-ethoxy-5-[bis(3-acetoxypropyl)amidosulfonyl]phenyl}-3-n-propyl-1,6-dihydro-7H-pyrazolo[4,3-d]pyrimidin-7-one (compound of example 108)
1-methyl-5-{2-propoxy-5-[[1-(3-methoxypropyl)-1-(2-hydroxyethyl)]amidosulfonyl]phenyl}-3-n-propyl-1,6-dihydro-7H-pyrazolo[4,3-d]pyrimidin-7-one (compound of example 109)
1-methyl-5-{2-propoxy-5-[[1-methyl-1-(3-dimethylaminopropyl)]amidosulfonyl]phenyl}-3-n-propyl-1,6-dihydro-7H-pyrazolo[4,3-d]pyrimidin-7-one (compound of example 110)
1-methyl-5-{2-propoxy-5-[[1-(2-ethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 111)
1-methyl-5-{2-propoxy-5-[[1-ethyl-1-(3-morpholin-1-yl)propyl]amidosulfonyl]phenyl}-3-n-propyl-1,6-dihydro-7H-pyrazolo[4,3-d]pyrimidin-7-one (compound of example 112)
1-methyl-5-{2-propoxy-5-[[1-benzyl-1-(3-morpholin-1-yl)propyl]amidosulfonyl]phenyl}-3-n-propyl-1,6-dihydro-7H-pyrazolo[4,3-d]pyrimidin-7-one (compound of example 113)
1-methyl-5-{2-propoxy-5-[[1-(3-methoxypropyl)-1-(2-acetoxyethyl)]amidosulfonyl]phenyl}-3-n-propyl-1,6-dihydro-7H-pyrazolo[4,3-d]pyrimidin-7-one (compound of example 114)
1-methyl-5-{2-ethoxy-5-[[1-(3-acetoxypropyl)-1-(3-hydroxypropyl)]amidosulfonyl]phenyl}-3-n-propyl-1,6-dihydro-7H-pyrazolo[4,3-d]pyrimidin-7-one (compound of example 115)
1-methyl-5-{2-propoxy-5-[[1-(3-ethoxyethyl)-1-(2-hydroxyethyl)]amidosulfonyl]phenyl}-3-n-propyl-1,6-dihydro-7H-pyrazolo[4,3-d]pyrimidin-7-one (compound of example 116)
1-methyl-5-{2-propoxy-5-[[1-benzyl-1-(4-morpholin-1-yl)butyl]amidosulfonyl]phenyl}-3-n-propyl-1,6-dihydro-7H-pyrazolo[4,3-d]pyrimidin-7-one (compound of example 117)
1-methyl-5-{2-propoxy-5-[[1-(3-methoxypropyl)-1-(3-acetoxypropyl)]amidosulfonyl]phenyl}-3-n-propyl-1,6-dihydro-7H-pyrazolo[4,3-d]pyrimidin-7-one (compound of example 118)

The present invention also provides the processes for the preparation of compounds of formula 1A and 1B.

There are two different types of preparation methods according to their structural features.

Type 1, some of the compounds of formula 1A (both R⁴ and R⁵ are not H) and formula 1B may be prepared from 2A and 2B. The scheme is as follows: 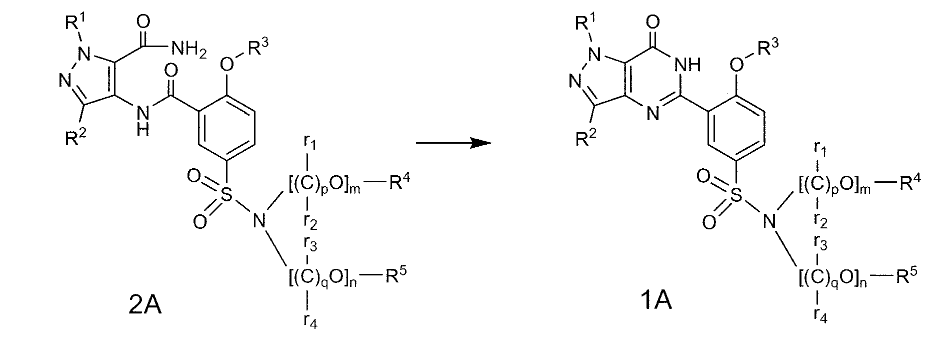 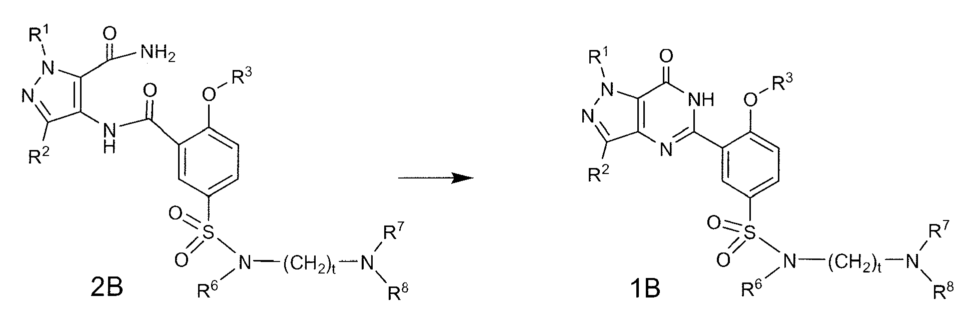 wherein R¹, R², R³, R⁴, R⁵, R⁶, R⁷, R⁸, r₁, r₂, r₃, r₄, m, n, p, q and t are as previously defined for formula 1A and 1B, but R⁴, R⁵ are not H.

This step was achieved by the existing cyclization method for pyrimidinone compounds. The reaction is usually carried out in the presence of a suitable base and a suitable solvent at temperatures at a range from 50 to 200°C. Preferred bases include metal alkoxides (e.g. potassium tertbutoxide, sodium ethoxide), alkaline earth metal or hydrides of alkali metal, amine (e.g. triethylamine), metal salts of ammonia, hydroxides (e.g. sodium hydroxide), carbonates and bicarbonates. Preferred solvents include alcohols (e.g. t-butanol, methanol, ethanol, isopropanol, glycol, 2-methoxyethanol), aromatic hydrocarbons (e.g. benzene, toluene, chlorobenzene), pyridine, halogenated hydrocarbon, acetonitrile, tetrahydrofuran, dioxane, dimethylsulfoxide, N,N-dimethylformamide, N-methylpyrrolidin-2-one.

Type 2, some of the compounds of formula 1A containing hydroxyl group at the ending chain may be prepared form the hydrolysis of their corresponding ester derivates, which is to say that when at least one of R⁴ and R⁵ is H (e.g. 1A-1), the hydroxyl derivates may be prepared through hydrolysis reaction. The scheme is as follows: 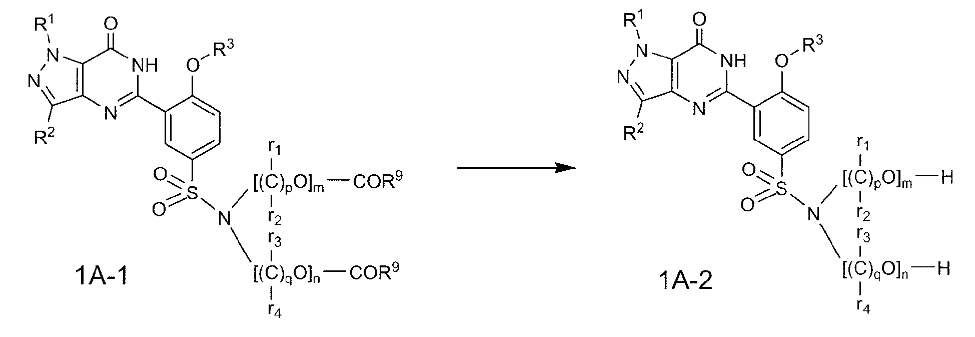 wherein R¹, R², R³, R⁴, R⁵, R⁶, R⁷, R⁸, R⁹, r₁, r₂, r₃, r₄, m, n, p, q and t are as previously defined for formulae 1A and 1B.

The compounds of formula (2A) and (2B) are usually prepared by reacting the compounds of formula (3A) and (3B) with the compounds of formula 4 respectively. The scheme is as follows: 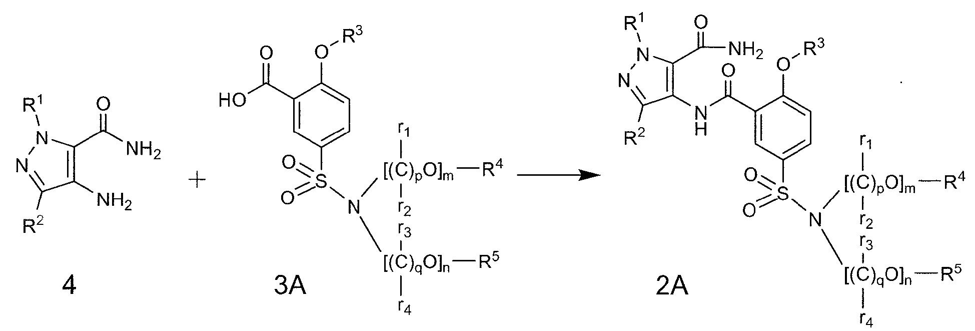 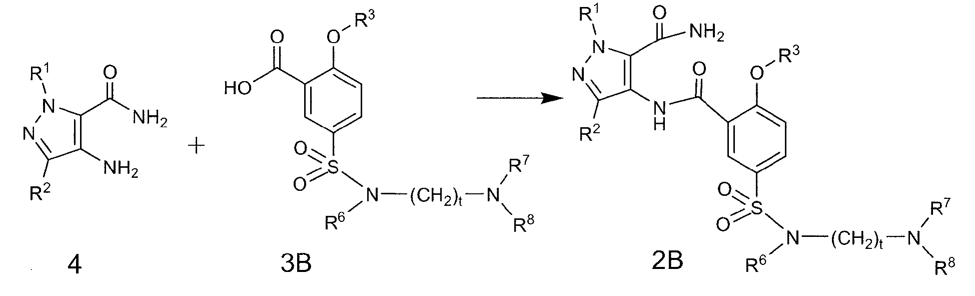 wherein R¹, R², R³, R⁴, R⁵, R⁶, R⁷, R⁸, r₁, r₂, r₃, r₄, m, n, p, q and t are as previously defined for formulae 1A and 1B, but R⁴, R⁵ are not H.

Method 1, the carboxyl group in the compounds of formula (3A) or (3B) was transformed into acyl chloride or mixed anhydride by using thionyl chloride, oxalyl chloride or ethyl chlorformate, then the mixture was reacted with the compounds of formula 4 to get the corresponding acid amide (2A) or (2B). The acidylation reaction is usually carried out in the presence of a suitable deacidification reagent and a common solvent. Preferred deacidification reagents include organic bases (preferred triethylamine, N,N-diisopropylethylamine, pyridine) and inorganic bases (preferred hydroxides, carbonates). Preferred solvents include diolefines (preferred petroleum, n-hexane, cyclohexane), halohydrocarbon (preferred dichlormethane, chloroform), ethers (preferred tetrahydrofuran, dioxane, ether), aromatic solvents (preferred toluene) and alcohols (preferred t-butanol, isopropanol).

Method 2, carboxylic acid was reacted with amine derivates directly to get the formula (2A) and (2B). The reaction is usually carried out in the anhydrous solvent with the presence of activating agent or dehydrating agent. Preferred activating agents or dehydrating agents include DCC, EDCI, EEDQ, CDI, HOBt. Preferred solvents include halogenated hydrocarbons (e.g. dichlormethane, dichlormethane), ethers (e.g. tetrahydrofuran, dioxane, ether), aromatic hydrocarbons (e.g. benzene, toluene), polarity aprotic solvent (dimethylsulfoxide, N,N-dimethylformamide), or their mixture.

The compounds of formula 3A, 3B and 4 can be prepared according to literatures or supplied commercially.

In addition, the present invention provides the pharmaceutical compositions containing the compounds of formula 1A and/or 1B.

The above mentioned compositions contain one or more compounds of formula 1A and/or 1B (or their pharmaceutically acceptable salts, or their pharmaceutically acceptable solvates) and at least one kind of pharmaceutical excipient. The pharmaceutical excipient, which is used according the administration route and their functional properties, are normally fillers, diluents, adhesives, moistening agent, disintegrants, emulsifier, suspending agent, etc.

The compositions according to the invention can be administrated by any suitable route, for example by oral, parenteral (including intravenous, intramuscular, subcutaneous, and intracoronary), sublingual, buccal, rectal, transurethral, vaginal, nasal, inhalation or topical administration. Oral administration is the preferred route.

The compounds of formula 1A and/or 1B should preferably be presented in the above mentioned pharmaceutical compositions in a concentration of about 0.1 to 99.9%, preferably 1 to 99% by weight of the total mixture.

The present invention also provides processes for the preparation of the pharmaceutical compositions containing the compounds of formula 1A and/or 1B. The compounds of formulae 1A and/or 1B can be mixed with pharmaceutical excipient or excipients and made into dosage forms according the administration route in the conventional method. The dosage forms include tablets, capsules, granules, pills, solutions, solutions, emulsion, emulsions, membranes, creams, aerosols, injection and suppositories etc. Tablets and capsules are preferred.

Tablets and capsules can contain one or more compounds of formula of 1A and/or 1B in addition to one or more conventional excipients, such as (a) fillers, for example starches, sucrose, lactose, glucose, microcrystalline cellulose, and mannitol, (b) binders, for example carboxymethylcellulose, gelatine, alginates and polyvinylpyrrolidone, (c) humectants, for example glycerol, (d) disintegrating agents, for example agar-agar, ethyl cellulose, sodium starch glycolate and calcium carbonate (e) lubricants, for example magnesiumstearate, talc, and polyethylene glycols.

The dosages of the compounds of the invention are generally 1 to 500mg per day, preferrely 10 to 100mg, taken once or several times. However, it may be necessary to properly deviate from the dosages mentioned. The optimal dosages, which may be determined by specialist with their professional knowledge, depend on the severity of the disease, the individual response towards the medicament, the characteristics of the formulation, and the administration routes.

Moreover, the invention provides the compounds of formulae 1A and/or 1B, or the pharmaceutically acceptable salt thereof, or the pharmaceutically acceptable solvate of either entity, or the pharmaceutical composition containing any of the foregoing, for use as a human medicament.

The invention further provides the use of the compounds of formulae 1A and/or 1B, or the pharmaceutically acceptable salt thereof, or the pharmaceutically acceptable solvate of either entity, for the manufacture of a human medicament for the curative or prophylactic treatment of a medical condition for which a cGMP PDE5 inhibitor is indicated.

Still further, the invention provides the use of the compounds of formulae 1A and/or 1B, or the pharmaceutically acceptable salt thereof, or the pharmaceutically acceptable solvate containing either entity, for the manufacture of a human medicament for the curative or prophylactic treatment of male erectile dysfunction, benign prostatic hyperplasia (BPH), female sexual dysfunction, premature labour, dysmenorrhoea, bladder outlet obstruction, incontinence, stable, unstable and variant (Prinzmetal) angina, hypertension, pulmonary hypertension, congestive heart failure, kidney failure, atherosclerosis, stroke, peripheral vascular disease, conditions of reduced blood vessel patency, inflammatory disease, bronchitis, chronic asthma, allergic asthma, allergic rhinitis, glaucoma or diseases characterized by disorders of gut motility (e.g. irritable bowel syndrome, IBS).

### Detailed Description of Embodiments

### Examples

The following examples serve to explain the compounds in this invention and the methods for the intermediates, but without limiting it.

¹H NMR spectra were determined on a Mercury 400 NMR spectrometer or Mercury 400 NMR spectrometer (Varian Company). The conventional abbreviation was as follows: s, singlet; d, doublet; t, triplet; q, quartet; m, multiplet; br, broad peak.

### Example 1: 1-methyl-5-{2-propyloxy-5-[bis(2-acetoxyethyl)amidosulfonyl]phenyl}-3-n-propyl-1,6-dihydro-7H-pyrazolo[4,3-d]pyrimidin-7-one

### Step 1: preparation of 4-{2-propoxy-5-[bis(2-acetoxyethyl)amidosulfonyl]benzoylamino}-1-methyl-3-n-propylpyrazolo-5- carboxamine

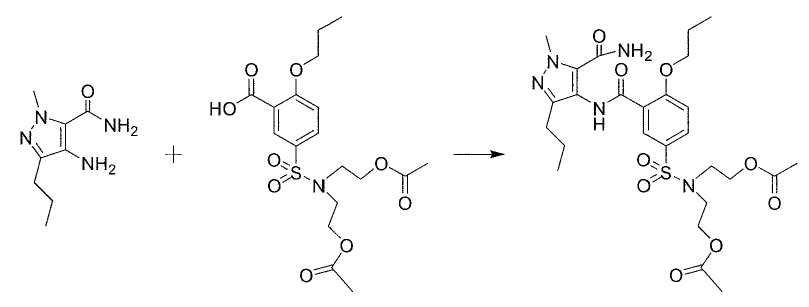

2-propoxy-5-[bis(2-acetoxyethyl)amidosulfonyl]benzoic acid (0.43g, 1mmol) was dissolved in CH₂Cl₂ (20mL), Carbonyldiimidazole (CDI, 3mmol) was added to the solution and stirred at room temperature for 0.5 hours, then 4-amino-3-propylpyrazolo-5-carboxamine (0.18g, 1mmol) was added and stirred another 1-6h, the stopping point was detected by TLC. When the reaction was finished, the reaction mixture was washed with the ammonium chloride solution and brine, the CH₂Cl₂ layer was dried over anhydrous magnesium sulfate and the solvent was concentrated to dryness under reduced pressure, the resulting residue was recrystallized from alcohol to get white powder (0.51g), yield 86%.

### Step 2: preparation of 1-methyl-5-{2-propoxy-5-[bis(2-acetoxyethyl)amidosulfonyl]phenyl}-3-n-propyl-1,6-dihydro-7H-pyrazolo[4,3-d]pyrimidin-7-one

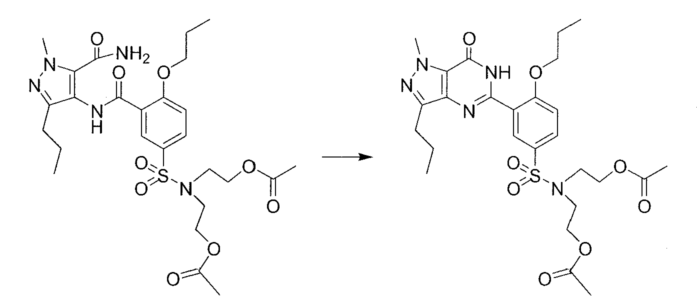

Potassium tert-butoxide (0.06g, 0.55mmol) and the product of step 1 (0.3g, 0.5mmol) were added to tert-butyl alcohol (15ml) successively, and the mixture was heated to reflux for 30 minutes to produce a clear solution. The solution was refluxed for another 10 hours, then cooled to room temperature and water (20ml) was added. The resulted solution was adjusted to neutral by adding 4% acetic acid. cooled to 5-10°C and a white solid was precipitated. The white solid was collected by filtration, washed with cold water (3×10ml) and dried. The solid was recrystallized from MeOH/EtOAc to afford the title compound (0.22g), yield 76%. ¹H NMR (CDCl₃) δ: 10.83 (1H, s), 8.87 (1H, d), 7.90 (1H, dd), 7.14 (1H, d), 4.27 (3H, s), 4.25 (6H, m), 3.49 (4H, t), 2.93 (2H, t), 2.04 (2H, m), 2.03 (6H, s), 1.86 (2H, m), 1.17 (3H, t), 1.02 (3H, t).

### Example 2: 1-methyl-5-{2-propyloxy-5-[bis(2-hydroxyethyl)amidosulfonyl]phenyl}-3-n-propyl-1,6-dihydro-7H-pyrazolo[4,3-d]pyrimidin-7-one

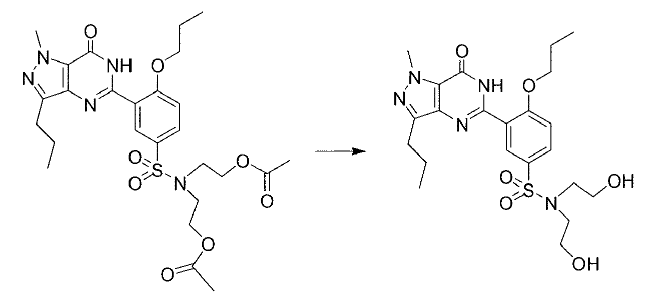

To the solution alcohol (5ml),water (10ml) and potassium carbonate (0.1g, 0.7mmol), the compound of example 1 (0.12g, 0.2mmol) was added and the solution was heated to reflux, the stopping point was detected by TLC. When the reaction was finished, the PH value of the solution was adjusted to neutral by dilute hydrochloric acid and a white solid was precipitated, the solid was collected by filtration, washed with water and dried to get crude product. The crude product was recrystallized from dichloromethane and n-hexane to afford the title compound (0.06g), yield 61%. H NMR (CDCl₃) δ: 10.82 (1H, s), 8.84 (1H, d), 7.91 (1H, dd), 7.14 (1H, d), 4.26 (3H, s), 4.25 (2H, t), 3.88 (4H, t), 3.49 (2H, s), 3.38 (4H, t), 2.92 (2H, t), 2.02 (2H, m), 1.84 (2H, m), 1.17 (3H, t), 1.02 (3H, t).

### Examples 3-120:

The examples 3-120 were prepared from different substitute start materials following the procedure of example 1 and example 2 (unless otherwise noted, NMR spectra were determined in CDCl₃ solution)

| Example | Structure | Name and ¹H-NMR (δ) |
|---|---|---|
| 3 | 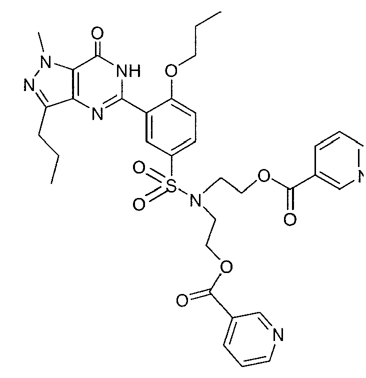 | 1-methyl-5-{2-propoxy-5-[bis(2-nicotinoyloxyethyl)amidosulfonyl]phenyl}-3-n-propyl-1,6-dihydro-7H-pyrazolo[4,3-d]pyrimidin-7-one 11.08 (1H, s), 8.92 (2H, s), 8.75 (3H, m), 8.21 (2H, d), 7.90 (1H, dd), 7.32 (2H, dd), 7.01 (1H, d), 4.56 (4H, t), 4.27 (3H, s), 4.11 (2H, t), 3.77 (4H, t), 2.91 (2H, t), 1.97 (2H, m), 1.84 (2H, m), 1.15 (3H, t), 1.00 (3H, t) |
| 4 | 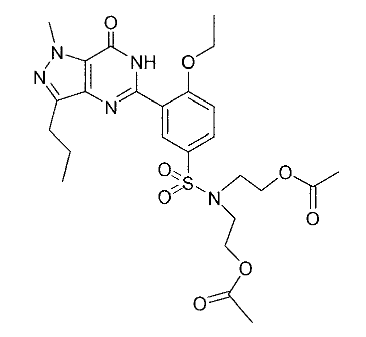 | 1-methyl-5-{2-ethoxy-5-[bis(2-acetoxyethyl)amidosulfonyl]phenyl}-3-n-propyl-1,6-dihydro-7H-pyrazolo[4,3-d]pyrimidin-7-one 10.80(1H, s), 8.87(1H, d), 7.90(1H, dd), 7.14(1H, d), 4.37(2H, q), 4.25(4H, m), 4.24(3H, s), 3.50(4H, m), 2.94(2H, t), 2.02(6H, s), 1.88(2H, m), 1.63(3H, t), 1.01(3H, t) |
| 5 | 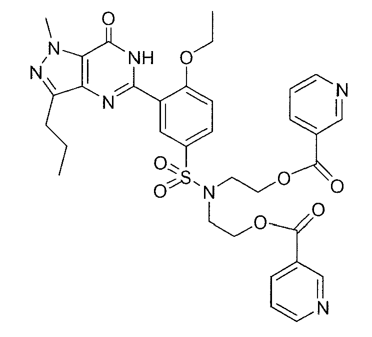 | 1-methyl-5-{2-ethoxy-5-[bis(2-nicotinoyloxyethyl)amidosulfonyl]phenyl}-3-n-propyl-1,6-dihydro-7H-pyrazolo[4,3-d]pyrimidin-7-one 11.05(1H, s), 8.93(2H, d), 8.76(m, 3H), 8.22(2H, m), 7.92(1H, dd), 7.32(2H, m), 7.01(1H, d), 4.57(4H, t), 4.27(3H, s), 4.24(2H, m), 3.76(t, 4H), 2.90(2H, t), 1.80(2H, m), 1.56(3H, t), 1.00(3H, t) |
| 6 | 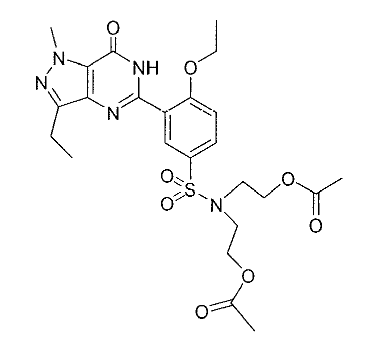 | 1-methyl-5-{2-ethoxy-5-[bis(2-acetoxyethyl)amidosulfonyl]phenyl}-3-ethyl-1,6-dihydro-7H-pyrazolo[4,3-d]pyrimidin-7-one 10.81 (1H, s), 8.87 (1H, d), 7.89 (1H, dd), 7.14 (1H, d), 4.37 (2H, q), 4.27 (3H, s), 4.25 (4H, t), 3.50 (4H, t), 2.98 (2H, q), 2.02 (6H, s), 1.63 (3H, t), 1.41 (3H, t) |
| 7 | 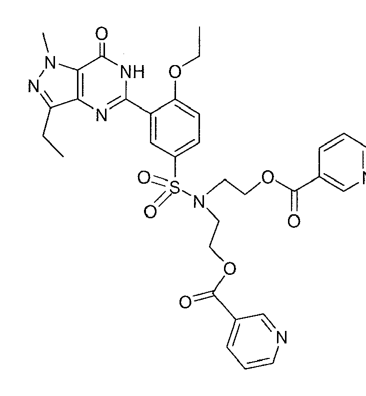 | 1-methyl-5-{2-ethoxy-5-[bis(2-nicotinoyloxyethyl)amidosulfonyl]phenyl}-3-ethyl-1,6-dihydro-7H-pyrazolo[4,3-d]pyrimidin-7-one 11.05 (1H, s), 8.93 (2H, s), 8.75 (3H, m), 8.21 (2H, ddd), 7.90 (1H, dd), 7.33 (2H, ddd), 7.00 (1H, d), 4.56 (4H, t), 4.27 (3H, s), 4.21 (2H, q), 3.77 (4H, t), 2.96 (2H, q), 1.58 (3H, t), 1.39 (3H, t) |
| 8 | 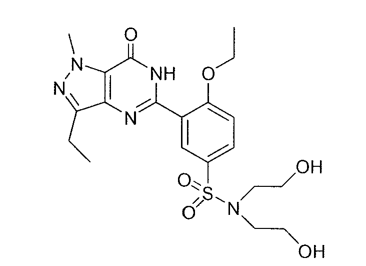 | 1-methyl-5-{2-propoxy-5-[bis(2-hydroxyethyl)amidosulfonyl]phenyl}-3-ethyl-1,6-dihydro-7H-pyrazolo[4,3-d]pyrimidin-7-one 10.79 (1H, s), 8.85 (1H, d), 7.92 (1H, dd), 7.15 (1H, d), 4.37 (2H, q), 4.27 (3H, s), 3.89 (4H, t), 3.39 (4H, t), 2.98 (2H, q), 1.66 (3H, t), 1.40 (3H, t) |
| 9 | 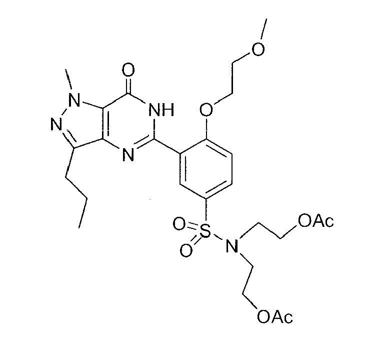 | 1-methyl-5-{2-methoxyethoxy-5-[bis(2-acetoxyethyl)amidosulfonyl]phenyl}-3-n-propyl-1,6-dihydro-7H-pyrazolo[4,3-d]pyrimidin-7-one 11.07 (1H, s), 8.78 (1H, d), 7.90 (1H, dd), 7.12 (1H, d), 4.42 (2H, t), 4.27 (3H, s), 4.24 (4H, t), 3.88 (2H, t), 3.60 (3H, s), 3.49 (4H, t), 2.92 (2H, t), 2.03 (6H, s), 1.86 (2H, m), 1.02 (3H, t) |
| 10 | 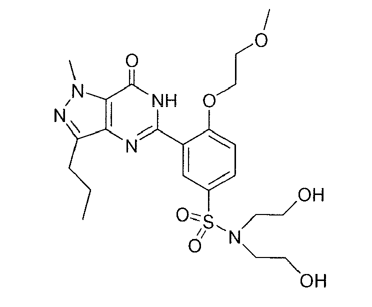 | 1-methyl-5-{2-methoxyethoxy-5-[bis(2-hydroxyethyl)amidosulfonyl]phenyl}-3-n-propyl-1,6-dihydro-7H-pyrazolo[4,3-d]pyrimidin-7-one 11.07 (1H, s), 8.75 (1H, d), 7.91 (1H, dd), 7.13 (1H, d), 4.42 (2H, t), 4.27 (3H, s), 3.87 (6H, m), 3.59 (3H, s), 3.49 (2H, s), 3.38 (4H, t), 2.91 (2H, t), 1.84 (2H, m), 1.02 (3H, t) |
| 11 | 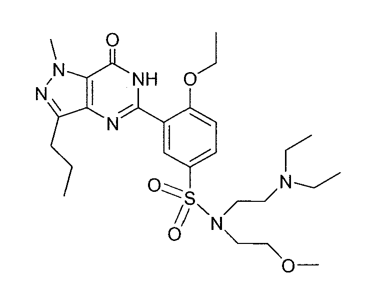 | 1-methyl-5-{2-ethoxy-5-[[1-(2-diethylaminoethyl)-1-(2-methoxyethyl)]amidosulfonyl]phenyl}-3-n-propyl-1,6-dihydro-7H-pyrazolo[4,3-d]pyrimidin-7-one 7.97(1H, d), 7.89(1H, dd), 7.32(1H, d), 4.19(m, 2H), 4.11(3H, s), 3.30(2H, t), 3.20(3H, s), 3.14(2H, t), 2.76(2H, t), 2.50(2H, t), 2.42(4H, m), 1.34(3H, t), 1.26(2H, m), 0.80(9H, m) (DMSO). |
| 12 | 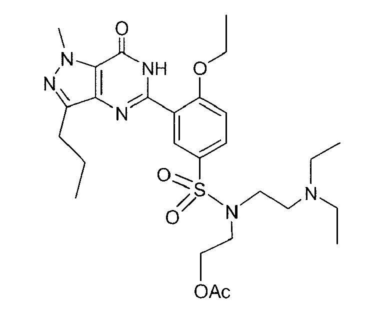 | 1-methyl-5-{2-ethoxy-5-[[1-(2-diethylaminoethyl)-1-(2-acetoxyethyl)]amidosulfonyl]phenyl}-3-n-propyl-1,6-dihydro-7H-pyrazolo[4,3-d]pyrimidin-7-one 10.80(1H, s), 8.88(1H, d), 7.91(1H, dd), 7.12(1H, d), 4.39(2H, q), 4.26(3H, s), 4.23(2H, t), 3.48(2H, t), 3.30(2H, t), 2.92(2H, t), 2.70(2H, m), 2.58(4H, q), 2.04(3H, s), 1.83(2H, m), 1.63(3H, t), 1.01(9H, m) |
| 13 | 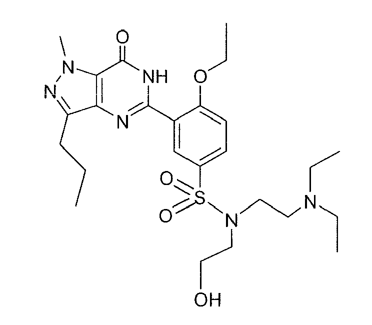 | 1-methyl-5-{2-ethoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one 10.80(1H, s), 8.87 ( 1H , d ) , 7.89 (1H, dd), 7.14 (1H, d), 4.37 (2H, m), 4.27 (3H, s), 3.81 (2H, t), 3.25 (4H, m), 2.93 (2H, t), 2.86 (2H, t), 2.61 (4H, t), 1.86 (2H, m), 1.64 (3H, t), 1.11 (6H, t), 1.03 (3H,t). |
| 14 | 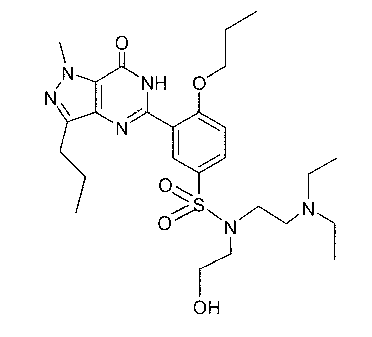 | 1-methyl-5-{2-propoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one 10.86(1H, s), 8.87(1H, d), 7.88(1H, dd), 7.15(1H, d), 4.29(2H, q), 4.27(3H, s), 3.67(2H, t), 3.34(2H, t), 3.24(2H, q), 2.93(2H, t), 2.61(2H, t), 2.56(2H, t), 2.53(2H, t), 2.03(2H, m), 1.85(2H, m), 1.18(3H, t), 1.11(3H, t), 1.01(6H, t). |
| 15 | 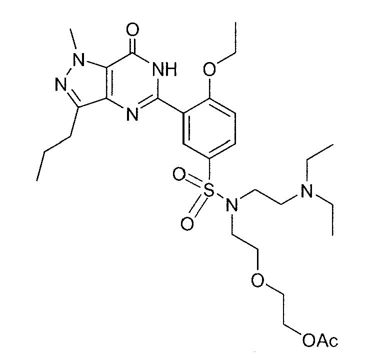 | 1-methyl-5-{2-ethoxy-5-[1-(2-diethylaminoethyl)-1-(2-acetoxyethoxyethyl)amidosulfonyl]phenyl}-3-n-propyl-1,6-dihydro-7H-pyrazolo[4,3-d]pyrimidin-7-one 10.84(1H, s), 8.88(1H, d), 7.92(1H, dd), 7.13(1H, d), 4.37(2H, q), 4.27(3H, s), 4.14(2H, t), 3.67(2H, t), 3.62(2H, q), 3.43(2H, t), 3.33(2H, t), 2.92(2H, t), 2.72(2H, m), 2.57(4H, q), 2.05(3H, s), 1.85(3H, m), 1.66(3H, t), 1.00(9H, m) |
| 16 | 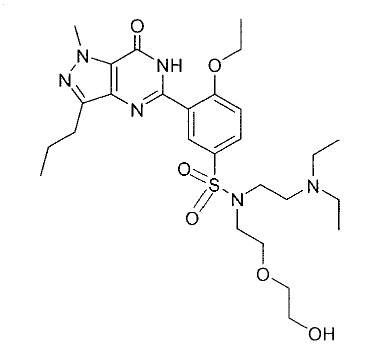 | 1-methyl-5-{2-ethoxy-5-[1-(2-diethylaminoethyl)-1-(2-hydroxyethoxyethyl)amidosulfonyl]phenyl}-3-n-propyl-1,6-dihydro-7H-pyrazolo[4,3-d]pyrimidin-7-one 12.17 (1H, s), 7.97(1H , d), 7.92 (1H, dd), 7.33 (1H, d), 4.20 (2H,m), 4.16 (3H, s), 3.53 (2H, t), 3.44 (2H, t), 3.39 (2H, t), 3.30 (2H, t), 3.17 (2H, t), 2.77 (2H, t), 2.55 (2H, t), 2.44 (4H, m), 1.74 (2H, m), 1.33 (3H, t), 0.92 (9H, m)(DMSO) |
| 17 | 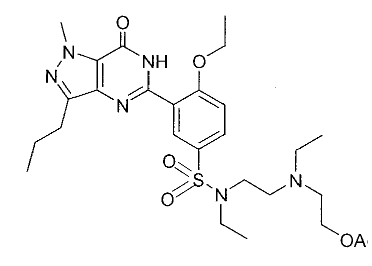 | 1-methyl-5-{2-ethoxy-5-{[1-ethyl-1-[2-[1-ethyl-1-(2-acetoxyethyl)]amino]ethyl]amidosulfonyl]}phenyl }-3-n-propyl-1,6-dihydro-7H-pyrazolo[4,3-d]pyrimidin-7-one 10.84(1H, s), 8.88(1H, d), 7.91(1H, dd), 7.12(1H, d), 4.36(2H, q), 4.33(3H, S), 4.07(2H, t), 3.32(2H, q), 3.20(2H, t), 2.92(2H, t), 2.73(4H, m), 2.58(2H, q), 2.02(3H, s), 1.85(2H, m), 1.64(3H, t), 1.21(3H, t), 1.01(6H, m) |
| 18 | 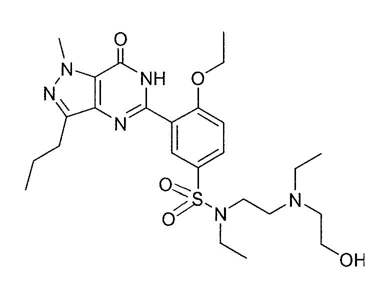 | 1-methyl-5-{2-ethoxy-5-{[1-ethyl-1-[2-[1-ethyl-1-(2-hydroxyethyl)]amino]ethyl]amidosulfonyl]}phenyl }-3-n-propyl-1,6-dihydro-7H-pyrazolo[4,3-d]pyrimidin-7-one 10.85 (1H, s), 8.87 ( 1H , d ) , 7.91 (1H, dd), 7.13 (1H, d), 4.37 (2H, m), 4.27 (3H, s), 3.55 (2H, t), 3.31 (2H, q), 3.24 (2H, q), 2.92 (2H, t), 2.74 (2H, t), 2.64 (2H, t), 2.62 (2H, t), 1.85 (2H, m), 1.64 (3H, t), 1.17 (3H, t), 1.03 (6H, m) |
| 19 | 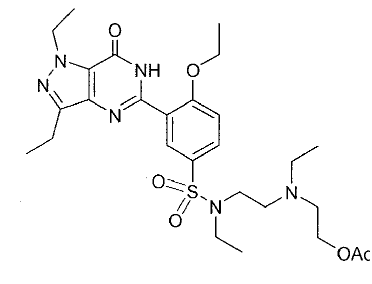 | 1-ethyl-5-{2-ethoxy-5-{[1-ethyl-1-[2-[1-ethyl-1-(2-acetoxyethyl)]amino]ethyl]amidosulfonyl}phenyl} -3-n-propyl-1,6-dihydro-7H-pyrazolo[4,3-d]pyrimidin-7-one 10.88(1H, s), 8.89(1H, d), 7.91(1H, dd), 7.13(1H, d), 4.64(2H, q), 4.37(2H, q), 4.09(2H, t), 3.32(2H, q), 3.26(2H, t), 2.98(2H, q), 2.75(4H, q), 2.62(2H, q), 2.05(3H, s), 1.65(3H, t), 1.52(3H, t), 1.41(3H, t), 1.18(3H, t), 1.03(3H, t) |
| 20 | 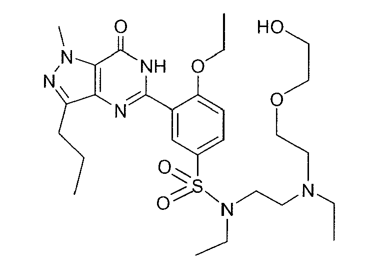 | 1-methyl-5-{2-ethoxy-5-{[1-ethyl-1-[2-[1-ethyl-1-(2-hydroxyethoxyethyl)]amino]ethyl]amidosulfonyl}p henyl}-3-n-propyl-1,6-dihydro-7H-pyrazolo[4,3-d]pyrimidin-7-one 10.84 (1H, s), 8.85 ( 1H, d ) , 7.91 (1H, dd), 7.12 (1H, d), 4.36 (2H, q), 4.27 (3H, s), 3.66 (2H, m), 3.57 (4H, m), 3.31 (2H, m), 3.24 (2H, t), 2.92 (2H, m), 2.77 (2H, t), 2.68 (2H, t), 2.60 (2H, m), 1.85 (2H, m), 1.63 (3H, t), 1.03 (3H, t). |
| 21 | 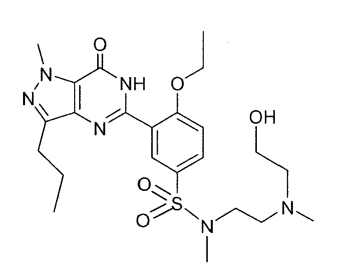 | 1-methyl-5-{2-ethoxy-5-{[1-methyl-1-[2-[1-methyl-1-(2-hydroxyethyl)]amino]ethyl]amidosulfonyl}phenyl} -3-n-propyl-1,6-dihydro-7H-pyrazolo[4,3-d]pyrimidin-7-one 10.83 (1H, s), 8.84 ( 1H, d) , 7.89 (1H, dd), 7.14 (1H, d), 4.37 (2H, m), 4.27 (3H, s), 3.56 (4H, m), 2.91 (2H, t), 2.83 (3H, s), 2.66 (2H, t), 2.58 (2H, t), 2.33 (3H, s), 1.84 (2H, m), 1.64 (3H, t), 1.03(3H, t). |
| 22 | 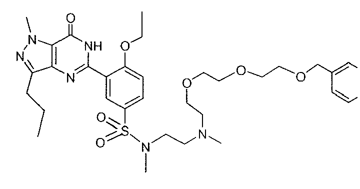 | 1-methyl-5-{2-ethoxy-5-{[1-methyl-1-[2-[1-methyl-1-(2-benzyloxyethoxyethoxyethyl)]amino]ethyl]amidos ulfonyl}phenyl}-3-n-propyl-1,6-dihydro-7H-pyrazolo[4,3-d]pyrimidin-7-one 8.81 ( 1H, d ) , 7.86 (1H, dd), 7.30 (5H, m), 7.12 (1H, d), 4.53 (2H, s), 4.33 (2H, m), 4.27 (3H, s), 3.62 (8H, m), 3.54 (2H, t), 3.18(2H, t), 2.92 (2H, t), 2.83 (3H, s), 2.62 (4H, m), 2.28 (3H, s), 1.85 (2H, m), 1.61 (3H, t), 1.01(3H, t). |
| 23 | 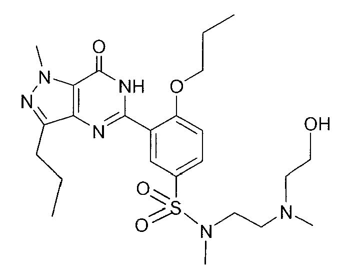 | 1-methyl-5-{2-propoxy-5-{[1-methyl-1-[2-[1-methyl-1-(2-hydroxyethyl)]amino]ethyl]amidosulfonyl}phenyl} -3-n-propyl-1,6-dihydro-7H-pyrazolo[4,3-d]pyrimidin-7-one 10.87(1H, s), 8.85(1H, d), 7.89(1H, dd), 7.16(1H, d), 4.28(3H, s), 4.27(2H, q), 3.58(2H, t), 3.24(2H, t), 2.93(2H, t), 2.83(3H, s), 2.67(2H, t), 2.59(2H, t), 2.34(3H, s), 2.04(2H, m), 1.86(2H, m), 1.19(3H, t), 1.02(3H, t) |
| 24 | 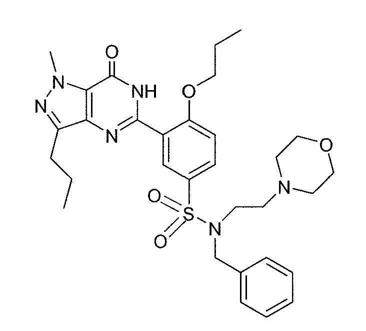 | 1-methyl-5-{2-propoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one 7.98 (2H, m), 7.35 (6H, m), 4.37 (2H, s), 4.16 (3H, s), 4.11 (2H, t), 3.86 (2H, t), 3.68 (2H, t), 3.57 (2H, t), 3.25 (2H, t), 3.10 (2H, t), 2.95 (2H, t), 2.77 (2H, t), 1.68-1.76 (4H, m), 0.89-0.96 (6H, 2×t). (DMSO) |
| 25 | 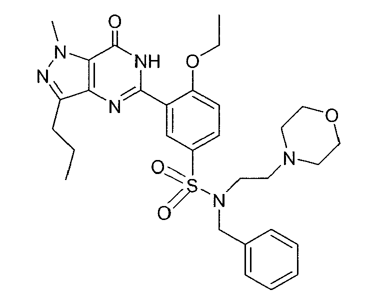 | 1-methyl-5-{2-ethoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one 8.01 (1H, dd), 7.96 (1H, d), 7.34 (6H, m), 4.38 (2H, s), 4.12 (3H, s), 3.88 (2H, t), 3.67 (2H, t), 3.55 (2H, t), 3.26 (2H, t), 3.11 (2H, t), 2.97 (2H, t), 2.78 (2H, t), 1.71 (2H, m), 0.92 (3H, t). (DMSO) |
| 26 | 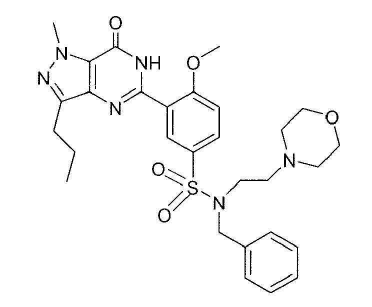 | 1-methyl-5-{2-methoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one 8.03 (1H, dd), 7.95 (1H, d), 7.32 (6H, m), 4.37 (2H, s), 4.15 (3H, s), 3.92 (3H, s), 3.86 (2H, t), 3.65 (2H, t), 3.53 (2H, t), 3.26 (2H, t), 3.09 (2H, t), 2.98 (2H, t), 2.77 (2H, t), 1.72 (2H, m), 0.92 (3H, t). (DMSO) |
| 27 | 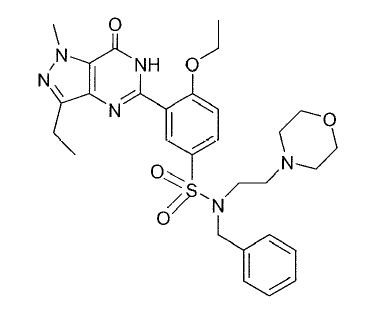 | 1-methyl-5-{2-ethoxy-5-[[1-benzyl-1-(2-morpholin-1-yl)ethyl]amidosulfonyl]phenyl}-3-ethyl-1,6-dihydro-7H-pyrazolo[4,3-d]pyrimidin-7-one 10.84 (1H, s), 8.94 (1H, d), 7.95 (1H, dd), 7.29 (5H, m), 7.14 (1H, d), 4.44 (2H, s), 4.28 (3H, s), 3.55 (2H, t), 3.28 (2H, t), 2.97 (2H, q), 2.36(2H, t), 2.25 (4H, t), 1.65 (3H, t), 1.25 (3H, t) |
| 28 | 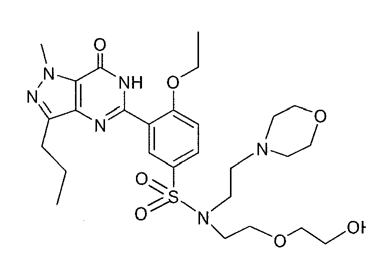 | 1-methyl-5-{2-ethoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one 8.84 (1H , d), 7.90 (1H, dd), 7.13 (1H, d), 4.37 (2H, m), 4.27 (3H, s), 3.65 (8H, m), 3.49 (4H, m), 3.43 (2H, t), 2.94 (2H, t), 2.64 (4H, d), 2.46 (2H, t), 1.85 (2H, m), 1.64 (3H, t), 1.01 (3H, t) (DMSO). |
| 29 | 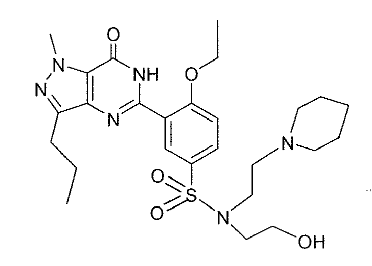 | 1-methyl-5-{2-ethoxy-5-[[1-(2-hydroxyethyl)-1-(2-piperidin-1-yl)ethyl]amidosulfonyl]phenyl}-3-n-propyl-1,6-dihydro-7H-pyrazolo[4,3-d]pyrimidin-7-one 10.84(1H, s), 8.86(1H, d), 7.89(1H, dd), 7.15(1H, d), 4.37(2H, q), 4.27(3H, s), 3.68(2H, t), 3.39(2H, t), 2.93(2H, t), 2.69(2H, t), 2.49(2H, t), 2.41(4H, br s), 1.85(2H, m), 1.64(3H, t), 1.62(4H, m), 1.44(2H, m), 1.02(3H, t) |
| 30 | 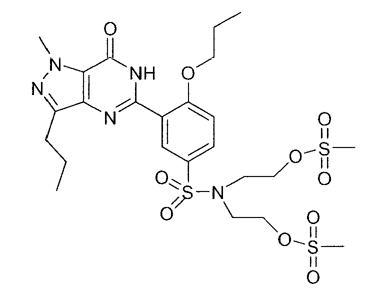 | 1-methyl-5-{2-ethoxy-5-[bis(2-methylsulfonylethyl)amidosulfonyl]phenyl}-3-n-propyl-1,6-dihydro-7H-pyrazolo[4,3-d]pyrimidin-7-one 10.80 (1H, s), 8.22 (1H, d), 7.92 (1H, dd), 7.21 (1H, d), 4.43 (4H, t), 4.27 (3H, s), 4.27(2H, t), 3.59 (4H, t), 3.07 (6H, s), 2.94 (2H, t), 2.04 (2H, m), 1.87 (2H, m), 1.19 (3H, t), 1.04 (3H, t). |
| 31 | 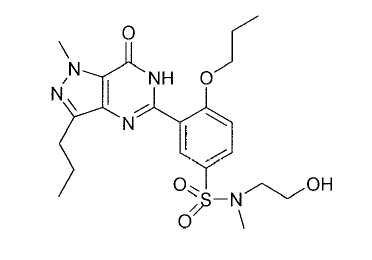 | 1-methyl-5-{2-propoxy-5-[[1-methyl-1-(2-hydroxyethyl)]amidosulfonyl]phenyl}-3-n-propyl-1,6-dihydro-7H-pyrazolo[4,3-d]pyrimidin-7-one 10.83 (1H, s), 8.84(1H,d), 7.90 (1H, dd), 7.16 (1H, d), 4.27 (3H, s), 4.26 (2H, t), 3.81 (2H, t), 3.25(2H, t), 2.93 (2H, t), 2.90 (3H, t), 2.11 (1H, t), 2.05 (2H, m), 1.85 (2H, m), 1.18 (3H, t), 1.03(3H, t) |
| 32 | 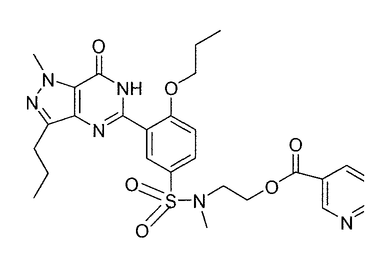 | 1-methyl-5-{2-propoxy-5-[[1-methyl-1-(2-nicotinoyloxyethyl)]amidosulfonyl]phenyl}-3-n-propyl-1,6-dihydro-7H-pyrazolo[4,3-d]pyrimidin-7-one 10.93 (1H, s), 9.05 (1H, dd), 8.83 (1H, d), 8.78 (1H, dd), 8.28 (1H, dt), 7.89 (1H, dd), 7.38(1H, m), 7.12 (1H, d), 4.53 (2H, t), 4.27 (3H, s), 4.21 (2H, t), 3.55 (2H, t), 2.98 (3H, s), 2.92 (2H, t), 2.02 (2H, t), 1.86 (2H, m), 1.17 (3H, t), 1.01(3H, t) |
| 33 | 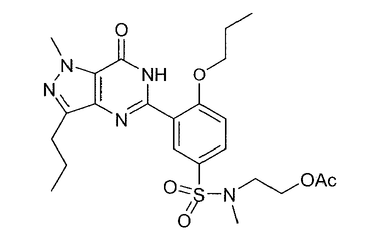 | 1-methyl-5-{2-propoxy-5-[[1-methyl-1-(2-acetoxyethyl)]amidosulfonyl]phenyl}-3-n-propyl-1,6-dihydro-7H-pyrazolo[4,3-d]pyrimidin-7-one 10.84 (1H, s), 8.86 (1H,d), 7.88 (1H, dd), 7.16 (1H, d), 4.27 (3H, s), 4.24 (4H, m), 3.35 (2H, t), 2.92 (2H, t), 2.90 (3H, t), 2.07 (3H, s), 2.03 (2H, t), 1.86 (2H, m), 1.18 (3H, t), 1.02 (3H, t) |
| 34 | 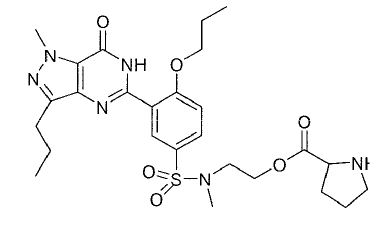 | 1-methyl-5-{2-propoxy-5-[[1-methyl-1-(2-(pyrrolidin-2-yl)formoxylethyl)]amidosulfonyl]phenyl}-3-n-propyl-1,6-dihydro-7H-pyrazolo[4,3-d]pyrimidin-7-one 8.75 (1H,d), 7.88 (1H, dd), 7.14 (1H, d), 4.30(2H, t), 4.27 (3H, s), 4.23 (2H, t), 3.68 (1H, m), 3.37(2H, t), 3.08 (1H, m), 2.92 (2H, m), 2.90(3H, t), 2.12 (2H, m), 2.01 (2H, q), 1.88 (2H, m), 1.80 (2H, m), 1.75 (2H, m), 1.16 (3H, t), 1.10 (3H, t) |
| 35 | 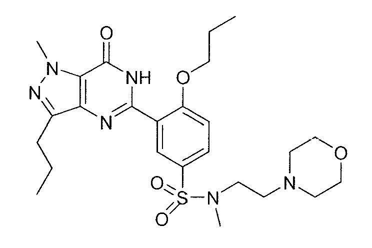 | 1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one 10.86 (1H, s), 8.86 (1H,d), 7.89 (1H, dd), 7.15 (1H, d), 4.27 (3H, s), 4.25 (2H, t), 3.67 (4H, t), 3.21 (2H, t), 2.91 (2H, m), 2.87 (3H, s), 2.58 (2H, t), 2.47 (4H, t), 2.03 (2H, m), 1.84 (2H, m), 1.18 (3H, t), 1.02 (3H, t) |
| 36 | 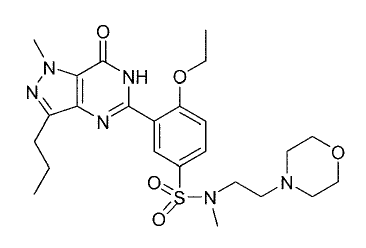 | 1-methyl-5-{2-ethoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one 10.82 (1H, s), 8.85 (1H,d), 7.89 (1H, dd), 7.14 (1H, d), 4.37 (2H, q), 4.27 (3H, s), 3.68 (4H, t), 3.22 (2H, t), 2.92 (2H, t), 2.87 (3H, s), 2.58 (2H, t), 2.48 (4H, t), 1.84 (2H, m), 1.64 (3H, t), 1.02 (3H, t) |
| 37 | 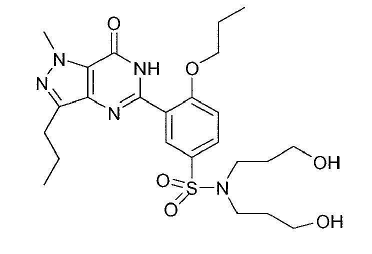 | 1-methyl-5-{2-propoxy-5-[bis(3-hydroxypropyl)amidosulfonyl]phenyl}-3-ethyl-1,6-dihydro-7H-pyrazolo[4,3-d]pyrimidin-7-one 10.75 (1H, s), 8.80 (1H, d), 7.92 (1H, dd), 7.14 (1H, d), 4.27 (3H, s), 4.25 (2H, t), 3.71 (4H, t), 3.37 (4H, t), 2.93 (2H, t), 2.55 (2H, s), 2.04 (2H, m), 1.82 (6H, m), 1.17 (3H, t), 1.02 (3H, t) |
| 38 | 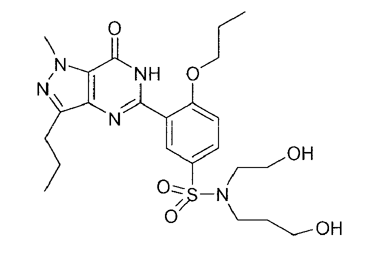 | 1-methyl-5-{2-propoxy-5-[[1-(2-hydroxyethyl-1-(3-hydroxypropyl)]amidosulfonyl]phenyl}-3-n-propyl-1,6-dihydro-7H-pyrazolo[4,3-d]pyrimidin-7-one 10.78 (1H, s), 8.84 (1H, d), 7.95 (1H, dd), 7.15 (1H, d), 4.27 (3H, s), 4.25 (2H, t), 3.81 (4H, t), 3.37 (4H, m), 2.93 (3H, t), 2.73 (1H, s), 2.03 (2H, m), 1.85 (4H, m), 1.18 (3H, t), 1.02 (3H, t) |
| 39 | 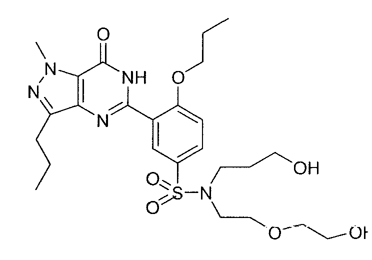 | 1-methyl-5-{2-propoxy-5-[[1-(2-hydroxyethoxyethyl-1-(3-hydroxypropyl)]amidosulfonyl]phenyl}-3-n-propyl-1,6-dihydro-7H-pyrazolo[4,3-d]pyrimidin-7-one 10.80 (1H, s), 8.82 (1H, d), 7.92 (1H, dd), 7.14 (1H, d), 4.27 (3H, s), 4.25 (2H, t), 3.75 (2H, t), 3.68 (4H, t), 3.53 (2H, t), 3.42 (2H, t), 3.37 (2H, t), 2.92 (2H, t), 2.69 (2H, s), 2.02 (2H, m), 1.87 (4H, m), 1.17 (3H, t), 1.02 (3H, t) |
| 40 | 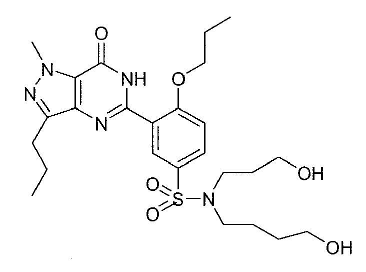 | 1-methyl-5-{2-propoxy-5-[[1-(4-hydroxybutyl)-1-(3-hydroxypropyl)]amidosulfonyl]phenyl}-3-n-propyl-1,6-dihydro-7H-pyrazolo[4,3-d]pyrimidin-7-one 10.78 (1H, s), 8.78 (1H, d), 7.89 (1H, dd), 7.13 (1H, d), 4.27 (3H, s), 4.24 (2H, t), 3.74 (2H, t), 3.66 (2H, t), 3.34 (2H, t), 3.25 (2H, t), 3.01 (1H, s), 2.92 (2H, t), 2.58 (1H, s), 2.02 (2H, m), 1.82 (4H, m), 1.60 (4H, m), 1.16 (3H, t), 1.02 (3H, t)) |
| 41 | 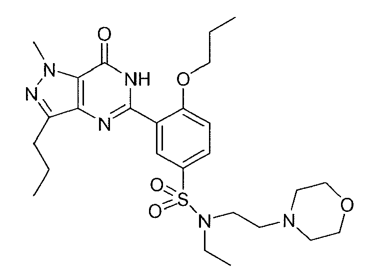 | 1-methyl-5-{2-propoxy-5-[[1-ethyl-1-(2-morpholin-1-yl)ethyl]amidosulfonyl]phenyl}-3-propyl-1,6-dihydro-7H-pyrazolo[4,3-d]pyrimidin-7-one 10.88 (1H, s), 8.89 (1H,d), 7.92 (1H, dd), 7.12 (1H, d), 4.27 (3H, s), 4.25 (2H, t), 3.65 (4H, t), 3.33 (4H, m), 2.92 (2H, t), 2.58 (2H, t), 2.47 (4H, t), 2.03(2H, m), 1.85 (2H, m), 1.19 (3H, t), 1.18 (3H, t), 1.02 (3H, t) |
| 42 | 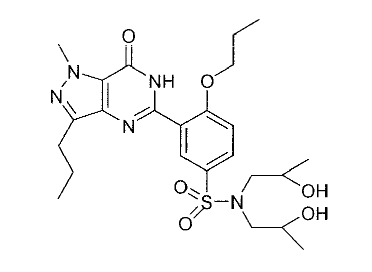 | 1-methyl-5-{2-propoxy-5-[bis(2-hydroxypropyl)amidosulfonyl]phenyl}-3-propyl-1,6-dihydro-7H-pyrazolo[4,3-d]pyrimidin-7-one 10.84 (1H, s), 8.89 (1H, d), 7.92 (1H, dd), 7.16 (1H, d), 4.27 (3H, s), 4.26(2H, t), 4.18(2H, m), 3.12∼3.49 (m, 4H), 2.05 (2H, m), 1.85 (2H, m), 1.19 (9H, m), 1.02(3H, t) |
| 43 | 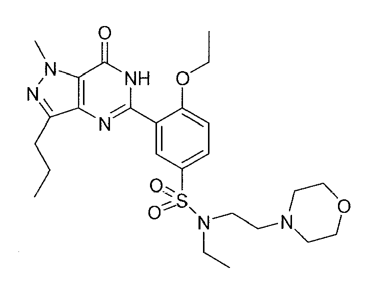 | 1-methyl-5-{2-ethoxy-5-[[1-ethyl-1-(2-morpholin-1-yl)ethyl]amidosulfonyl]phenyl}-3-n-propyl-1,6-dihydro-7H-pyrazolo[4,3-d]pyrimidin-7-one 10.84 (1H, s), 8.88 (1H,d), 7.92 (1H, dd), 7.12 (1H, d), 4.36 (2H, q), 4.27 (3H, s), 3.65 (4H, t), 3.33 (4H, m), 2.92 (2H, t), 2.58 (2H, t), 2.46 (4H, t), 1.85 (2H, m), 1.63 (3H, t), 1.18 (3H, t), 1.02 (3H, t) |
| 44 | 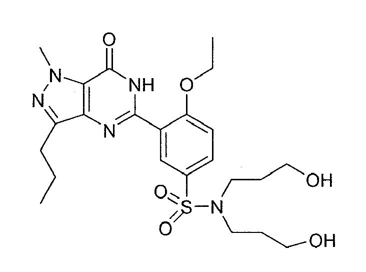 | 1-methyl-5-{2-ethoxy-5-[bis(3-hydroxypropyl)amidosulfonyl]phenyl}-3-n-propyl-1,6-dihydro-7H-pyrazolo[4,3-d]pyrimidin-7-one 10.71 (1H, s), 8.79 (1H, d), 7.92 (1H, dd), 7.13 (1H, d), 4.36(2H, q), 4.27 (3H, s), 3.70 (4H, t), 3.38 (4H, t), 2.93 (2H, t), 2.55 (2H, t), 1.80 (6H, m), 1.62 (3H, t), 1.02 (3H, t) |
| 45 | 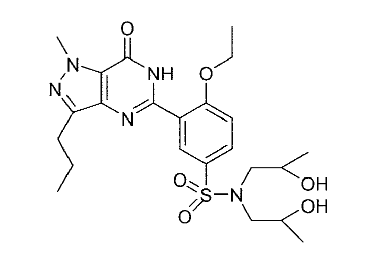 | 1-methyl-5-{2-ethoxy-5-[bis(2-hydroxypropyl)amidosulfonyl]phenyl}-3-propyl-1,6-dihydro-7H-pyrazolo[4,3-d]pyrimidin-7-one 10.84(1H, s), 8.86(1H, d), 7.91(1H, dd), 7.15(1H, d), 4.28 (3H, s), 4.27(2H, t), 4.19(2H, m), 3.09∼3.48 (m, 4H), 2.92 (2H, t), 1.84 (2H, m), 1.63(3H, t), 1.21 (3H, d), 1.16 (3H, d), 1.00(3H, t) |
| 46 | 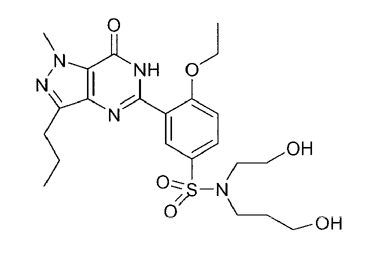 | 1-methyl-5-{2-ethoxy-5-[[1-(2-hydroxyethyl)-1-(3-hydroxypropyl)]amidosulfonyl]phenyl}-3-n-propyl-1,6-dihydro-7H-pyrazolo[4,3-d]pyrimidin-7-one 10.76 (1H, s), 8.81 (1H, d), 7.93 (1H, dd), 7.14 (1H, d), 4.36 (2H, q), 4.27 (3H, s), 3.81 (4H, t), 3.36 (4H, m), 3.04 (1H, s), 2.93 (2H, t), 2.80 (1H, s), 1.84 (4H, m), 1.63 (3H, t), 1.02 (3H, t) |
| 47 | 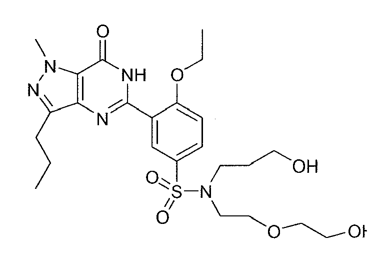 | 1-methyl-5-{2-ethoxy-5-[[1-(2-hydroxyethoxyethyl)-1-(3-hydroxypropyl)]amidosulfonyl]phenyl}-3-n-propyl-1,6-dihydro-7H-pyrazolo[4,3-d]pyrimidin-7-one 10.76 (1H, s), 8.81 (1H, d), 7.92 (1H, dd), 7.14 (1H, d), 4.36 (2H, q), 4.27 (3H, s), 3.75 (2H, t), 3.68 (4H, t), 3.53 (2H, t), 3.43 (2H, t), 3.38 (2H, t), 2.93 (2H, t), 2.68 (2H, s), 1.86 (4H, m), 1.63 (3H, t), 1.02 (3H, t) |
| 48 | 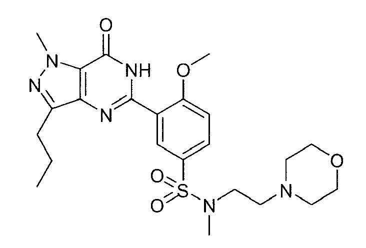 | 1-methyl-5-{2-methoxy-5-[[1-methyl-1-(2-morpholin-1-yl)ethyl]amidosulfonyl]phenyl}-3-n-propyl-1,6-dihydro-7H-pyrazolo[4,3-d]pyrimidin-7-one 10.63 (1H, s), 8.84 (1H,d), 7.91 (1H, dd), 7.17 (1H, d), 4.27 (3H, s), 4.13 (3H, s), 3.67 (4H, t), 3.22 (2H, t), 2.92 (2H, t), 2.87 (3H, s), 2.58 (2H, t), 2.48 (4H, t), 1.85 (2H, m), 1.01 (3H, t) |
| 49 | 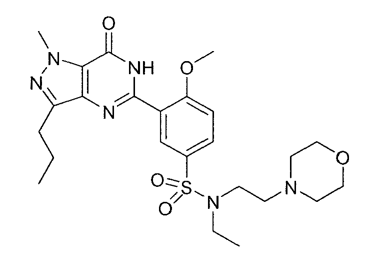 | 1-methyl-5-{2-methoxy-5-[[1-ethyl-1-(2-morpholin-1-yl)ethyl]amidosulfonyl]phenyl}-3-n-propyl-1,6-dihydro-7H-pyrazolo[4,3-d]pyrimidin-7-one 10.65 (1H, s), 8.88 (1H,d), 7.95 (1H, dd), 7.15 (1H, d), 4.27 (3H, s), 4.12 (3H, s), 3.65 (4H, t), 3.32 (4H, m), 2.92 (2H, t), 2.58 (2H, t), 2.46 (4H, t), 1.85 (2H, m), 1.19 (3H, t), 1.01 (3H, t) |
| 50 | 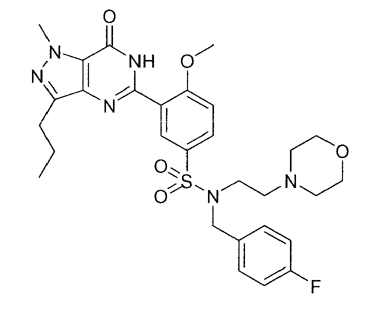 | 1-methyl-5-{2-methoxy-5-[[1-(4-fluorobenzyl)-1-(2-morpholin-1-yl)ethyl]amidosulfonyl]phenyl}-3-n-propyl-1,6-dihydro-7H-pyrazolo[4,3-d]pyrimidin-7-one 10.65 (1H, s), 8.91 (1H, d), 7.96 (1H, dd), 7.31 (2H, m), 7.16 (1H, d), 7.01 (2H, m), 4.41 (2H, s), 4.28 (3H, s), 4.14 (3H, s), 3.56 (4H, t), 3.27 (2H, t), 2.91 (2H, t), 2.33 (2H, t), 2.26 (4H, t), 1.84 (2H, m), 0.99 (3H, t). |
| 51 | 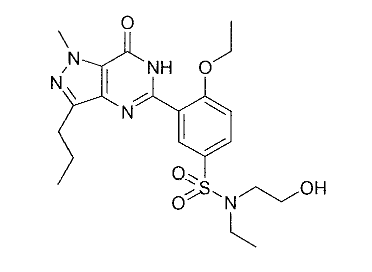 | 1-methyl-5-{2-ethoxy-5-[[1-ethyl-1-(2-hydroxyethyl)]amidosulfonyl]phenyl}-3-n-propyl-1,6-dihydro-7H-pyrazolo[4,3-d]pyrimidin-7-one 10.82 (1H, s), 8.87 (1H,d), 7.92 (1H, dd), 7.13 (1H, d), 4.36 (2H, q), 4.27 (3H, s), 3.80 (2H, q), 3.35(4H, m), 2.92(2H, t), 2.38 (1H, t), 1.85 (2H, m), 1.63 (3H, t), 1.20 (3H, t), 1.02 (3H, t) |
| 52 | 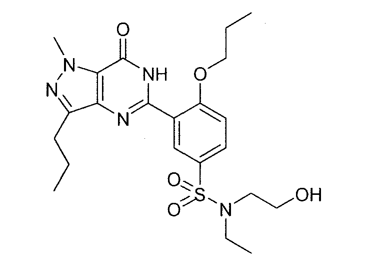 | 1-methyl-5-{2-propoxy-5-[[1-ethyl-1-(2-hydroxyethyl)]amidosulfonyl]phenyl}-3-n-propyl-1,6-dihydro-7H-pyrazolo[4,3-d]pyrimidin-7-one 10.86 (1H, s), 8.88 (1H,d), 7.93 (1H, dd), 7.14 (1H, d), 4.27 (3H, s), 4.25 (2H, t), 3.81 (2H, t), 3.36 (4H, m), 2.94 (2H, t), 2.02 (3H, m), 1.86 (2H, m), 1.21 (3H, t), 1.18 (3H, t), 1.02 (3H, t) |
| 53 | 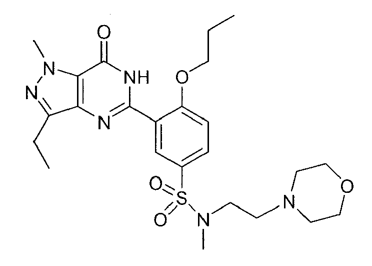 | 1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one 10.63 (1H, s), 8.86 (1H,d), 7.89 (1H, dd), 7.15 (1H, d), 4.27 (3H, s), 4.26 (2H, t), 3.70 (4H, t), 3.26 (2H, t), 2.97 (2H, q), 2.87 (3H, s), 2.63 (2H, t), 2.54 (4H, t), 2.04 (2H, m), 1.41 (3H, t), 1.18 (3H, t) |
| 54 | 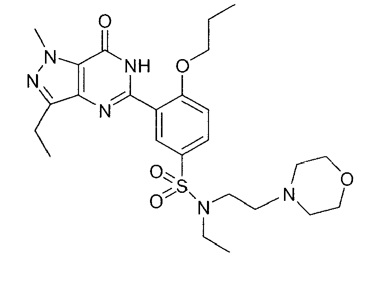 | 1-methyl-5-{2-propoxy-5-[[1-ethyl-1-(2-morpholin-1-yl)ethyl]amidosulfonyl]phenyl}-3-ethyl-1,6-dihydro-7H-pyrazolo[4,3-d]pyrimidin-7-one 10.88 (1H, s), 8.90 (1H,d), 7.92 (1H, dd), 7.13 (1H, d), 4.27 (3H, s), 4.25 (2H, t), 3.69 (4H, t), 3.34 (4H, m), 2.97 (2H, q), 2.63 (2H, t), 2.52 (4H, t), 2.04 (2H, m), 1.41 (3H, t), 1.19 (3H, t), 1.18 (3H, t) |
| 55 | 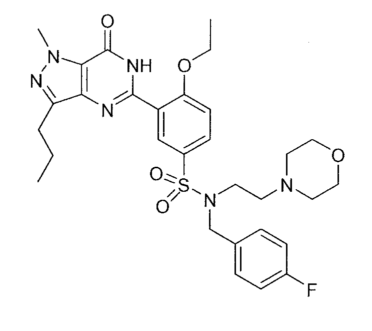 | 1-methyl-5-{2-ethoxy-5-[[1-(4-fluorobenzyl)-1-(2-morpholin-1-yl)ethyl]amidosulfonyl]phenyl}-3-n-propyl-1,6-dihydro-7H-pyrazolo[4,3-d]pyrimidin-7-one 10.83 (1H, s), 8.92 (1H, d), 7.94 (1H, dd), 7.31 (2H, m), 7.14 (1H, d), 7.01 (2H, m), 4.42 (2H, s), 4.37 (2H, q), 4.28 (3H, s), 3.56 (4H, t), 3.27 (2H, t), 2.92 (2H, t), 2.33 (2H, t), 2.26 (4H, t), 1.85 (2H, m), 1.65 (3H, t), 1.00 (3H, t). |
| 56 | 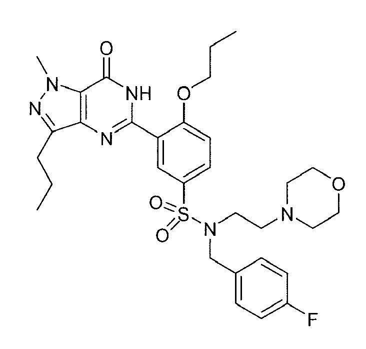 | 1-methyl-5-{2-propoxy-5-[[1-(4-fluorobenzyl)-1-(2-morpholin-1-yl)ethyl]amidosulfonyl]phenyl}-3-n-propyl-1,6-dihydro-7H-pyrazolo[4,3-d]pyrimidin-7-one 10.86 (1H, s), 8.92 (1H, d), 7.92 (1H, dd), 7.33 (2H, m), 7.16 (1H, d), 7.02 (2H, m), 4.41 (2H, s), 4.28 (3H, s), 4.27 (2H, t), 3.61 (4H, t), 3.31 (2H, t), 2.92 (2H, t), 2.39 (2H, t), 2.31 (4H, t), 2.05 (2H, m), 1.85 (2H, m), 1.19 (3H, t), 1.00 (3H, t). |
| 57 | 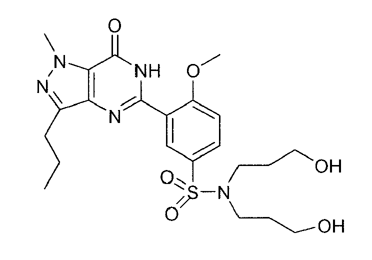 | 1-methyl-5-{2-ethoxy-5-[bis(3-hydroxypropyl)amidosulfonyl]phenyl}-3-n-propyl-1,6-dihydro-7H-pyrazolo[4,3-d]pyrimidin-7-one 10.56 (1H, s), 8.77 (1H, d), 7.94 (1H, dd), 7.15 (1H, d), 4.27 (3H, s), 4.12 (3H, s), 3.70 (4H, t), 3.37 (4H, t), 2.93 (2H, t), 2.15 (2H, s), 1.80 (6H, m), 1.01 (3H, t) |
| 58 | 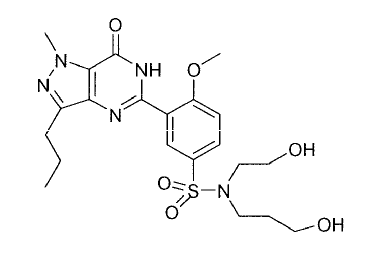 | 1-methyl-5-{2-methoxy-5-[[1-(2-hydroxyethyl)-1-(3-hydroxypropyl)]amidosulfonyl]phenyl}-3-n-propyl-1,6-dihydro-7H-pyrazolo[4,3-d]pyrimidin-7-one 10.52 (1H, s), 8.84 (1H, d), 7.95 (1H, dd), 7.17 (1H, d), 4.27 (3H, s), 4.13 (3H, s), 3.82 (4H, t), 3.38 (4H, m), 2.93 (2H, t), 2.88 (1H, s), 2.73 (1H, s), 1.85 (4H, m), 1.02 (3H, t) |
| 59 | 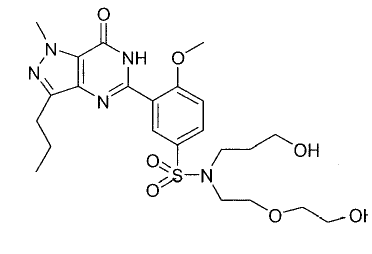 | 1-methyl-5-{2-methoxy-5-[[1-(2-hydroxyethoxyethyl)-1-(3-hydroxypropyl)]amidosulfonyl]phenyl}-3-n-propyl-1,6-dihydro-7H-pyrazolo[4,3-d]pyrimidin-7-one 10.57 (1H, s), 8.81 (1H, d), 7.95 (1H, dd), 7.16 (1H, d), 4.27 (3H, s), 4.12 (3H, s), 3.75 (2H, t), 3.68 (4H, t), 3.52 (2H, t), 3.43 (2H, t), 3.38 (2H, t), 2.92 (2H, t), 2.63 (2H, s), 1.86 (4H, m), 1.02 (3H, t) |
| 60 | 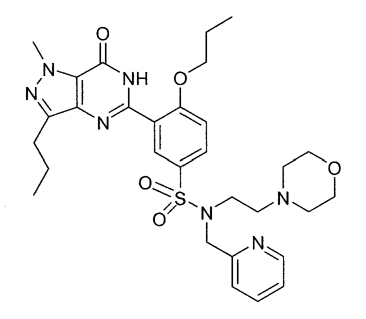 | 1-methyl-5-{2-propoxy-5-[[1-(2-pyridylmethyl)-1-(2-morpholin-1-yl)ethyl]amidosulfonyl]phenyl}-3-n-propyl-1,6-dihydro-7H-pyrazolo[4,3-d]pyrimidin-7-one 10.87 (1H, s), 8.91 (1H, d), 7.92 (1H, dd), 8.45 (1H, d), 7.94 (1H, d), 7.68 (1H, t), 7.60 (1H, d), 7.18 (1H, t), 7,16 (1H, d), 4.56 (2H, s), 4.28 (3H, s), 4.26 (2H, t), 3.58 (4H, t), 3.42 (2H, t), 2.92 (2H, t), 2.45 (2H, t), 2.34 (4H, t), 2.04 (2H, m), 1.85 (2H, m), 1.19 (3H, t), 1.01 (3H, t). |
| 61 | 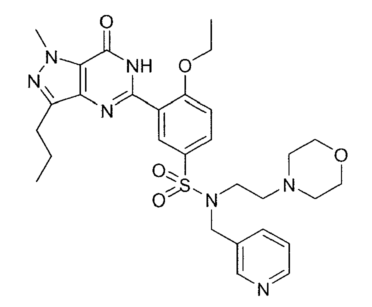 | 1-methyl-5-{2-ethoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one 10.84 (1H, s), 8.91 (1H, d), 8.54 (1H, dd), 8.48 (1H, d), 7.95 (1H, dd), 7.84 (1H, d), 7.31 (1H, dd), 7.15 (1H, d), 4.48 (2H, s), 4.38 (2H, q), 4.28 (3H, s), 3.56 (4H, t), 3.32 (2H, t), 2.92 (2H, t), 2.38 (2H, t), 2.28 (4H, t), 1.85 (2H, m), 1.65 (3H, t), 1.00 (3H, t). |
| 62 | 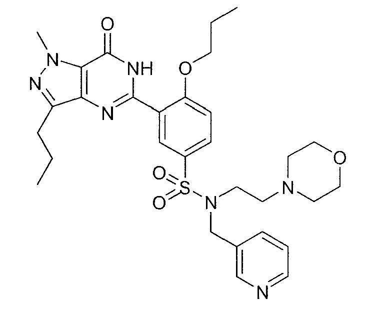 | 1-methyl-5-{2-propoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one 10.88 (1H, s), 8.92 (1H, d), 8.54 (1H, d), 8.49 (1H, s), 7.94 (1H, dd), 7.82 (1H, d), 7.30(1H, dd), 7.16(1H, d), 4.47 (2H, s), 4.28 (3H, s), 4.27 (2H, t), 3.60 (4H, t), 3.34 (2H, t), 2.92 (2H, t), 2.41 (2H, t), 2.32 (4H, t), 2.05 (2H, m), 1.85 (2H, m), 1.19 (3H, t), 1.00 (3H, t). |
| 63 | 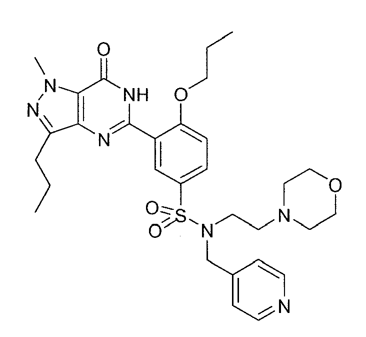 | 1-methyl-5-{2-propoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one 10.87 (1H, s), 8.91 (1H, d), 8.56 (2H, d), 7.93 (1H, dd), 7.29 (2H, d), 7.15(1H, d), 4.48 (2H, s), 4.27 (3H, s), 4.26 (2H, t), 3.55 (4H, t), 3.33 (2H, t), 2.92 (2H, t), 2.38 (2H, t), 2.26 (4H, t), 2.04 (2H, m), 1.86 (2H, m), 1.19 (3H, t), 1.01 (3H, t) |
| 64 | 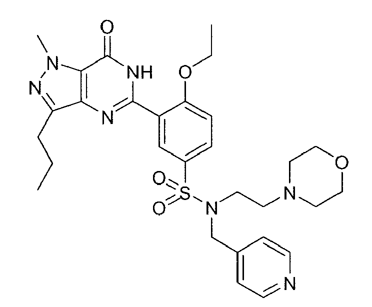 | 1-methyl-5-{2-ethoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one 10.83 (1H, s), 8.92 (1H, d), 8.56 (2H, dd), 7.93(1H, dd), 7.29(2H, d), 7.14 (1H, d), 4.49 (2H, s), 4.38 (2H, q), 4.28 (3H, s), 3.55 (4H, t), 3.34 (2H, t), 2.92 (2H, t), 2.39 (2H, t), 2.26 (4H, t), 1.82 (2H, m), 1.65 (3H, t), 1.01 (3H, t) |
| 65 | 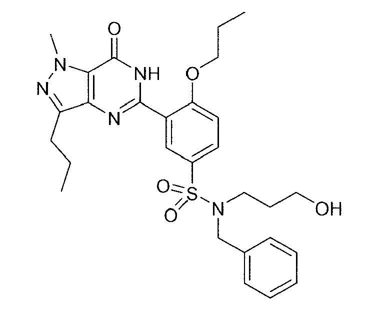 | 1-methyl-5-{2-propoxy-5-[[1-benzyl-1-(3-hydroxypropyl)]amidosulfonyl]phenyl}-3-n-propyl-1,6-dihydro-7H-pyrazolo[4,3-d]pyrimidin-7-one 10.80(1H, s), 8.89(1H, d), 7.91 (1H, dd), 7.27-7.32(5H, m), 7.14 (1H, d), 4.38 (2H, s), 4.27 (s, 3H), 4.26 (2H, t), 3.55 (2H, t), 3.33 (2H, t), 2.92 (2H, t), 2.03 (2H, m), 1.83 (2H, m), 1.50 (2H, m), 1.18 (3H, t), 1.03(3H, t) |
| 66 | 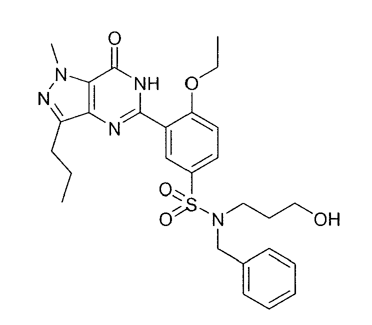 | 1-methyl-5-{2-ethoxy-5-[[1-benzyl-1-(3-hydroxypropyl)]amidosulfonyl]phenyl}-3-n-propyl-1,6-dihydro-7H-pyrazolo[4,3-d]pyrimidin-7-one 10.80(1H, s), 8.87(1H,d), 7.92 (1H, dd), 7.28-7.33(5H, m), 7.14 (1H, d), 4.39 (2H, s), 4.36 (2H, t), 4.28 (s, 3H), 3.55 (2H, t), 3.33 (2H, t), 2.92 (2H, t), 1.83 (2H, m), 1.64 (3H, t), 1.50(2H, m), 0.99(3H, t) |
| 67 | 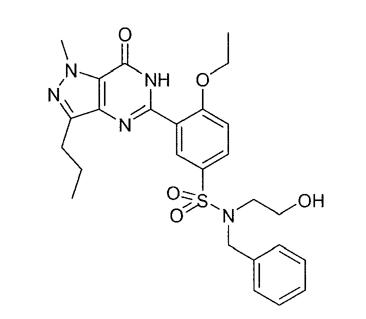 | 1-methyl-5-{2-ethoxy-5-[[1-benzyl-1-(2-hydroxyethyl)]amidosulfonyl]phenyl}-3-n-propyl-1,6-dihydro-7H-pyrazolo[4,3-d]pyrimidin-7-one 10.80 (1H, s), 8.92 (1H, d), 7.94 (1H, dd), 7.28-7.34 (5H, m), 7.14 (1H, d), 4.45 (2H, s), 4.36 (2H, t), 4.27 (s, 3H), 3.56 (2H, t), 3.32 (2H, t), 2.92 (2H, t), 1.85 (2H, m), 1.64 (3H, t), 0.99(3H, t) |
| 68 | 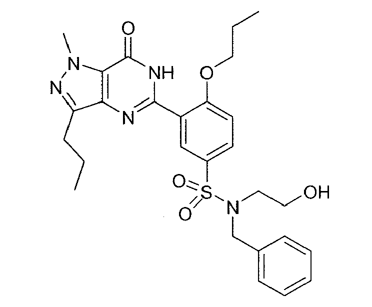 | 1-methyl-5-{2-propoxy-5-[[1-benzyl-1-(2-hydroxyethyl)]amidosulfonyl]phenyl}-3-n-propyl-1,6-dihydro-7H-pyrazolo[4,3-d]pyrimidin-7-one 10.81(1H, s), 8.94(1H, d), 7.95 (1H, dd), 7.28-7.34(5H, m), 7.15 (1H, d), 4.45 (2H, s), 4.27(s, 3H), 4.26 (2H, t), 3.57 (2H, t), 3.33(2H, t), 2.92 (2H, t), 2.03 (2H, m), 1.83 (2H, m), 1.18 (3H, t), 0.99 (3H, t) |
| 69 | 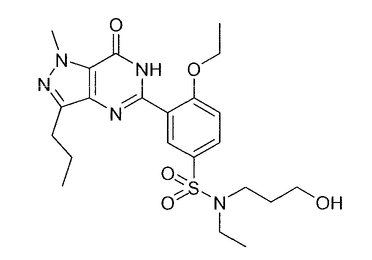 | 1-methyl-5-{2-ethoxy-5-[[1-ethyl-1-(3-hydroxypropyl)]amidosulfonyl]phenyl}-3-n-propyl-1,6-dihydro-7H-pyrazolo[4,3-d]pyrimidin-7-one 10.78 (1H, s), 8.85 (1H, d), 7.91 (1H, dd), 7.12 (1H, d), 4.36 (2H, q), 4.26 (s, 3H), 3.74 (2H, t), 3.34 (2H, t), 3.32 (2H, q), 2.92 (2H, t), 1.85 (2H, m), 1.78 (2H, m), 1.62 (3H, t), 1.18 (3H, t), 1.01 (3H, t) |
| 70 | 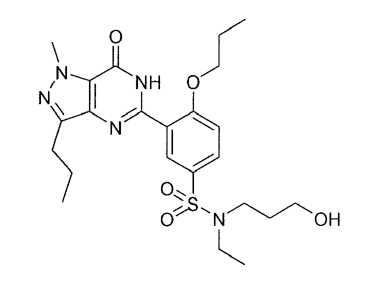 | 1-methyl-5-{2-propoxy-5-[[1-ethyl-1-(3-hydroxypropyl)]amidosulfonyl]phenyl}-3-n-propyl-1,6-dihydro-7H-pyrazolo[4,3-d]pyrimidin-7-one 10.82 (1H, s), 8.84 (1H, d), 7.90 (1H, dd), 7.13 (1H, d), 4.27(s, 3H), 4.24 (2H, t), 3.74 (2H, t), 3.35 (2H, t), 3.32 (2H, q), 2.93 (2H, t), 2.02 (2H, m), 1.85 (2H, m), 1.78 (2H, m), 1.18 (3H, t), 1.17 (3H, t), 1.02 (3H, t) |
| 71 | 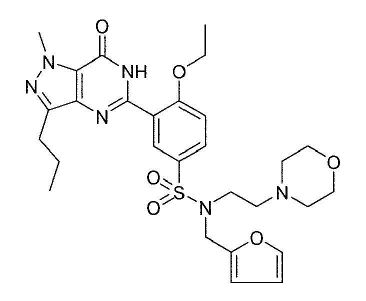 | 1-methyl-5-{2-ethoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7-one 10.80 (1H, s), 8.86 (1H, d), 7.86 (1H, dd), 7.25 (1H, m), 7.22 (1H, m), 7.07 (1H, d), 6.22 (2H, m), 4.55 (2H, s), 4.35 (2H, q), 4.27 (3H, s), 3.62 (4H, t), 3.34 (2H, t), 2.92 (2H, t), 2.53 (2H, t), 2.44 (4H, t), 1.85 (2H, m), 1.63 (3H, t), 1.00 (3H, t) |
| 72 | 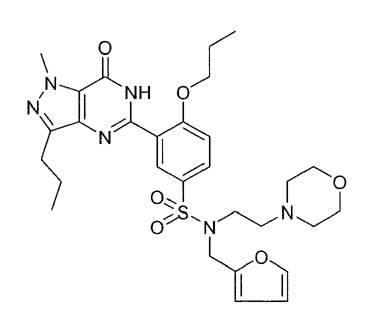 | 1-methyl-5- {2-propoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7-one 10.82 (1H, s), 8.87 (1H, d), 7.86 (1H, dd), 7.25 (1H, m), 7.22 (1H, m), 7.08 (1H, d), 6.22 (2H, m), 4.55 (2H, s), 4.27 (3H, s), 4.24 (2H, t), 3.66 (4H, t), 3.33 (2H, t), 2.92 (2H, t), 2.53 (2H, t), 2.44 (4H, t), 2.02 (2H, m), 1.85 (2H, m), 1.17 (3H, t), 1.00 (3H, t) |
| 73 | 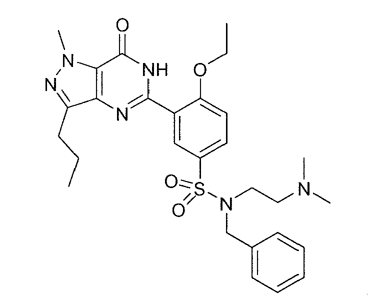 | 1-methyl-5-{2-ethoxy-5-[[1-benzyl-1-(2-dimethylaminoethyl)]amidosulfonyl]phenyl}-3-n-propyl-1,6-dihydro-7H-pyrazolo[4,3-d]pyrimidin-7-one 10.85 (1H, s), 8.94 (1H, d), 7.95 (1H, dd), 7.29-7.33 (5H, m), 7.14 (1H, d), 4.42 (2H, s), 4.38 (2H, q), 4.27 (s, 3H), 3.25 (2H, m), 2.92 (2H, t), 2.32 (2H, m), 2.08 (6H, s), 1.85 (2H, m), 1.65 (3H, t), 1.00 (3H, t) |
| 74 | 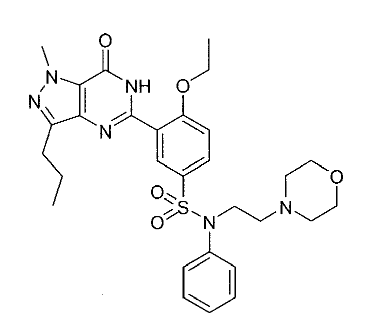 | 1-methyl-5-{2-ethoxy-5-[[1-phenyl-1-(2-morpholin-1-yl)ethyl]amidosulfonyl]phenyl}-3-ethyl-1,6-dihydro-7H-pyrazolo[4,3-d]pyrimidin-7-one 10.80 (1H, s), 8.71 (1H, d), 7.67 (1H, dd), 7.32 (3H, m), 7.15 (2H, m), 7.06 (1H, d), 4.36 (2H, q), 4.27 (3H, s), 3.76 (2H, t), 3.64 (4H, t), 2.88 (2H, t), 2.53 (2H, t), 2.44 (4H, t), 1.82 (2H, m), 1.64 (3H, t), 1.01 (3H, t) |
| 75 | 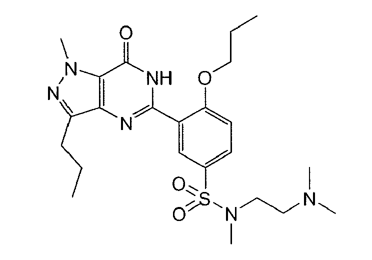 | 1-methyl-5-{2-propoxy-5-[[1-methyl-1-(2-dimethylaminoethyl)]amidosulfonyl]phenyl}-3-n-propyl-1,6-dihydro-7H-pyrazolo[4,3-d]pyrimidin-7-one 10.85 (1H, s), 8.86 (1H, d), 7.88 (1H, dd), 7.16 (1H, d), 4.27 (s, 3H), 4.26 (2H, t), 3.29 (2H, t), 2.92 (2H, t), 2.87 (3H, s), 2.72 (2H, t), 2.40 (6H, s), 2.04 (2H, m), 1.85 (2H, m), 1.18 (3H, t), 1.01 (3H, t) |
| 76 | 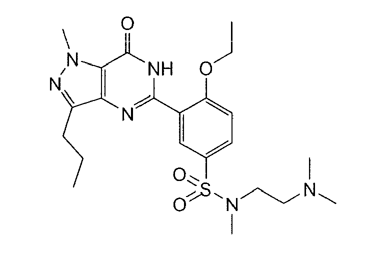 | 1-methyl-5-{2-ethoxy-5-[[1-methyl-1-(2-dimethylaminoethyl)]amidosulfonyl]phenyl}-3-n-propyl-1,6-dihydro-7H-pyrazolo[4,3-d]pyrimidin-7-one 10.82 (1H, s), 8.86 (1H, d), 7.88 (1H, dd), 7.16 (1H, d), 4.37 (2H, q), 4.27 (s, 3H), 3.22 (2H, t), 2.92 (2H, t), 2.86 (3H, s), 2.60 (2H, t), 2.32 (6H, s), 1.85 (2H, m), 1.64 (3H, t), 1.01 (3H, t) |
| 77 | 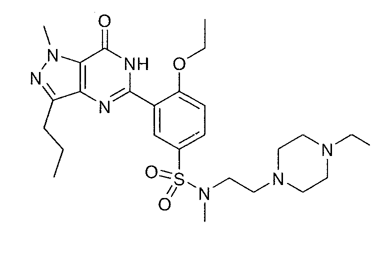 | 1-methyl-5-{2-ethoxy-5-[[1-methyl-1-[2-(4-ethylpiperazin-1-yl)]ethyl]amidosulfonyl]phenyl}-3-n-propyl-1,6-dihydro-7H-pyrazolo[4,3-d]pyrimidin-7-one 10.80 (1H, s), 8.85 (1H, d), 7.88 (1H, dd), 7.14 (1H, d), 4.37 (2H, q), 4.27 (s, 3H), 3.22 (2H, t), 2.92 (2H, t), 2.87 (3H, s), 2.58 (2H, t), 2.52 (4H, t), 2.44 (4H, t), 2.38 (2H, q), 1.85 (2H, m), 1.65 (3H, t), 1.06 (3H, t), 1.02 (3H, t) |
| 78 | 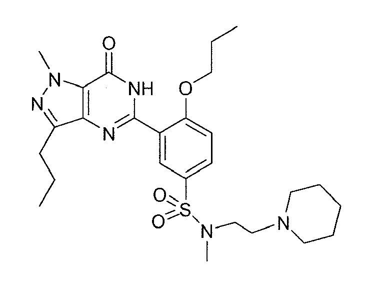 | 1-methyl-5-{2-propoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one 10.85 (1H, s), 8.86 (1H, d), 7.89 (1H, dd), 7.14 (1H, d), 4.27 (3H, s), 4.26 (2H, t), 3.22 (2H, t), 2.93 (2H, t), 2.86 (3H, s), 2.54 (2H, t), 2.40 (4H, t), 2.04 (2H, m), 1.85 (2H, m), 1.54 (4H, t), 1.41 (2H, m), 1.18 (3H, t), 1.02 (3H, t) |
| 79 | 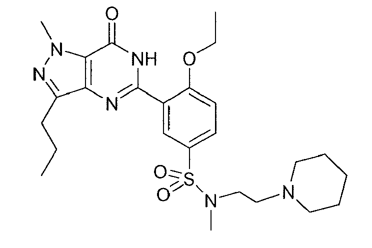 | 1-methyl-5-{2-ethoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one 10.83 (1H, s), 8.85 (1H, d), 7.89 (1H, dd), 7.13(1H, d), 4.37 (2H, q), 4.27 (3H, s), 3.21 (2H, t), 2.93 (2H, t), 2.86 (3H, s), 2.54 (2H, t), 2.40 (4H, t), 1.86 (2H, m), 1.64 (3H, t), 1.53 (4H, t), 1.40 (2H, m), 1.02 (3H, t) |
| 80 | 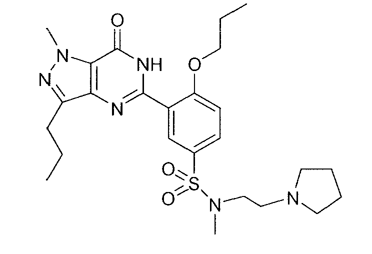 | 1-methyl-5-{2-propoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one 10.86 (1H, s), 8.87 (1H, d), 7.89 (1H, dd), 7.15 (1H, d), 4.27 (3H, s), 4.25 (2H, t), 3.22 (2H, t), 2.92 (2H, t), 2.86 (3H, s), 2.70 (2H, t), 2.54 (4H, m), 2.04 (2H, m), 1.86 (2H, m), 1.75 (4H, m), 1.18 (3H, t), 1.02 (3H, t) |
| 81 | 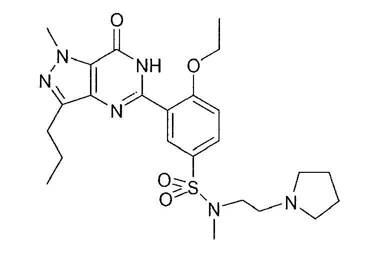 | 1-methyl-5-{2-ethoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one 10.85 (1H, s), 8.85 (1H, d), 7.88 (1H, dd), 7.13 (1H, d), 4.37 (2H, q), 4.27 (3H, s), 3.22 (2H, t), 2.92 (2H, t), 2.86 (3H, s), 2.70 (2H, t), 2.53 (4H, m), 1.86 (2H, m), 1.75 (4H, m), 1.64 (3H, t), 1.02 (3H, t) |
| 82 | 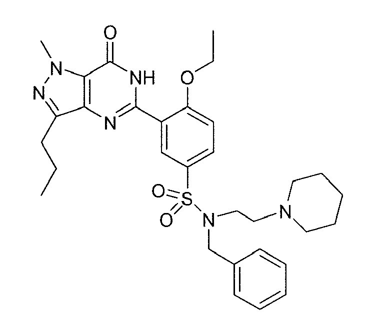 | 1-methyl-5-{2-ethoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one 10.84 (1H, s), 8.93 (1H, d), 7.95 (1H, dd), 7.29-7.32 (5H, m), 7.13 (1H, d), 4.46 (2H, s), 4.38 (2H, q), 4.28 (3H, s), 3.30 (2H, t), 2.92 (2H, t), 2.33 (2H, t), 2.24 (4H, t), 1.85 (2H, m),1.65 (3H, t), 1.46 (4H, t), 1.35 (2H, m), 1.00 (3H, t) |
| 83 | 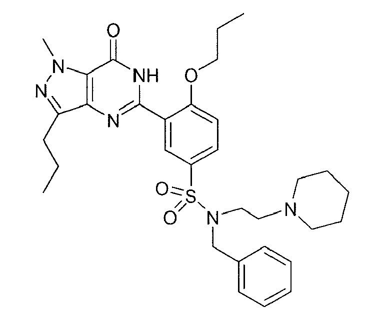 | 1-methyl-5-{2-propoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one 10.89 (1H, s), 8.94 (1H, d), 7.95 (1H, dd), 7.28-7.33 (5H, m), 7.14 (1H, d), 4.45 (2H, s), 4.28 (3H, s), 4.26 (2H, t), 3.33 (2H, t), 2.92 (2H, t), 2.38 (2H, t), 2.27 (4H, t), 2.05 (2H, m), 1.85 (2H, m), 1.50 (4H, t), 1.36 (2H, m), 1.19 (3H, t), 1.00 (3H, t) |
| 84 | 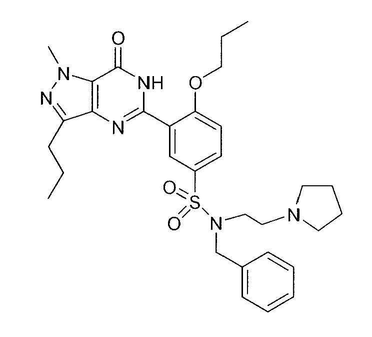 | 1-methyl-5-{2-propoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one 10.89 (1H, s), 8.94 (1H, d), 7.95 (1H, dd), 7.30-7.34 (5H, m), 7.14 (1H, d), 4.42 (2H, s), 4.28 (3H, s), 4.27 (2H, t), 3.32 (2H, t), 2.92 (2H, t), 2.54 (2H, t), 2.45 (4H, s), 2.05 (2H, m), 1.85 (2H, m), 1.72 (4H, m), 1.19 (3H, t), 1.00 (3H, t) |
| 85 | 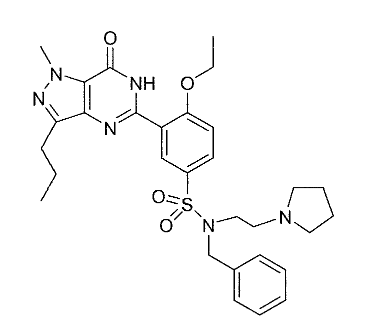 | 1-methyl-5-{2-ethoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one 10.88 (1H, s), 8.93 (1H, d), 7.95 (1H, dd), 7.29-7.34 (5H, m), 7.14 (1H, d), 4.43 (2H, s), 4.38 (2H, q), 4.28 (3H, s), 3.32 (2H, t), 2.92 (2H, t), 2.55 (2H, t), 2.46 (4H, m), 1.85 (2H, m), 1.72 (4H, m), 1.65 (3H, t), 1.00 (3H, t) |
| 86 | 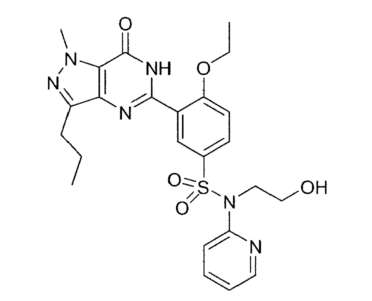 | 1-methyl-5-{2-propoxy-5-[1-(2-pyridyl)-1-(2-hydroxyethyl)amidosulfonyl]phenyl}-3-n-propyl-1,6-dihydro-7H-pyrazolo[4,3-d]pyrimidin-7-one 10.85 (1H, s), 8.85 (1H, d), 7.92 (1H, dd), 7.33 (2H, m), 7.12 (1H, d), 6.54 (1H, dd), 6.18 (1H, ddd), 4.36 (2H, q), 4.27(s, 3H), 4.10 (2H, t), 3.39 (2H, t), 2.92 (2H, t), 1.85 (2H, m), 1.65 (3H, t), 1.02 (3H, t) |
| 87 | 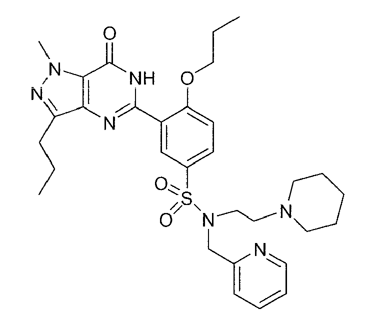 | 1-methyl-5-{2-propoxy-5-[[1-(2-pyridylmethyl)-1-(2-piperidin-1-yl)ethyl]amidosulfonyl]phenyl}-3 -n-propyl-1,6-dihydro-7H-pyrazolo[4,3-d]pyrimidin-7-one 10.87 (1H, s), 8.91 (1H, d), 7.92 (1H, dd), 8.45 (1H, d), 7.95 (1H, d), 7.68 (1H, ddd), 7.57 (1H, d), 7.17 (1H, t), 7.13 (1H, d), 4.55 (2H, s), 4.28 (3H, s), 4.26 (2H, t), 3.45 (2H, t), 2.93 (2H, t), 2.47 (2H, t), 2.33 (4H, t), 2.05 (2H, m), 1.86 (2H, m), 1.50 (4H, t), 1.38 (2H, m), 1.19 (3H, t), 1.01 (3H, t) |
| 88 | 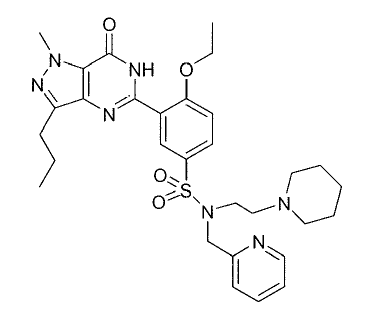 | 1-methyl-5-{2-ethoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one 10.83 (1H, s), 8.90 (1H, d), 8.45 (1H, d), 7.95(1H, d), 7.68 (1H, ddd), 7.56 (1H, d), 7.18 (1H, m), 7.13 (1H, dd), 4.56 (2H, s), 4.38 (2H, q), 4.28 (3H, s), 3.46 (2H, t), 2.92 (2H, t), 2.48 (2H, t), 2.34 (4H, t), 1.85 (2H, m), 1.65 (3H, t), 1.52 (4H, t), 1.38 (2H, m), 1.02 (3H, t) |
| 89 | 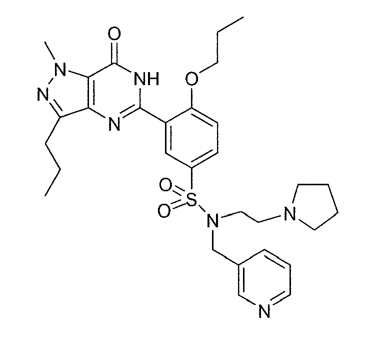 | 1-methyl-5-{2-propoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one 10.88 (1H, s), 8.93 (1H, d), 8.54 (1H, dd), 8.50 (1H, s), 7.94 (1H, dd), 7.81 (1H, d), 7.30 (1H, dd), 7.15 (1H, d), 4.46 (2H, s), 4.28 (3H, s), 4.27 (2H, t), 3.36 (2H, t), 2.92 (2H, t), 2.58 (2H, t), 2.48 (4H, t), 2.05 (2H, m), 1.85 (2H, m), 1.73 (4H, t), 1.19 (3H, t), 1.00 (3H, t) |
| 90 | 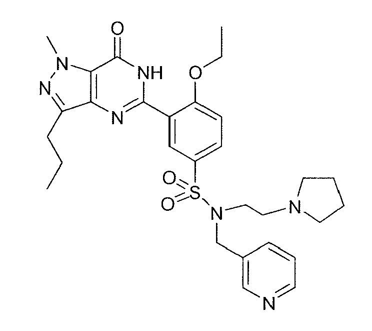 | 1-methyl-5- {2-ethoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one 10.85 (1H, s), 8.91 (1H, d), 8.54 (1H, dd), 8.50 (1H, d), 7.94 (1H, dd), 7.81 (1H, d), 7.30 (1H, dd), 7.15 (1H, d), 4.46 (2H, s), 4.38 (2H, q), 4.28 (3H, s), 3.37 (2H, t), 2.92 (2H, t), 2.60 (2H, t), 2.50 (4H, t), 1.85 (2H, m), 1.74 (4H, t), 1.65 (3H, t), 1.00 (3H, t) |
| 91 | 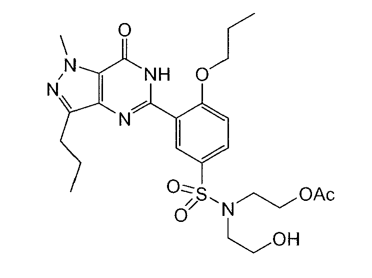 | 1-methyl-5-{2-propoxy-5-[[1-(2-acetoxyethyl)-1-(2-hydroxyethyl)]amidosulfonyl]phenyl}-3-n-propyl-1,6-dihydro-7H-pyrazolo[4,3-d]pyrimidin-7-one 10.84 (1H, s), 8.87 (1H, d), 7.92 (1H, dd), 7.15 (1H, d), 4.30 (2H, t), 4.27 (3H, s), 4.25 (2H, t), 3.82(2H, t), 3.48 (2H, t), 3.38 (2H, t), 2.93(2H, t), 2.06 (3H, s), 2.04 (2H, m), 1.85(2H, m), 1.17 (3H, t), 1.02 (3H, t) |
| 92 | 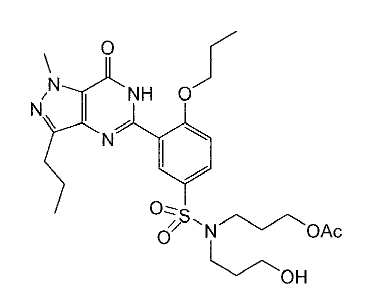 | 1-methyl-5-{2-propoxy-5-[[1-(3-acetoxypropyl)-1-(3-hydroxypropyl)]amidosulfonyl]phenyl}-3-n-propyl-1,6-dihydro-7H-pyrazolo[4,3-d]pyrimidin-7-one 10.80 (1H, s), 8.82 (1H, d), 7.90 (1H, dd), 7.14 (1H, d), 4.27 (3H, s), 4.25 (2H, t), 4.08 (2H, t), 3.72 (2H, t), 3.35 (2H, t), 3.27 (2H, t), 2.92 (2H, t), 2.05 (3H, s), 2.04 (2H, m), 1.95 (2H, m), 1.85(2H, m), 1.78 (2H, m), 1.17 (3H, t), 1.02 (3H, t) |
| 93 | 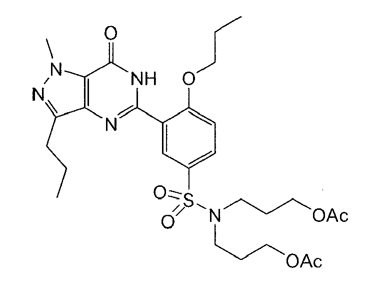 | 1-methyl-5-{2-propoxy-5-[bis(3-acetoxypropyl)amidosulfonyl]phenyl}-3-n-propyl-1,6-dihydro-7H-pyrazolo[4,3-d]pyrimidin-7-one 10.84 (1H, s), 8.86 (1H, d), 7.88 (1H, dd), 7.14 (1H, d), 4.27 (3H, s), 4.25 (2H, t), 4.08 (4H, t), 3.26 (4H, t), 2.92 (2H, t), 2.03 (6H, s), 2.02 (2H, m), 1.94 (4H, m), 1.85(2H, m), 1.17 (3H, t), 1.02 (3H, t) |
| 94 | 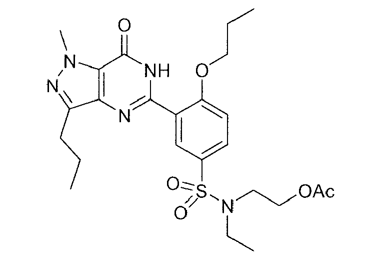 | 1-methyl-5-{2-propoxy-5-[[1-ethyl-1-(2-acetoxyethyl)]amidosulfonyl]phenyl}-3-n-propyl-1,6-dihydro-7H-pyrazolo[4,3-d]pyrimidin-7-one 10.85 (1H, s), 8.88 (1H, d), 7.90 (1H, dd), 7.12(1H, d), 4.27 (3H, s), 4.24 (4H, m), 3.42 (2H, t), 3.34(2H, q), 2.92 (2H, t), 2.04 (3H, s), 2.02 (2H, m), 1.85 (2H, m), 1.20 (3H, t), 1.17 (3H, t), 1.02 (3H, t) |
| 95 | 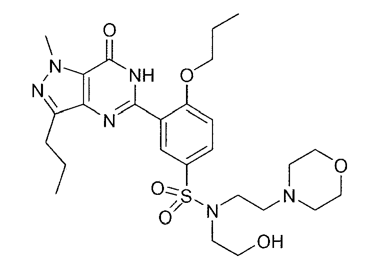 | 1-methyl-5-{2-propoxy-5-[[1-(2-hydroxyethyl)-1-(2-morpholin-1-yl)ethyl]amidosulfonyl]phenyl}-3-n-propyl-1,6-dihydro-7H-pyrazolo[4,3-d]pyrimidin-7-one 10.81 (1H, s), 8.85 (1H,d), 7.88 (1H, dd), 7.15 (1H, d), 4.27 (3H, s), 4.25 (2H, t), 3.83 (2H, t), 3.74 (4H, t), 3.28 (2H, t), 3.22 (2H, t), 2.92 (2H, t), 2.72 (2H, t), 2.58 (4H, t), 2.03 (2H, m), 1.85 (2H, m), 1.18 (3H, t), 1.02 (3H, t) |
| 96 | 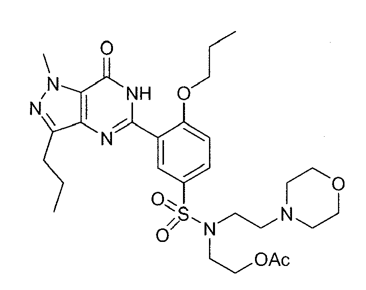 | 1-methyl-5-{2-propoxy-5-[[1-(2-acetoxyethyl)-1-(2-morpholin-1-yl)ethyl]amidosulfonyl]phenyl}-3-n-propyl-1,6-dihydro-7H-pyrazolo[4,3-d]pyrimidin-7-one 10.84 (1H, s), 8.88 (1H,d), 7.91 (1H, dd), 7.13 (1H, d), 4.27 (3H, s), 4.25 (2H, t), 4.23 (2H, t), 3.63 (4H, t), 3.48 (2H, t), 3.35 (2H, t), 2.92 (2H, t), 2.58 (2H, t), 2.44 (4H, t), 2.04 (2H, m), 2.02 (3H, s), 1.85 (2H, m), 1.17 (3H, t), 1.02 (3H, t) |
| 97 | 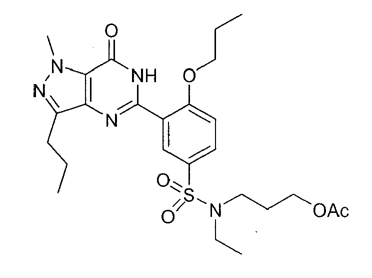 | 1-methyl-5-{2-propoxy-5-[[1-ethyl-1-(3-acetoxypropyl)]amidosulfonyl]phenyl}-3-n-propyl-1,6-dihydro-7H-pyrazolo[4,3-d]pyrimidin-7-one 10.85 (1H, s), 8.87 (1H, d), 7.90 (1H, dd), 7.12 (1H, d), 4.27 (3H, s), 4.25 (2H, t), 4.10 (2H, t), 3.30 (2H, q), 3.26 (2H, t), 2.92 (2H, t), 2.05 (2H, m), 2.04 (3H, s), 1.95 (2H, m), 1.85 (2H, m), 1.18 (6H, t), 1.02 (3H, t) |
| 98 | 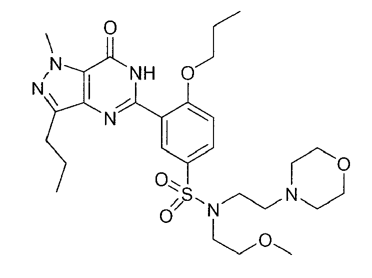 | 1-methyl-5-{2-propoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one 10.86 (1H, s), 8.89 (1H,d), 7.92 (1H, dd), 7.12 (1H, d), 4.27 (3H, s), 4.24 (2H, t), 3.64 (4H, t), 3.56 (2H, t), 3.43 (2H, t), 3.37 (2H, t), 3.29 (3H, s), 2.92 (2H, t), 2.60 (2H, t), 2.45 (4H, t), 2.03 (2H, m), 1.85 (2H, m), 1.18 (3H, t), 1.02 (3H, t) |
| 99 | 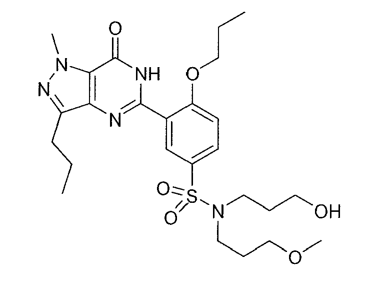 | 1-methyl-5-{2-propoxy-5-[[1-(3-methoxypropyl)-1-(3-hydroxypropyl)]amidosulfonyl]phenyl}-3-n-propyl-1,6-dihydro-7H-pyrazolo[4,3-d]pyrimidin-7-one 10.84 (1H, s), 8.86 (1H, d), 7.91 (1H, dd), 7.13(1H, d), 4.27 (3H, s), 4.25 (2H, t), 3.74 (2H, t), 3.56 (2H, t), 3.38 (4H, m), 3.29 (3H, s), 2.92 (2H, t), 2.02 (2H, m), 1.85 (4H, m), 1.17 (3H, t), 1.02 (3H, t) |
| 100 | 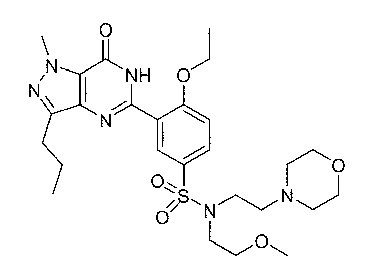 | 1-methyl-5-{2-ethoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one 10.82 (1H, s), 8.88 (1H, d), 7.92 (1H, dd), 7.11 (1H, d), 4.36 (2H, q), 4.27(3H, s), 3.64 (4H, t), 3.55 (2H, t), 3.43 (2H, t), 3.37 (2H, t), 3.29 (3H, s), 2.92 (2H, t), 2.60 (2H, t), 2.45 (4H, t), 1.86 (2H, m), 1.63 (3H, t), 1.02(3H, t) |
| 101 | 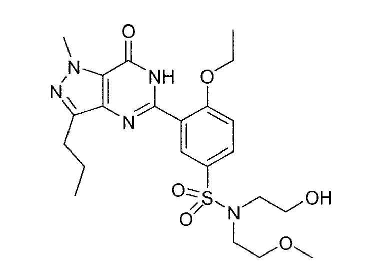 | 1-methyl-5-{2-ethoxy-5-[[1-(2-methoxyethyl)-1-(2-hydroxyethyl)]amidosulfonyl]phenyl}-3-n-propyl-1,6-dihydro-7H-pyrazolo[4,3-d]pyrimidin-7-one 10.82 (1H, s), 8.89 (1H, d), 7.93 (1H, dd), 7.16 (1H, d), 4.39 (2H, q), 4.30 (3H, s), 3.81 (2H, t), 3.72 (2H, t), 3.44 (2H, t), 3.41 (3H, s), 3.36 (2H, t), 2.96 (2H, t), 1.88 (2H, m), 1.66 (3H, t), 1.05 (3H, t) |
| 102 | 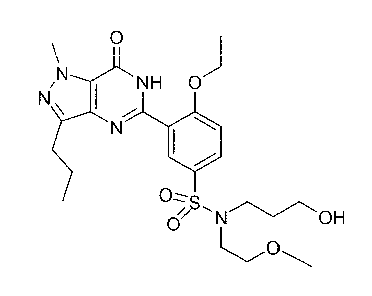 | 1-methyl-5-{2-ethoxy-5-[[1-(2-methoxyethyl)-1-(3-hydroxypropyl)]amidosulfonyl]phenyl}-3-n-propyl-1,6-dihydro-7H-pyrazolo[4,3-d]pyrimidin-7-one 10.82 (1H, s), 8.88 (1H, d), 7.94 (1H, dd), 7.15 (1H, d), 4.39 (2H, q), 4.29 (3H, s), 3.76 (2H, t), 3.59 (2H, t), 3.41 (4H, m), 3.32 (3H, s), 2.95 (2H, t), 1.87 (2H, m), 1.84 (2H, m), 1.66 (3H, t), 1.05 (3H, t) |
| 103 | 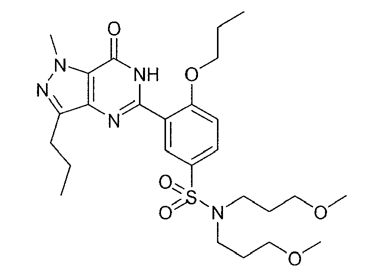 | 1-methyl-5-{2-propoxy-5-[bis(3-methoxypropyl)amidosulfonyl]phenyl}-3-n-propyl-1,6-dihydro-7H-pyrazolo[4,3-d]pyrimidin-7-one 10.88 (1H, s), 8.90 (1H, d), 7.92 (1H, dd), 7.15 (1H, d), 4.29 (3H, s), 4.27 (2H, t), 3.43 (4H, t), 3.32 (6H, s), 3.29 (4H, t), 2.96 (2H, t), 2.05 (2H, m), 1.89 (6H, m), 1.20 (3H, t), 1.05 (3H, t) |
| 104 | 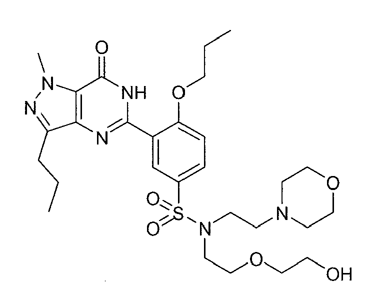 | 1-methyl-5-{2-propoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one 10.85 (1H, s), 8.84 (1H , d), 7.90 (1H, dd), 7.13 (1H, d), 4.27 (3H, s), 4.25 (2H, t), 3.65 (8H, m), 3.49 (4H, t), 3.42 (2H, t), 2.94 (2H, t), 2.64 (2H, t), 2.44 (4H, t), 2.03 (2H, m), 1.85 (2H, m), 1.17 (3H, t), 1.01 (3H, t) |
| 105 | 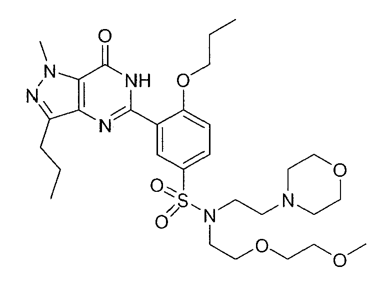 | 1-methyl-5-{2-propoxy-5-[[1-(2-methoxyethoxyethyl)-1-(2-morpholin-1-yl)ethyl]amidosulfonyl]phenyl}-3-n-propyl-1,6-dihydro-7H-pyrazolo[4,3-d]pyrimidin-7-one 10.85 (1H, s), 8.88 (1H , d), 7.93 (1H, dd), 7.12 (1H, d), 4.27 (3H, s), 4.25 (2H, t), 3.67 (2H, t), 3.65 (4H, t), 3.57 (2H, t), 3.48 (2H, t), 3.43 (2H, t), 3.38 (2H, t), 3.33 (3H, s), 2.92 (2H, t), 2.62 (2H, t), 2.46 (4H, t), 2.03 (2H, m), 1.85 (2H, m), 1.18 (3H, t), 1.02 (3H, t) |
| 106 | 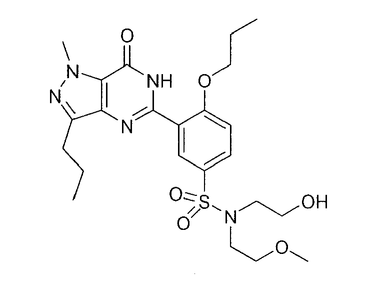 | 1-methyl-5-{2-propoxy-5-[[1-(2-methoxyethyl)-1-(2-hydroxyethyl)]amidosulfonyl]phenyl}-3-n-propyl-1,6-dihydro-7H-pyrazolo[4,3-d]pyrimidin-7-one 10.84 (1H, s), 8.87 (1H, d), 7.89 (1H, dd), 7.13 (1H, d), 4.26 (3H, s), 4.24 (2H, q), 3.78 (2H, t), 3.69 (2H, t), 3.40 (2H, t), 3.38 (3H, s), 3.32 (2H, t), 2.92 (2H, t), 2.03 (2H, m), 1.88 (2H, m), 1.17 (3H, t), 1.02 (3H, t) |
| 107 | 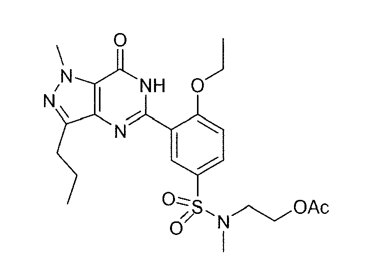 | 1-methyl-5-{2-ethoxy-5-[[1-methyl-1-(2-acetoxyethyl)]amidosulfonyl]phenyl}-3-n-propyl-1,6-dihydro-7H-pyrazolo[4,3-d]pyrimidin-7-one 10.82 (1H, s), 8.83 (1H, d), 7.88 (1H, dd), 7.14 (1H, d), 4.36 (4H, q), 4.26 (3H, s), 3.79 (2H, t), 3.24 (2H, t), 2.92 (2H, t), 2.89 (3H, s), 1.85 (2H, m), 1.62 (3H, s), 1.01 (3H, t) |
| 108 | 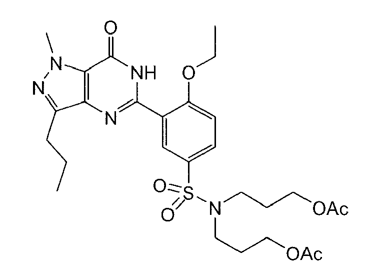 | 1-methyl-5-{2-ethoxy-5-[bis(3-acetoxypropyl)amidosulfonyl]phenyl}-3-n-propyl-1,6-dihydro-7H-pyrazolo[4,3-d]pyrimidin-7-one 10.80 (1H, s), 8.84 (1H, d), 7.88 (1H, dd), 7.13 (1H, d), 4.36 (2H, q), 4.27 (3H, s), 4.08 (4H, t), 3.26 (4H, t), 2.92 (2H, t), 2.03 (6H, s), 1.94 (4H, m), 1.85(2H, m), 1.64 (3H, s), 1.01 (3H, t) |
| 109 | 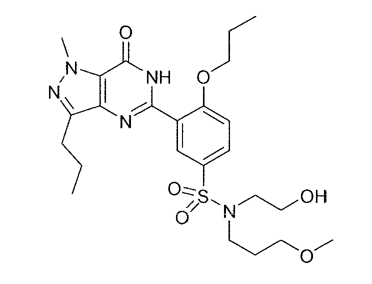 | 1-methyl-5-{2-propoxy-5-[[1-(3-methoxypropyl)-1-(2-hydroxyethyl)]amidosulfonyl]phenyl}-3-n-propyl-1,6-dihydro-7H-pyrazolo[4,3-d]pyrimidin-7-on 10.84 (1H, s), 8.87 (1H, d), 7.90 (1H, dd), 7.14 (1H, d), 4.26 (3H, s), 4.24 (2H, t), 3.80 (2H, t), 3.48 (2H, t), 3.32 (3H, s), 3.30 (4H, m), 2.92 (2H, t), 2.03 (2H, m), 1.92 (2H, m), 1.85 (2H, m), 1.17 (3H, t), 1.02 (3H, t) |
| 110 | 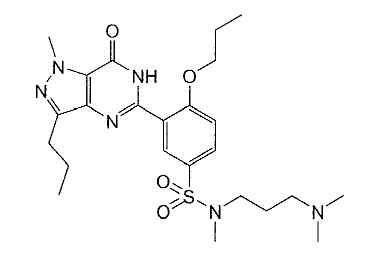 | 1-methyl-5-{2-propoxy-5-[[1-methyl-1-(3-dimethylaminopropyl)]amidosulfonyl]phenyl}-3-n-propyl-1,6-dihydro-7H-pyrazolo[4,3-d]pyrimidin-7-one 10.88 (1H, s), 8.88 (1H, d), 7.90 (1H, dd), 7.15 (1H, d), 4.30 (s, 3H), 4.27 (2H, t), 3.15 (2H, t), 2.95 (2H, t), 2.83 (3H, s), 2.43 (2H, t), 2.30 (6H, s), 2.06 (2H, m), 1.89 (2H, m), 1.80 (2H, m), 1.20 (3H, t), 1.05 (3H, t) |
| 111 | 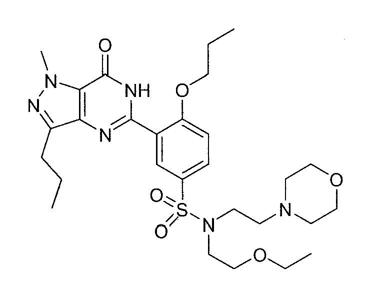 | 1-methyl-5-{2-propoxy-5-[[1-(2-ethoxyethyl)-1-(2-morpholin-1-yl)ethyl]amidosulfonyl]phenyl}-3 -n-propyl-1,6-dihydro-7H-pyrazolo[4,3-d]pyrimidin-7-one 10.85 (1H, s), 8.88 (1H, d), 7.92 (1H, dd), 7.12 (1H, d), 4.27(3H, s), 4.24 (2H, t), 3.64 (4H, t), 3.59 (2H, t), 3.44 (2H, q), 3.42 (2H, t), 3.39 (2H, t), 2.92 (2H, t), 2.62 (2H, t), 2.46 (4H, t), 2.03 (2H, t), 1.85 (2H, m), 1.18 (3H, t),1.12 (3H, t), 1.02(3H, t) |
| 112 | 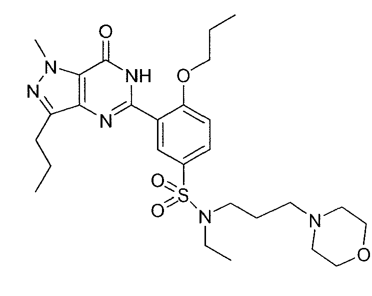 | 1-methyl-5-{2-propoxy-5-[[1-ethyl-1-(3-morpholin-1-yl)propyl]amidosulfonyl]phenyl}-3-n-propyl-1,6-dihydro-7H-pyrazolo[4,3-d]pyrimidin-7-one 10.86 (1H, s), 8.86 (1H, d), 7.89 (1H, dd), 7.12 (1H, d), 4.26 (3H, s), 4.24 (2H, t), 3.70 (4H, t), 3.30 (2H, q), 3.23 (2H, t), 2.92 (2H, t),2.45 (4H, t), 2.42 (2H, t), 2.03 (2H, m), 1.85 (4H, m), 1.17 (3H, t), 1.15 (3H, t), 1.01 (3H, t) |
| 113 | 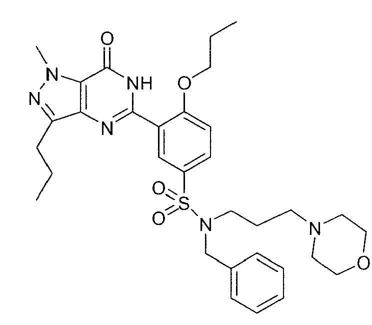 | 1-methyl-5-{2-propoxy-5-[[1-benzyl-1-(3-morpholin-1-yl)propyl]amidosulfonyl]phenyl}-3-n-propyl-1,6-dihydro-7H-pyrazolo[4,3-d]pyrimidin-7-one 10.87 (1H, s), 8.91 (1H, d), 7.91 (1H, dd), 7.27-7.32 (5H, m), 7.14 (1H, d), 4.37 (2H, s), 4.27 (3H, s), 4.26 (2H, t), 4.24 (2H, t), 3.57 (4H, t), 3.20 (2H, t), 2.92 (2H, t), 2.19 (4H, t), 2.04 (2H, m), 1.84 (2H, m), 1.58 (2H, m), 1.18 (3H, t), 0.99 (3H, t) |
| 114 | 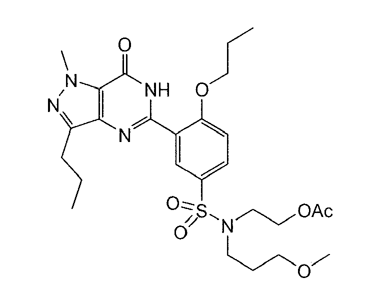 | 1-methyl-5-{2-propoxy-5-[[1-(3-methoxypropyl)-1-(2-acetoxyethyl)]amidosulfonyl]phenyl}-3-n-propyl-1,6-dihydro-7H-pyrazolo[4,3-d]pyrimidin-7-one 10.84 (1H, s), 8.87 (1H, d), 7.89 (1H, dd), 7.13 (1H, d), 4.26 (3H, s), 4.22 (2H, t), 3.41 (4H, m), 3.31 (2H, t), 3.29 (3H, s), 2.92 (2H, t), 2.04 (2H, m), 2.02 (3H, s), 1.92 (2H, m), 1.86 (4H, m), 1.17 (3H, t), 1.02 (3H, t) |
| 115 | 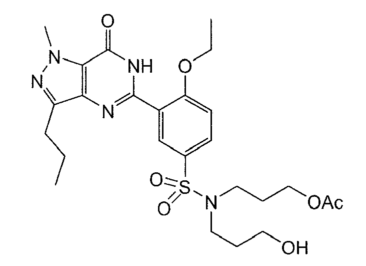 | 1-methyl-5-{2-ethoxy-5-[[1-(3-acetoxypropyl)-1-(3-hydroxypropyl)]amidosulfonyl]phenyl}-3-n-propyl-1,6-dihydro-7H-pyrazolo[4,3-d]pyrimidin-7-one 10.76 (1H, s), 8.82 (1H, d), 7.90 (1H, dd), 7.13 (1H, d), 4.36 (2H, q), 4.26 (3H, s), 4.08 (2H, t), 3.72 (2H, t), 3.35 (2H, t), 3.27 (2H, t), 2.92 (2H, t), 2.43 (1H, s), 2.02 (3H, s), 2.04 (2H, m), 1.94 (2H, m), 1.86 (2H, m), 1.78 (2H, m), 1.64 (3H, t), 1.01 (3H, t) |
| 116 | 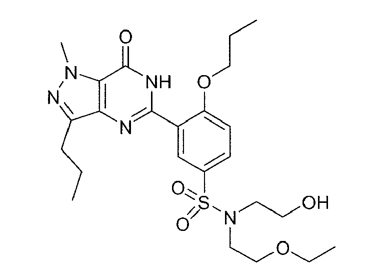 | 1-methyl-5-{2-propoxy-5-[[1-(3-ethoxyethyl)-1-(2-hydroxyethyl)]amidosulfonyl]phenyl}-3-n-propyl-1,6-dihydro-7H-pyrazolo[4,3-d]pyrimidin-7-one 10.84 (1H, s), 8.87 (1H, d), 7.89 (1H, dd), 7.14 (1H, d), 4.26 (3H, s), 4.25 (2H, t), 3.78 (2H, t), 3.74 (2H, t), 3.54 (2H, q), 3.39 (2H, t), 3.32 (2H, t), 2.92 (2H, t), 2.03 (2H, m), 1.85 (2H, m), 1.19 (3H, t), 1.17 (3H, t), 1.02 (3H, t) |
| 117 | 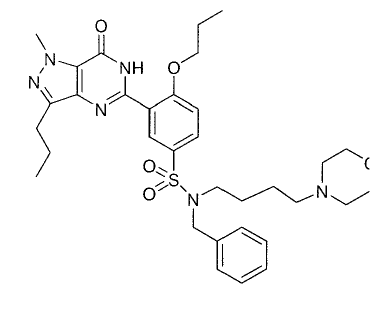 | 1-methyl-5-{2-propoxy-5-[[1-benzyl-1-(4-morpholin-1-yl)butyl]amidosulfonyl]phenyl}-3-n-propyl-1,6-dihydro-7H-pyrazolo[4,3-d]pyrimidin-7-one 10.88 (1H, s), 8.91 (1H, d), 7.91 (1H, dd), 7.27-7.30 (5H, m), 7.14 (1H, d), 4.37 (2H, s), 4.27 (3H, s), 4.25 (2H, t), 3.62 (4H, t), 3.17 (2H, t), 2.92 (2H, t), 2.30 (4H, t), 2.15 (2H, t), 2.04 (2H, m), 1.84 (2H, m), 1.35 (4H, m), 1.18 (3H, t), 0.99 (3H, t) |
| 118 | 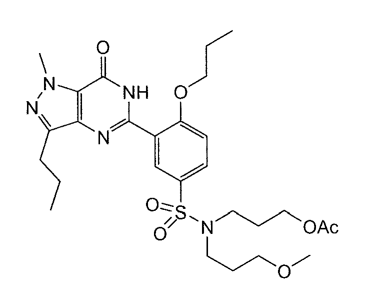 | 1-methyl-5-{2-propoxy-5-[[1-(3-methoxypropyl)-1-(3-acetoxypropyl)]amidosulfonyl]phenyl}-3-n-propyl-1,6-dihydro-7H-pyrazolo[4,3-d]pyrimidin-7-one 10.86 (1H, s), 8.88 (1H, d), 7.90 (1H, dd), 7.14 (1H, d), 4.28 (3H, s), 4.26 (2H, t), 4.08 (2H, t), 3.41 (2H, t), 3.30 (3H, s), 3.27 (2H, t), 3.25 (2H, t), 2.93 (2H, t), 2.06 (2H, m), 2.03 (3H, s), 1.92 (2H, m), 1.86 (4H, m), 1.19 (3H, t), 1.03 (3H, t) |
| 119 | 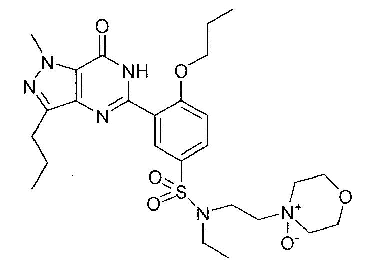 | 1-methyl-5-{2-propoxy-5-[[1-ethyl-1-[2-(morpholin-N-oxide)-1-yl)ethyl]amidosulfonyl]phenyl}-3-n-propyl-1,6-dihydro-7H-pyrazolo[4,3-d]pyrimidin-7-one 10.87 (1H, s), 8.86 (1H,d), 7.89 (1H, dd), 7.17 (1H, d), 4.36 (2H, m), 4.28 (3H, s), 4.26 (2H, t), 3.84 (2H, d), 3.77 (4H, m), 3.45 (4H, m), 3.35 (2H, q), 2.92 (2H, t), 2.03 (2H, m), 1.85 (2H, m), 1.22 (3H, t), 1.18 (3H, t), 1.01 (3H, t) |
| 120 | 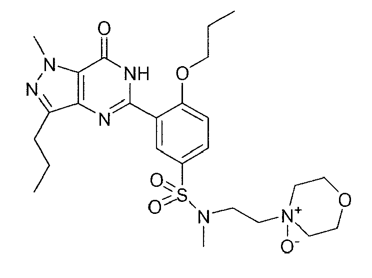 | 1-methyl-5-{2-propoxy-5-[[1-methyl-1-[2-(morpholin-N-oxide)-1-yl)ethyl]amidosulfonyl]phenyl}-3-n-propyl-1,6-dihydro-7H-pyrazolo[4,3-d]pyrimidin-7-one 10.88 (1H, s), 8.88 (1H,d), 7.87 (1H, dd), 7.17 (1H, d), 4.41 (2H, t), 4.27 (3H, s), 4.26 (2H, t), 3.80 (2H, d), 3.70 (2H, t), 3.60 (2H, t), 3.43 (2H, t), 3.23 (2H, t), 2.92 (2H, t), 2.03 (2H, m), 1.85 (2H, m), 1.18 (3H, t), 1.01 (3H, t) |

### Example 121: Capsules

| Formulation | Quantity/1000 Capsules |
|---|---|
| Active ingredient (Pyrazolopyrimidinone derivatives) | 20g |
| Starch | 80g |
| Lactose | 60g |
| Microcrystalline cellulose | 35g |
| 10% Polyvinylpyrrolidone ethanol solution | q.s. |
| Magnesium stearate | 0.5g |
| Total 1000 Capsules | |

The active ingredient containing pyrazolopyrimidinone derivatives and the excipients are passed through a #80 mesh sieve, weigh out the appropriate amount of active ingredient and the excipients according the formulation. Granulate the powder mixture with 10% polyvinylpyrrolidone ethanol solution, and pass through a #16 mesh sieve to obtain suitable granules. After drying at 65°C, the granules were screened through a #14 sieve and blended with the magnesium stearate. Test the content of active ingredient in the granules, calculate the fill weight, and then fill the granules in capsules.

### Example 122 Tablets (wet granulation)

| Formulation | Quantity/1000 Tablets |
|---|---|
| Active ingredient (Pyrazolopyrimidinone derivatives) | 20g |
| Lactose | 120g |
| Microcrystalline cellulose | 40g |
| 8% Starch paste | q.s. |
| Sodium starch glycolate | 10g |
| Magnesium stearate | 1.0g |
| Total 1000 Tablets | |

Pass the active ingredient containing pyrazolopyrimidinone derivatives, microcrystalline cellulose, lactose, sodium starch glycolate through #80 mesh sieve, mix well. Granulate the powder mixture with 8% starch paste, and pass through a #16 mesh sieve to obtain suitable granules. After drying, the granules were screened and blended with the magnesium stearate. Test the content of active ingredient in the granules, calculate the tablet weight, and then compress the tablets.

### Example 123: Tablets (Direct Compression)

| Formulation | Quantity/1000 Tablets |
|---|---|
| Active ingredient (Pyrazolopyrimidinone derivative) | 20.0g |
| Microcrystalline cellulose | 30.0g |
| Lactose, anhydrous | 45.0g |
| Polyvinyl pyrrolidone | 3.0g |
| Aerosil | 0.2g |
| Magnesium stearate | 0.5g |
| Total 1000 Tablets | |

Charge the active ingredient containing pyrazolopyrimidinone derivatives, lactose, polyvinyl pyrrolidone, aerosol in a mixer and mix well. Blend the mixture with magnesium stearate and then compress into tablets.

### Test 1: pharmacodynamic test

The test was carried out based on the methods reported (International Journal of Impotence Research 2002, 14, 251 and The Journal of Urology 1992, 147, 1124). After fasted for 12 hours, 4 male SD rats were randomized into each group. After anesthetizing the rats with sodium pentobarbital (50 mg/kg, i.p.), the penile skin was incised and the prepuce was degloved to expose completely the corpora cavemosa (CC). A needle linked to an electrophysiology instrument was inserted into the CC on the right side in order to measure the intracavernous pressure (ICP). The right carotid artery was cannulated in a similar manner to the polyethylene tube in order to monitor the mean blood pressure (MBp) continuously. After exposing the lateral surface of the prostate via an incision in the midline inferior abdomen, a bipolar platinum microelectrode was placed on the cavernous nerve. Electric stimulation was performed at 2 Hz, for 60 s with a pulse duration of 5ms and 3V using a stimulator. The compounds were administrated orally (5mg/kg). The change of the ICP and MBp were monitored continuously before and after the administration. The effect of the compounds on the erection induced by electric stimulation was evaluated by the ratio of ICP to MBp. The parameter (ICP/MBp) was used to estimate the influence of the compounds to the rat corpora cavernosa. We tested the effect of sildenafil and some of the example compounds on the rat corpora cavernosa according to the aforesaid method. The statistical significance of the differences between groups was calculated using Duncan's multiple comparison. The results are shown below:

| Test compound | ICP/BP |
|---|---|
| blank | 27.5±2.3 |
| sildenafil | 80.1±5.1*** |
| 33 | 72.3±28.6*** |
| 54 | 86.3±6.4*** |
| 62 | 69.8±12.2*** |
| 75 | 74.2±7.8*** |
| 78 | 71.6±8.3*** |
| 89 | 70.9±9.8*** |
| 91 | 55.8±16.8* |
| 92 | 86.4±4.8*** |
| 93 | 77.2±24.8*** |
| 95 | 57.2±10.1* |
| 96 | 81.0±9.5*** |
| 97 | 72.0±13.5*** |
| 99 | 56.2±9.3*** |
| 111 | 89.1±6.9*** |
| 118 | 77.9±2.3*** |

| | |
|---|---|
| Note: Compared with the blank group , *P<0.05 , ***P<0.001 | |

As shown in the results, the test compounds have the same pharmacodynamic effect as sildenafil. After administration, the ICP of the rats CC, and the ICP/BP increase significantly, the penile erections of the rats are enhanced, thus, the compounds can be administrated orally for the treatment of erectile dysfunction.

### Test 2: Enzyme inhibitory activity test

The Enzymes used in the inhibitory activity test were isolated from different kinds of tissues after appropriate treatment by FPLC using a method similar to Thrombosis Res. 1991, 62, 31 and J. Biol. Chem. 1997, 272, 2714. The PDE5 and PDE3 were isolated form human platelets, while the PDE6 was isolated form bovine retinas. The enzyme inhibitory activity test conducted immediately after the enzymes had been isolated, using a scintillation proximity assay for the direct detection of AMP/GMP by the TRKQ7100 and TRKQ7090 kit. In summary, the effect of PDE inhibitors was investigated by assaying a fixed amount of enzyme in the presence of varying inhibitor concentrations and low substrate. The final assay volume was made up to 100µl with 10µl assay buffer (50mM Tris/HCl PH 7.5, 8.3mM MgCl2, 1.7mM EGTA), and water. Reactions were initiated with enzyme, incubate for 30 minutes at 30°C and terminated with 50µl yttrium silicate SPA beads suspension containing zinc sulphate. Shook for 20 minutes and settled for 30 minutes in the dark, then counted on a BECKMAN LS6500 MULTIPURPOSE SCINTILLATION COUNTER. The IC50 value for the compounds according the present invention was calculated according the counts.

### PDE5 inhibitory activity test

According to the above-mentioned method, the inhibitory activities of some compounds of formula 1A and 1B according to the invention against PDE5 from human paletelets were determined. The result is given in the following table:

| Test compound | PDE5 IC₅₀ (nM) | Test compound | PDE5 IC₅₀ (nM) |
|---|---|---|---|
| sildenafil | 15.7 | 25 | 0.335 |
| 1 | 0.080 | 26 | 24.9 |
| 2 | 0.133 | 27 | 0.456 |
| 3 | 0.056 | 28 | 0.681 |
| 4 | 3.37 | 29 | 0.310 |
| 5 | 1.08 | 37 | 4.90 |
| 6 | 0.74 | 41 | 10.47 |
| 7 | 0.50 | 54 | 9.81 |
| 8 | 2.14 | 78 | 8.72 |
| 9 | 16.9 | 84 | 11.72 |
| 10 | 22.2 | 87 | 8.77 |
| 11 | 38.1 | 89 | 7.85 |
| 12 | 6.98 | 92 | 3.85 |
| 13 | 7.37 | 93 | 3.80 |
| 14 | 0.862 | 94 | 5.41 |
| 15 | 10.6 | 96 | 4.24 |
| 16 | 13.4 | 97 | 13.08 |
| 17 | 8.55 | 99 | 4.03 |
| 18 | 6.32 | 103 | 5.61 |
| 19 | 4.29 | 104 | 13.08 |
| 20 | 0.505 | 108 | 17.44 |
| 21 | 0.928 | 111 | 6.83 |
| 22 | 0.294 | 112 | 12.08 |
| 23 | 0.072 | 116 | 11.21 |
| 24 | 0.087 | 118 | 5.41 |

The IC₅₀ values for the compounds in the previous table show that most of the compounds according to the invention have a stronger potency against PDE5 than sildenafil, therefore, the dosage for oral administration is less than sildenafil and the chance to induce side effects is relatively little.

### PDE6 inhibitory activity test

Considering the compounds according the invention may have inhibitory activity against PDE6 distributed in retina, and then lead to visual disorders, we tested the inhibitory activities of some of the compounds of formulae 1A and 1B according the invention against PDE6 from bovine retina. The result is given in the following table:

| Test compound | PDE6 IC₅₀ (nM) | PDE5 IC₅₀ (nM) | PDE6 IC₅₀ / PDE5 IC₅₀ |
|---|---|---|---|
| sildenafil | 195.6 | 15.7 | 12.4 |
| 1 | 16.8 | 0.080 | 210 |
| 2 | 56.5 | 0.133 | 425 |
| 3 | 32.9 | 0.056 | 588 |
| 4 | 276.4 | 3.37 | 82.1 |
| 5 | 130.9 | 1.08 | 131 |
| 24 | 5.71 | 0.087 | 65.6 |
| 25 | 93.6 | 0.335 | 279 |
| 54 | 234.7 | 9.81 | 23.9 |
| 89 | 215.2 | 7.85 | 27.4 |
| 92 | 254.3 | 3.85 | 66.0 |
| 96 | 244.5 | 4.24 | 57.7 |
| 118 | 203.2 | 5.41 | 37.6 |

The invention uses the value of IC₅₀ PDE6/ IC₅₀ PDE5 to estimate the selectivity of PDE5 versus PDE6. The results show that most of the example compounds have a better selectivity than sildenafil, thus, the chance of visual disorder induced by the compounds according to the invention is less than sildenafil.

### PDE3 inhibitory activity test

PDE3 is a PDE isozyme distributed mainly in heart, so the inhibiting of PDE3 may lead to side effects associated with heart. Accordingly, the inhibitory activities of some example compounds according to the invention against PDE3 were determined. The result is given in the following table:

| Test compound | PDE3 IC₅₀ (µM) | Test compound | PDE3 IC₅₀ (µM) |
|---|---|---|---|
| sildenafil | 7.31 | 93 | 6.0 |
| 33 | 30.38 | 96 | 12.41 |
| 54 | 16.27 | 97 | 5.12 |
| 62 | 2.86 | 111 | 8.73 |
| 92 | 10.52 | 118 | 20.54 |

As shown in the table, since the 50% inhibition concentration (IC₅₀) for PDE 3 is much higher than for PDE5 in some of the compounds of the pyrazolopyrimidinone derivatives, the probability of side effects in cardiovascular system caused by the compounds of the present invention is very little.

### Test 3: Acute Oarl Toxicity Test

In this test male KM mouse weighting 18-22g were used, and 10 or 11 mouse were assigned randomly to each group. The compounds of examples 23, 33, 35, 37, 41, 54, 62, 63, 89, 92, 93, 95, 96, 97, 99, 103, 104, 110, 111, 112, 118 and sildenafil were suspended in 0.5% sodium carboxymethylcellulose respectively, and administered orally with single dose of 3g/kg. The animals were fasted for 12 hours before the administration. After the administration, the animals were observed for clinical signs of toxicity or mortality. The results were shown in the following table:

| Test compound | Number of mouse | Number of death | mortality rate (%) |
|---|---|---|---|
| sildenafil | 10 | 6 | 60 |
| 23 | 10 | 0 | 0 |
| 33 | 10 | 0 | 0 |
| 35 | 10 | 0 | 0 |
| 37 | 10 | 3 | 30 |
| 41 | 10 | 0 | 0 |
| 54 | 10 | 0 | 0 |
| 62 | 10 | 6 | 60 |
| 63 | 11 | 1 | 9 |
| 89 | 11 | 5 | 45 |
| 92 | 10 | 0 | 0 |
| 93 | 10 | 0 | 0 |
| 95 | 10 | 1 | 10 |
| 96 | 10 | 0 | 0 |
| 97 | 11 | 5 | 45 |
| 99 | 10 | 1 | 10 |
| 103 | 10 | 0 | 0 |
| 111 | 10 | 0 | 0 |
| 112 | 10 | 2 | 20 |
| 118 | 10 | 0 | 0 |

There were no significant clinical symptoms, weight changes and mortalities during the test. The results of autopsy in dead animals show that no abnormal signals such as bleeding of the internal organs were found. The results show that the toxicities of most of the compounds according to the present invention to mouse are significantly lower than sildenafil.

## Claims

1. A compound of formula (1A) or (1B), or a prodrug thereof, or a pharmaceutically acceptable salt or solvate of either entity, 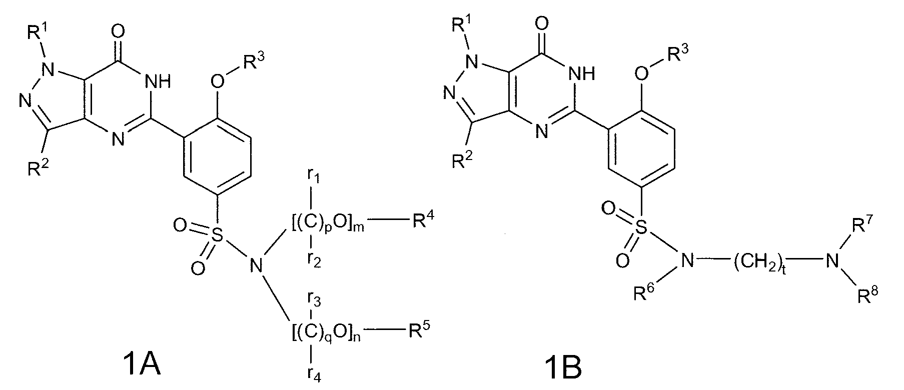 wherein
R¹ represents H, C₁-C₆ alkyl, C₃-C₆ cycloalkyl, C₁-C₃ alkyl substituted by halogen, or Cl-C3 alkyl substituted by C₃-C₆ cycloalkyl;
R² represents C2-C6 alkyl, C3-C6 cycloalkyl, C1-C3 alkyl substituted by halogen, or Cl-C3 alkyl substituted by C3-C6 cycloalkyl;
R³ represents C₁-C₆ alkyl, C₃-C₆ cycloalkyl, C₁-C₃ alkyl substituted by halogen, C1-C3 alkyl substituted by C₁-C₃ alkoxyl, or C1-C3 alkyl substituted by C₃-C₆ cycloalkyl;
wherein in 1A,
m=1-6 ; n=0-6;
p=1-5 ; q=1-5;
r₁, r₂, r₃ and r₄ independently represent H, C1-C3 alkyl, or r₁ and r₂, r₃ and r₄ together with the carbon to which they are attached form a ring;
R⁴ and R⁵ independently represent H, C₁-C₆ alkyl, (CH₂)ᵤAr , (CH₂)ᵥHet , COR⁹ , C1-C3 alkyl substituted by C1-C3 alkyl, or C1-C3 alkyl substituted by NR¹⁰R¹¹; in case of n=0, R⁵ does not represent H; in case of R¹ represents methyl, R² represents propyl, R³ represents ethyl, m=1, n=1, p=q=2 , r1, r2, r3 and r4 all represent H, R⁴ and R⁵ do not represent H at the same time.
R⁹ represents C₁-C₆ alkyl, C₁-C₆ alkyl substituted by C₁-C₃ alkoxyl, C1-C3 alkyl substituted by NR¹²R¹³, (CH₂)ᵤAr or (CH₂)ᵥHet ;
R¹⁰ and R¹¹ independently represent H, C₁-C₆ alkyl, C₁-C₆ alkyl substituted by C₁-C₃ alkoxyl, C1-C6 alkyl substituted by NR¹²R¹³, or R¹⁰ and R¹¹ together with the nitrogen to which they are attached form Het;
R¹² and R¹³ independently represent H or C₁-C₆ alkyl;
wherein in 1B,
t=1-5;
R⁶ represents C1-C6 alkyl, C3-C6 cycloalkyl, C1-C3 haloalkyl, C1-C3 alkoxyl, phenyl, pyridyl, furanyl, pyridazinyl, pyrazinyl, imidazolyl, C₁-C₃ substituted with hydroxyl, C₁-C₃ alkoxyl, acetoxyl, phenyl, pyridyl, furanyl, pyridazinyl, pyrimidinyl, pyrazinyl, imidazolyl; the above phenyl, pyridyl, furanyl, pyridazinyl, pyrimidinyl, pyrazinyl, imidazolyl optionally substituted with one or more substituents selected from halogen, C1-C3 alkyl, C1-C3 alkoxyl;
R⁷ and R⁸ independently represent H, C₁-C₆ alkyl, C3-C6 cycloalkyl, C₁-C₃ haloalkyl, C₁-C₆ alkoxyl, C1-C3 alkyl substituted with hydroxyl, acetoxyl, C₁-C₃ alkoxyl, or R⁷ and R⁸ together with the nitrogen to which they are attached form a four-membered to eight-membered heterocyclic ring, including morpholine, piperidine, pyrrole, piperazine; the above heterocyclic ring optionally substituted with one or more substituents selected from halogen, C1-C3 alkyl, C3-C6 cycloalkyl, C₁-C₃ haloalkyl, C1-C3 alkoxyl;
wherein u, v=0, 1 or 2;
Ar represents phenyl or phenyl substituted by one to two substituents selected from halogen, NH2, C1-C3 alkyl, C1-C3 alkoxy, CONH₂, CN, SO₂NH₂;
Het represents a four-membered and six-membered heterocyclic ring substituted with one or two substituents selected from halogen, C1-C3 alkyl, C1-C3 alkoxy, the heterocyclic contains one to four heteroatoms selected from nitrogen, sulfur, oxygen.

2. The compound according to claim 1,
wherein
R¹ represents C₁-C₄ alkyl or C₃-C₆ cycloalkyl;
R² represents C₂-C₄ alkyl or C3-C6 cycloalkyl;
R³ represents C₁-C₃ alkyl, C₁-C₃ alkyl substituted with C₁-C₃ alkoxyl;
wherein in 1A,
m=1-2 ; n=0-2;
p=2-4 ; q=2-4;
r₁, r₂, r₃ and r₄ independently represent H or methyl;
R⁴ and R⁵ independently represent H, C₁-C₃ alkyl, phenyl, pyridimethyl 4-piperidyl, COR⁹, C₁-C₃ alkyl substituted by NR¹⁰R¹¹; in case of n=0, R⁵ does not represent H; in case of R¹ represents methyl, R² represents propyl, R³ represents ethyl, m=1, n=1, p=q=2 , r₁, r₂, r₃ and r₄ all represent H, R⁴ and R⁵ do not represent H at same time;
R⁹ represents C₁-C4 alkyl, phenyl or pyridyl;
R¹⁰ and R¹¹ independently represent H, C₁-C₃ alkyl; or R¹⁰ and R¹¹ together with the nitrogen to which they are attached form a heterocyclic ring, including morpholine, piperazine, piperidine, pyrrole;
wherein in 1B,
t=2-3;
R⁶ represents C₁-C₃ alkyl, phenyl, pyridyl, benzyl or C₁-C₃ substituted with hydroxyl, C₁-C₃ alkoxyl, acetoxyl, phenyl, pyridyl;
R⁷ and R⁸ together with the nitrogen to which they are attached form a morpholine, piperidine or pyrrole heterocyclic ring.

3. The compound according to claim 2,
wherein
R¹ represents methyl or ethyl;
R² represents ethyl and n-propyl;
R³ represents ethyl, n-propyl or methyloxyethyl;
wherein in 1A,
m=1; n=0-1;
p=2-3; q=2-3;
r1, r2, r3 and r4 represent H;
R⁴ and R⁵ independently represent H, methyl, ethyl or COR⁹; C₁-C₃ alkyl substituted by NR¹⁰R¹¹; in case of n=0, R⁵ does not represent H; in case of R¹ represents methyl, R² represents propyl, R³ represents ethyl, m=1, n=1, p=q=2 , r1, r2, r3 and r4 all represent H, R⁴ and R⁵ do not represent H at same time;
R⁹ represents methyl or pyridyl;
wherein in 1B,
t=2-3,
R⁶ represents methyl, ethyl, benzyl, pyridylmethyl, or C₁-C₃ substituted with hydroxyl, C₁-C₃ alkoxyl, acetoxyl.

4. The compound according to claim 1, which is selected from the group consisting of:
1-methyl-5-{2-propyloxy-5-[bis(2-acetoxyethyl)amidosulfonyl]phenyl}-3-n-propyl-1,6-dihydro-7H-pyrazolo[4,3-d]pyrimidin-7-one (compound of example 1)
1-methyl-5-{2-propyloxy-5-[bis(2-hydroxyethyl)amidosulfonyl]phenyl}-3-n-propyl-1,6-dihydro-7H-pyrazolo[4,3-d]pyrimidin-7-one (compound of example 2)
1-methyl-5-{2-propoxy-5-[bis(2-nicotinoyloxyethyl)amidosulfonyl]phenyl}-3-n-propyl-1,6-dihydro-7H-pyrazolo[4,3-d]pyrimidin-7-one (compound of example 3)
1-methyl-5-{2-ethoxy-5-[bis(2-acetoxyethyl)amidosulfonyl]phenyl}-3-n-propyl-1,6-dihydro-7H-pyrazolo[4,3-d]pyrimidin-7-one (compound of example 4)
1-methyl-5-{2-ethoxy-5-[bis(2-nicotinoyloxyethyl)amidosulfonyl]phenyl}-3-n-propyl-1,6-dihydro-7H-pyrazolo[4,3-d]pyrimidin-7-one (compound of example 5)
1-methyl-5-{2-ethoxy-5-[bis(2-acetoxyethyl)amidosulfonyl]phenyl}-3-ethyl-1,6-dihydro-7H-pyrazolo[4,3-d]pyrimidin-7-one (compound of example 6)
1-methyl-5-{2-ethoxy-5-[bis(2-nicotinoyloxyethyl)amidosulfonyl]phenyl}-3-ethyl-1,6-dihydro-7H-pyrazolo[4,3-d]pyrimidin-7-one (compound of example 7)
1-methyl-5-{2-propoxy-5-[bis(2-hydroxyethyl)amidosulfonyl]phenyl}-3-ethyl-1,6-dihydro-7H-pyrazolo[4,3-d]pyrimidin-7-one (compound of example 8)
1-methyl-5-{2-methoxyethyl-5-[bis(2-acetoxyethyl)amidosulfonyl]phenyl}-3-n-propyl-1,6-dihydro-7H-pyrazolo[4,3-d]pyrimidin-7-one (compound of example 9)
1-methyl-5-{2-methoxyethyl-5-[bis(2-hydroxyethyl)amidosulfonyl]phenyl}-3-n-propyl-1,6-dihydro-7H-pyrazolo[4,3-d]pyrimidin-7-one (compound of example 10)
1-methyl-5-{2-ethoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one (compound of example 13)
1-methyl-5- {2-propoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one (compound of example 14)
1-methyl-5-{2-ethoxy-5-[1-(2-diethylaminoethyl)-1-(2-hydroxyethoxyethyl)amidosulfonyl]phenyl}-3-n-propyl-1,6-dihydro-7H-pyrazolo[4,3-d]pyrimidin-7-one (compound of example 16)
1-ethyl-5-{2-ethoxy-5-{[1-ethyl-1-[2-(1-ethyl-1-(2-acetoxyethyl))amino]ethyl]amidosulfonyl}phenyl}-3-n-propyl-1,6-dihydro-7H-pyrazolo[4,3-d]pyrimidin-7-one (compound of example 19)
1-methyl-5-{2-ethoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-one (compound of example 21)
1-methyl-5-{2-propoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-one (compound of example 23)
1-methyl-5-{2-propoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 24)
1-methyl-5-{2-ethoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 25)
1-methyl-5-{2-methoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 26)
1-methyl-5-{2-ethoxy-5-[[1-benzyl-1-(2-morpholin-1-yl)ethyl]amidosulfonyl]phenyl}-3-ethyl-1,6-dihydro-7H-pyrazolo[4,3-d]pyrimidin-7-one (compound of example 27)
1-methyl-5-{2-ethoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 28)
1-methyl-5-{2-ethoxy-5-[[1-(2-hydroxyethyl)-1-(2-piperidin-1-yl)ethyl]amidosulfonyl]phenyl}-3-n-propyl-1,6-dihydro-7H-pyrazolo[4,3-d]pyrimidin-7-one (compound of example 29)
1-methyl-5-{2-propoxy-5-[[1-methyl-1-(2-hydroxyethyl)]amidosulfonyl]phenyl}-3-n-propyl-1,6-dihydro-7H-pyrazolo[4,3-d]pyrimidin-7-one (compound of example 31)
1-methyl-5-{2-propoxy-5-[[1-methyl-1-(2-acetoxyethyl)]amidosulfonyl]phenyl}-3-n-propyl-1,6-dihydro-7H-pyrazolo[4,3-d]pyrimidin-7-one (compound of example 33)
1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 35)
1-methyl-5-{2-ethoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 36)
1-methyl-5-{2-propoxy-5-[bis(3-hydroxypropyl)amidosulfonyl]phenyl}-3-ethyl-1,6-dihydro-7H-pyrazolo[4,3-d]pyrimidin-7-one (compound of example 37)
1-methyl-5-{2-propoxy-5-[[1-(2-hydroxyethyl-1-(3-hydroxypropyl)]amidosulfonyl]phenyl}-3-n-propyl-1,6-dihydro-7H-pyrazolo[4,3-d]pyrimidin-7-one (compound of example 38)
1-methyl-5-{2-propoxy-5-[[1-(2-hydroxyethoxyethyl-1-(3-hydroxypropyl)]amidosulfonyl]phenyl}-3-n-propyl-1,6-dihydro-7H-pyrazolo[4,3-d]pyrimidin-7-one (compound of example 39)
1-methyl-5-{2-propoxy-5-[[1-ethyl-1-(2-morpholin-1-yl)ethyl]amidosulfonyl]phenyl}-3-propyl-1,6-dihydro-7H-pyrazolo[4,3-d]pyrimidin-7-one (compound of example 41)
1-methyl-5-{2-propoxy-5-[bis(2-hydroxypropyl)amidosulfonyl]phenyl}-3-propyl-1,6-dihydro-7H-pyrazolo[4,3-d]pyrimidin-7-one (compound of example 42)
1-methyl-5-{2-ethoxy-5-[[1-ethyl-1-(2-morpholin-1-yl)ethyl]amidosulfonyl]phenyl}-3-n-propyl-1,6-dihydro-7H-pyrazolo[4,3-d]pyrimidin-7-one (compound of example 43)
1-methyl-5-{2-ethoxy-5-[bis(3-hydroxypropyl)amidosulfonyl]phenyl}-3-n-propyl-1,6-dihydro-7H-pyrazolo[4,3-d]pyrimidin-7-one (compound of example 44)
1-methyl-5-{2-ethoxy-5-[bis(2-hydroxypropyl)amidosulfonyl]phenyl}-3-n-propyl-1,6-dihydro-7H-pyrazolo[4,3-d]pyrimidin-7-one (compound of example 45)
1-methyl-5-{2-ethoxy-5-[[1-(2-hydroxyethyl)-1-(3-hydroxypropyl)]amidosulfonyl]phenyl}-3-n-propyl-1,6-dihydro-7H-pyrazolo[4,3-d]pyrimidin-7-one (compound of example 46)
1-methyl-5-{2-ethoxy-5-[[1-(2-hydroxyethoxyethyl)-1-(3-hydroxypropyl)]amidosulfonyl]phenyl}-3-n-propyl-1,6-dihydro-7H-pyrazolo[4,3-d]pyrimidin-7-one (compound of example 47)
1-methyl-5-{2-ethoxy-5-[[1-ethyl-1-(2-hydroxyethyl)]amidosulfonyl]phenyl}-3-n-propyl-1,6-dihydro-7H-pyrazolo[4,3-d]pyrimidin-7-one (compound of example 51)
1-methyl-5-{2-propoxy-5-[[1-ethyl-1-(2-hydroxyethyl)]amidosulfonyl]phenyl}-3-n-propyl-1,6-dihydro-7H-pyrazolo[4,3-d]pyrimidin-7-one (compound of example 52)
1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 53)
1-methyl-5-{2-propoxy-5-[[1-ethyl-1-(2-morpholin-1-yl)ethyl]amidosulfonyl]phenyl}-3-ethyl-1,6-dihydro-7H-pyrazolo[4,3-d]pyrimidin-7-one (compound of example 54)
1-methyl-5-{2-propoxy-5-[[1-(4-fluorobenzyl)-1-(2-morpholin-1-yl)ethyl]amidosulfonyl]phenyl}-3-n-propyl-1,6-dihydro-7H-pyrazolo[4,3-d]pyrimidin-7-one (compound of example 56)
1-methyl-5-{2-propoxy-5-[[1-(2-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 60)
1-methyl-5-{2-ethoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 61)
1-methyl-5-{2-propoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 62)
1-methyl-5-{2-propoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 63)
1-methyl-5-{2-ethoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 64)
1-methyl-5-{2-propoxy-5-[[1-benzyl-1-(3-hydroxypropyl)]amidosulfonyl]phenyl}-3-n-propyl-1,6-dihydro-7H-pyrazolo[4,3-d]pyrimidin-7-one (compound of example 65)
1-methyl-5-{2-ethoxy-5-[[1-benzyl-1-(3-hydroxypropyl)]amidosulfonyl]phenyl}-3-n-propyl-1,6-dihydro-7H-pyrazolo[4,3-d]pyrimidin-7-one (compound of example 66)
1-methyl-5-{2-propoxy-5-[[1-benzyl-1-(2-hydroxyethyl)]amidosulfonyl]phenyl}-3-n-propyl-1,6-dihydro-7H-pyrazolo[4,3-d]pyrimidin-7-one (compound of example 68)
1-methyl-5-{2-propoxy-5-[[1-ethyl-1-(3-hydroxypropyl)]amidosulfonyl]phenyl}-3-n-propyl-1,6-dihydro-7H-pyrazolo[4,3-d]pyrimidin-7-one (compound of example 70)
1-methyl-5-{2-propoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7-one (compound of example 72)
1-methyl-5-{2-propoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one (compound of example 78)
1-methyl-5-{2-ethoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one (compound of example 79)
1-methyl-5-{2-propoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 80)
1-methyl-5-{2-ethoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 81)
1-methyl-5-{2-ethoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one (compound of example 82)
1-methyl-5-{2-propoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one (compound of example 83)
1-methyl-5-{2-propoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 84)
1-methyl-5-{2-ethoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 85)
1-methyl-5-{2-propoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one (compound of example 87)
1-methyl-5-{2-ethoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one (compound of example 88)
1-methyl-5-{2-propoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one (compound of example 89)
1-methyl-5-{2-ethoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one (compound of example 90)
1-methyl-5-{2-propoxy-5-[[1-(2-acetoxyethyl)-1-(2-hydroxyethyl)]amidosulfonyl]phenyl}-3-n-propyl-1,6-dihydro-7H-pyrazolo[4,3-d]pyrimidin-7-one (compound of example 91)
1-methyl-5- {2-propoxy-5-[[1-(3-acetoxypropyl)-1-(3-hydroxypropyl)]amidosulfonyl]phenyl}-3-n-propyl-1,6-dihydro-7H-pyrazolo[4,3-d]pyrimidin-7-one (compound of example 92)
1-methyl-5-{2-propoxy-5-[bis(3-acetoxypropyl)amidosulfonyl]phenyl}-3-n-propyl-1,6-dihydro-7H-pyrazolo[4,3-d]pyrimidin-7-one (compound of example 93)
1-methyl-5-{2-propoxy-5-[[1-ethyl-1-(2-acetoxyethyl)]amidosulfonyl]phenyl}-3-n-propyl-1,6-dihydro-7H-pyrazolo[4,3-d]pyrimidin-7-one (compound of example 94)
1-methyl-5-{2-propoxy-5-[[1-(2-hydroxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 95)
1-methyl-5-{2-propoxy-5-[[1-(2-acetoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 96)
1-methyl-5-{2-propoxy-5-[[1-ethyl-1-(3-acetoxypropyl)]amidosulfonyl]phenyl}-3-n-propyl-1,6-dihydro-7H-pyrazolo[4,3-d]pyrimidin-7-one (compound of example 97)
1-methyl-5-{2-propoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 98)
1-methyl-5-{2-propoxy-5-[[1-(methoxypropyl)-1-(3-hydroxypropyl)]amidosulfonyl]phenyl}-3-n-propyl-1,6-dihydro-7H-pyrazolo[4,3-d]pyrimidin-7-one (compound of example 99)
1-methyl-5-{2-ethoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 100)
1-methyl-5-{2-ethoxy-5-[[1-(2-methoxyethyl)-1-(2-hydroxyethyl)]amidosulfonyl]phenyl}-3-n-propyl-1,6-dihydro-7H-pyrazolo[4,3-d]pyrimidin-7-one (compound of example 101)
1-methyl-5-{2-ethoxy-5-[[1-(2-methoxyethyl)-1-(3-hydroxypropyl)]amidosulfonyl]phenyl}-3-n-propyl-1,6-dihydro-7H-pyrazolo[4,3-d]pyrimidin-7-one (compound of example 102)
1-methyl-5-{2-propoxy-5-[bis(3-methoxypropyl)amidosulfonyl]phenyl}-3-n-propyl-1,6-dihydro-7H-pyrazolo[4,3-d]pyrimidin-7-one (compound of example 103)
1-methyl-5-{2-propoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 104)
1-methyl-5-{2-propoxy-5-[[1-(2-methoxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 105)
1-methyl-5-{2-propoxy-5-[[1-(2-methoxyethyl)-1-(2-hydroxyethyl)]amidosulfonyl]phenyl}-3-n-propyl-1,6-dihydro-7H-pyrazolo[4,3-d]pyrimidin-7-one (compound of example 106)
1-methyl-5-{2-ethoxy-5-[[1-methyl-1-(2-acetoxyethyl)]amidosulfonyl]phenyl}-3-n-propyl-1,6-dihydro-7H-pyrazolo[4,3-d]pyrimidin-7-one (compound of example 107)
1-methyl-5-{2-ethoxy-5-[bis(3-acetoxypropyl)amidosulfonyl]phenyl}-3-n-propyl-1,6-dihydro-7H-pyrazolo[4,3-d]pyrimidin-7-one (compound of example 108)
1-methyl-5-{2-propoxy-5-[[1-(3-methoxypropyl)-1-(2-hydroxyethyl)]amidosulfonyl]phenyl}-3-n-propyl-1,6-dihydro-7H-pyrazolo[4,3-d]pyrimidin-7-one (compound of example 109)
1-methyl-5-{2-propoxy-5-[[1-methyl-1-(3-dimethylaminopropyl)]amidosulfonyl]phenyl}-3-n-propyl-1,6-dihydro-7H-pyrazolo[4,3-d]pyrimidin-7-one (compound of example 110)
1-methyl-5-{2-propoxy-5-[[1-(2-ethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 111)
1-methyl-5-{2-propoxy-5-[[1-ethyl-1-(3-morpholin-1-yl)propyl]amidosulfonyl]phenyl}-3-n-propyl-1,6-dihydro-7H-pyrazolo[4,3-d]pyrimidin-7-one (compound of example 112)
1-methyl-5-{2-propoxy-5-[[1-benzyl-1-(3-morpholin-1-yl)propyl]amidosulfonyl]phenyl}-3-n-propyl-1,6-dihydro-7H-pyrazolo[4,3-d]pyrimidin-7-one (compound of example 113)
1-methyl-5-{2-propoxy-5-[[1-(3-methoxypropyl)-1-(2-acetoxyethyl)]amidosulfonyl]phenyl}-3-n-propyl-1,6-dihydro-7H-pyrazolo[4,3-d]pyrimidin-7-one (compound of example 114)
1-methyl-5-{2-ethoxy-5-[[1-(3-acetoxypropyl)-1-(3-hydroxypropyl)]amidosulfonyl]phenyl}-3-n-propyl-1,6-dihydro-7H-pyrazolo[4,3-d]pyrimidin-7-one (compound of example 115)
1-methyl-5-{2-propoxy-5-[[1-(3-ethoxyethyl)-1-(2-hydroxyethyl)]amidosulfonyl]phenyl}-3-n-propyl-1,6-dihydro-7H-pyrazolo[4,3-d]pyrimidin-7-one (compound of example 116)
1-methyl-5-{2-propoxy-5-[[1-benzyl-1-(4-morpholin-1-yl)butyl]amidosulfonyl]phenyl}-3-n-propyl-1,6-dihydro-7H-pyrazolo[4,3-d]pyrimidin-7-one (compound of example 117)
1-methyl-5-{2-propoxy-5-[[1-(3-methoxypropyl)-1-(3-acetoxypropyl)]amidosulfonyl]phenyl}-3-n-propyl-1,6-dihydro-7H-pyrazolo[4,3-d]pyrimidin-7-one (compound of example 118).

5. A pharmaceutical composition comprising a compound of formula 1A or 1B as defined in any one of claims 1 to 4, or a prodrug thereof, or a pharmaceutically acceptable salt or solvate of either entity, together with one or more pharmaceutically acceptable excipients.

6. The composition according to claim 5, the effective dosage is 1-500mg/day, preferably 10-100mg/day.

7. A process for the preparation of the compounds of formula 1A according to claim 1, or the prodrug thereof, or the pharmaceutically acceptable salt or solvate of either entity:
The compounds of formula 1A (both R⁴ and R⁵ are not H) without hydroxyl group at the ending of the sulfonamide chain may be prepared by the following process:
step1, 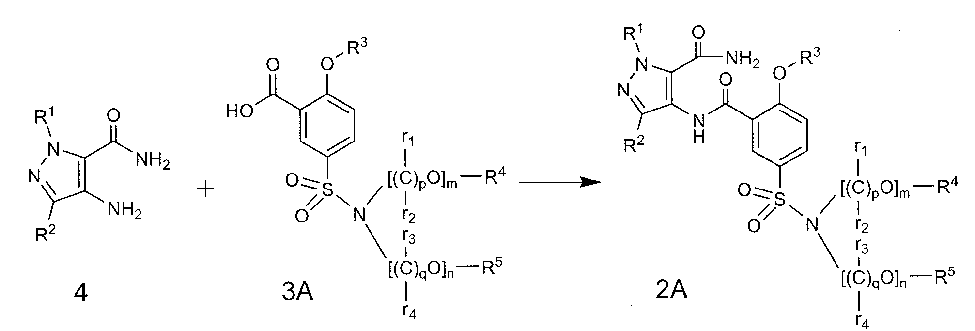 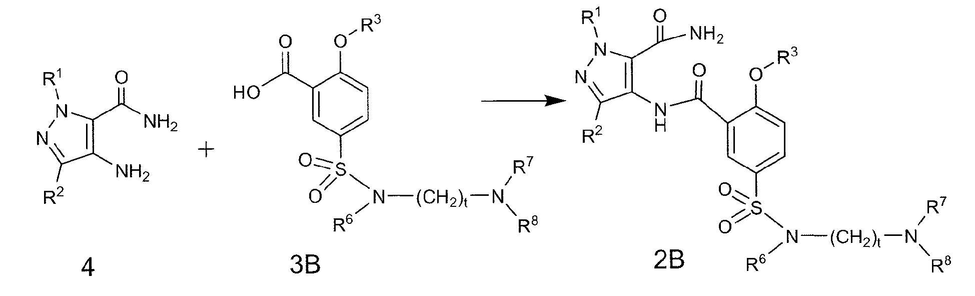 wherein R¹, R², R³, R⁴, R⁵, R⁶, R⁷, R⁸, r₁, r₂, r₃, r₄, m, n, p, q and t are as previously defined in claim 1, but R⁴, R⁵ are not H;
Method 1, the carboxyl group in the compounds of formula (3A) or (3B) was transformed into acyl chloride or mixed anhydride by using thionyl chloride, oxalyl chloride or ethyl chlorformate, then the mixture was reacted with the compounds of formula 4 to get the crossponding acid amide (2A) or (2B). The acidylation reaction is usually carried out in the presence of a suitable deacidification reagent and a common solvent. Preferred deacidification reagents include organic bases (preferred triethylamine, N,N-diisopropylethylamine, pyridine) and inorganic bases (preferred hydroxides, carbonates). Preferred solvents include diolefines (preferred petroleum, n-hexane, cyclohexane), halohydrocarbon (preferred dichlormethane, chloroform), ethers (preferred tetrahydrofuran, dioxane, ether), aromatic solvents (preferred toluene) and alcohols (preferred t-butanol, isopropanol);
Method 2, carboxylic acid was reacted with amine derivates directly to get the formula (2A) and (2B), which is usually carried out in the anhydrous solvent with the presence of activating agent or dehydrating agent. Preferred activating agents or dehydrating agents include DCC, EDCI, EEDQ, CDI, HOBt. Preferred solvents include halogenated hydrocarbons (e.g. dichlormethane, dichlormethane), ethers (e.g. tetrahydrofuran, dioxane, ether), aromatic hydrocarbons (e.g. benzene, toluene), polarity aprotic solvent (dimethylsulfoxide, N,N-dimethylformamide), or their mixture;
step 2, 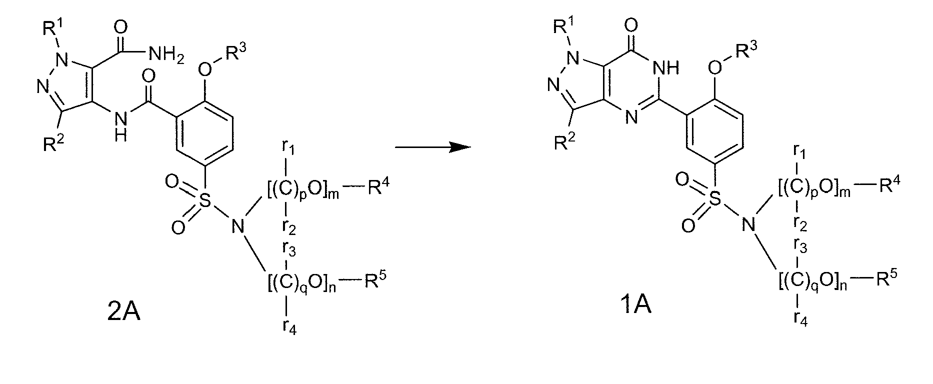 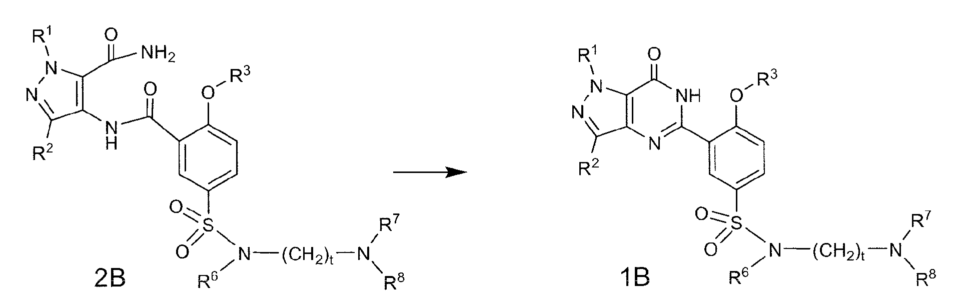 wherein R¹, R², R³, R⁴, R⁵, R⁶, R⁷, R⁸, r₁, r₂, r₃, r₄, m, n, p, q and t are as previously defined in claim 1, but R⁴, R⁵ are not H;
the reaction is usually carried out in the presence of a suitable base and a suitable solvent at temperatures in a range of from 30 to 200°C. Preferred bases include metal alkoxides (e.g. potassium tertbutoxide, sodium ethoxide), alkaline earth metal or hydrides of alkali metal, amine (e.g. triethylamine), metal salts of ammonia, hydroxides (e.g. sodium hydroxide), carbonates and bicarbonates. Preferred solvents include alcohols (e.g. t-butanol, methanol, ethanol, isopropanol, glycol, 2-methoxyethanol), aromatic hydrocarbons (e.g. benzene, toluene, chlorobenzene), pyridine, halogenated hydrocarbon, acetonitrile, tetrahydrofuran, dioxane, dimethylsulfoxide, N,N-dimethylformamide, N-methylpyrrolidin-2-one.

8. A process for the preparation of the compounds of formula 1A according to claim 1, or the prodrug thereof, or the pharmaceutically acceptable salt or solvate of either entity:
The compounds of formula 1A (at least one of R⁴ and R⁵ is H) containing hydroxyl group at the ending of the sulfonamide chain may be prepared form the hydrolysis of their corresponding ester derivates; 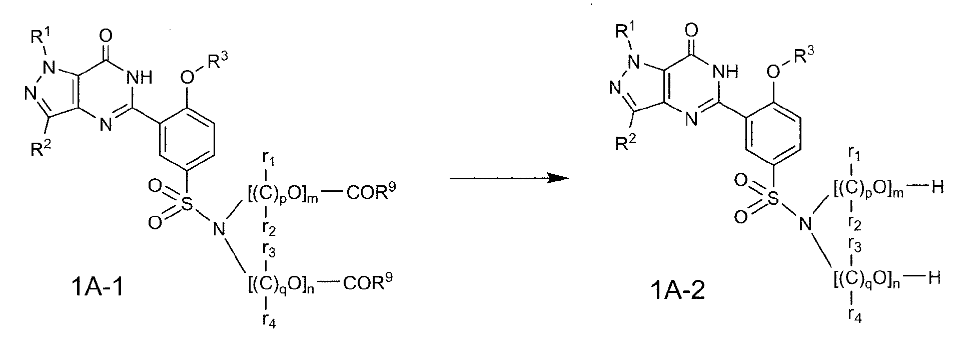
wherein R¹, R², R³, R⁴, R⁵, R⁹, r₁, r₂, r₃, r₄, m, n, p and q are as previously defined in claim 1.

9. The use of a compound of pyrazolopyrimidinone derivative according to claim 1, or a physiologically acceptable salt thereof, for the curative or prophylactic treatment of the diseases of male erectile dysfunction, female sexual dysfunction, premature labour, dysmenorrhoea, benign prostatic hyperplasia (BPH), bladder outlet obstruction, incontinence, stable, unstable and variant (Prinzmetal) angina, hypertension, pulmonary hypertension, congestive heart failure, kidney failure, atherosclerosis, stroke, peripheral vascular disease, conditions of reduced blood vessel patency, inflammatory disease, bronchitis, chronic asthma, allergic asthma, allergic rhinitis, glaucoma or diseases **characterized by** disorders of gut motility.
